(19) Europäisches Patentamt
European Patent Office
Office européen des brevets

(11) EP 1 857 551 A2

(12) **EUROPEAN PATENT APPLICATION**

(43) Date of publication:
**21.11.2007 Bulletin 2007/47**

(21) Application number: **07009163.2**

(22) Date of filing: **29.05.2001**

(51) Int Cl.:
***C12N 15/12*** *(2006.01)* ***C07K 14/47*** *(2006.01)*
***C07K 16/18*** *(2006.01)* ***G01N 33/50*** *(2006.01)*
***A61K 38/17*** *(2006.01)* ***C12N 15/83*** *(2006.01)*
***C12N 15/62*** *(2006.01)* ***C12N 9/18*** *(2006.01)*

(84) Designated Contracting States:
**AT BE CH CY DE DK ES FI FR GB GR IE IT LI LU MC NL PT SE TR**

(30) Priority: **01.06.2000 US 208550 P**
**04.08.2000 US 223542 P**

(62) Document number(s) of the earlier application(s) in accordance with Art. 76 EPC:
**01939708.2 / 1 290 169**

(71) Applicant: **Amgen Inc.**
**Thousand Oaks, CA 91320-1799 (US)**

(72) Inventors:
- **Paszty, Christopher J.**
**Ventura, CA 93003 (US)**
- **Gao, Yongming**
**Thousand Oaks, CA 91360 (US)**

(74) Representative: **Grünecker, Kinkeldey, Stockmair & Schwanhäusser Anwaltssozietät**
**Maximilianstrasse 58**
**80538 München (DE)**

Remarks:
This application was filed on 07 - 05 - 2007 as a divisional application to the application mentioned under INID code 62.

(54) **Cystine-knot polypeptides: cloaked-2 molecules and uses thereof**

(57) The present invention relates to novel Cloaked-2 polypeptides and nucleic acid molecules encoding the same. The invention also provides vectors, host cells, selective binding agents, and methods for producing Cloaked-2 polypeptides. Also provided for are methods for the treatment, diagnosis, amelioration, or prevention of diseases with Cloaked-2 polylpeptides.

EP 1 857 551 A2

## Description

This application claims priority of U.S. Provisional Application Serial No. 60/208,550 filed June 1, 2000 and U.S. Provisional Application Serial No. 60/223,542 filed August 4, 2000.

### Field of the Invention

The present invention relates to novel Cloaked-2 polypeptides and nucleic acid molecules encoding the same. The invention also relates to vectors, host cells, pharmaceutical compositions, selective binding agents and methods for producing Cloaked-2 polypeptides. Also provided for are methods for the diagnosis, treatment, amelioration, and/or prevention of diseases associated with Cloaked-2 polypeptides.

### Background of the Invention

Technical advances in the identification, cloning, expression and manipulation of nucleic acid molecules and the deciphering of the human genome have greatly accelerated the discovery of novel therapeutics. Rapid nucleic acid sequencing techniques can now generate sequence information at unprecedented rates and, coupled with computational analyses, allow the assembly of overlapping sequences into partial and entire genomes and the identification of polypeptide-encoding regions. A comparison of a predicted amino acid sequence against a database compilation of known amino acid sequences allows one to determine the extent of homology to previously identified sequences and/or structural landmarks. The cloning and expression of a polypeptide-encoding region of a nucleic acid molecule provides a polypeptide product for structural and functional analyses. The manipulation of nucleic acid molecules and encoded polypeptides may confer advantageous properties on a product for use as a therapeutic.

In spite of the significant technical advances in genome research over the past decade, the potential for the development of novel therapeutics based on the human genome is still largely unrealized. Many genes encoding potentially beneficial polypeptide therapeutics, or those encoding polypeptides, which may act as "targets" for therapeutic molecules, have still not been identified.

Accordingly, it is an object of the invention to identify novel polypeptides and nucleic acid molecules encoding the same, which have diagnostic or therapeutic benefit.

The cystine-knot growth factor structural superfamily is comprised of four families: TSF-β (transforming growth factor beta), PDGF (platelet-derived growth factor), NGF (nerve growth factor) and the Glycoprotein Hormones. Although there is no significant amino acid homology between these families, the crystal structures that have been determined for various members of these families are remarkably similar and has led to their grouping into a structural superfamily. See Isaacs, Current Opinion in Structural Biology, 5:391-395 (1995). This three dimensional similarity can be attributed to the fact that the major structural determinant for this class of proteins is a 6 cysteine (3 disulfide) structure called the "cystine-knot". For all members of the TGF-β, PDGF, and Glycoprotein Hormone families, the number 2 and 3 cysteines are found in the motif, "CxGxC" and the number 5 and 6 cysteines are found in the motif "CxC", where "x" refers to any amino acid. All members of the cystine-knot growth factor structural superfamily are secreted signaling molecules.

The inventors hypothesized that there might exist unidentified cystine-knot families and, if so, members of such families would not have significant homology to the known cystine-knot growth factor family members, and thus could not be identified using standard homology based computational approaches (such as Blast searches, profile searches, etc.). Any member of such a family would, however, contain the cystine-knot motifs.

### Summary of the Invention

With the goal of identifying novel secreted signaling molecules, a highly specific "CxGxC-class cystine-knot pattern" was developed for database mining. This pattern is extremely specific in terms of identifying known CxGxC-class cystine-knot proteins. The presence of a signal peptide, lack of transmembrane domain(s), and total polypeptide size being less than 550 amino acids were used as secondary screening criteria. A novel human secreted polypeptide that contains both cystine knot motifs (CxGxC and CxC) and meets the criteria of our highly specific "CxGxC cystine knot pattern" has now been identified, and is termed "Cloaked-2" herein. The mouse "Cloaked-2" polypeptide has also been identified. GAP analysis reveals that there is 88% amino acid identity between full length human Cloaked-2 and mouse Cloaked-2 polypeptides. Mouse Cloaked-2 contains both cystine knot motifs (CxGxC and CxC) and meets the criteria of our highly specific "CxGxC cystine knot pattern". Thus, Cloaked-2 is a member of a new family of cystine-knot proteins based on the fact that both human and mouse Cloaked-2 contain the two classic cystine-knot motifs (CxGxC and CxC), meet the criteria of our highly specific "CxGxC cystine-knot pattern", have N-terminal predicted signal peptides, have no predicted transmembrane domains and are each less than 550 amino acids in size.

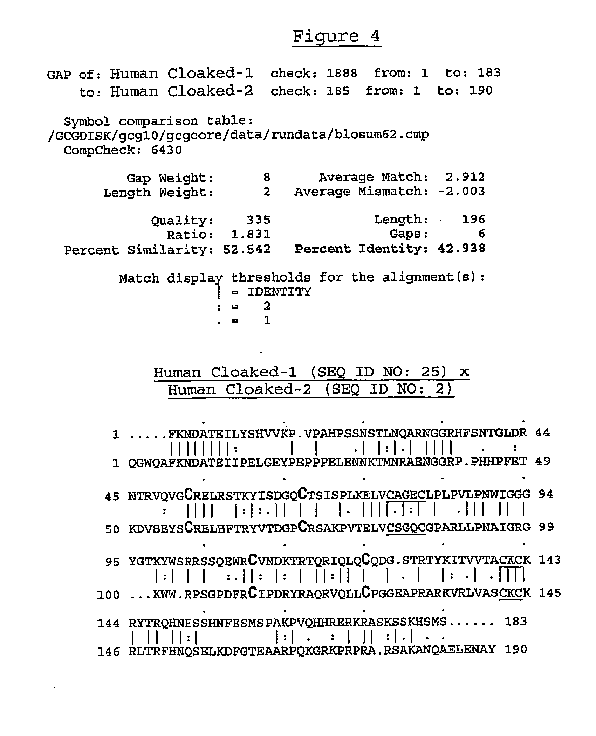

Among the known genes in the human genome, Cloaked-1 is most related to Cloaked-2, and these 2 genes comprise a divergent subgroup as compared to much more distantly related genes. Each polypeptide contains 8 conserved cystines. GAP analysis reveals that there is 43% amino acid identity between the mature forms of human Cloaked-1 and human Cloaked-2 polypeptides (Figure 4). Furthermore, the classic cystine-knot motifs, CxGxC and CxC, are conserved and the four additional conserved cystines are all identified as well in Figure 4. The cystines in the CxGxC and CxC motifs represent cystines number 2 and 3, and, respectively, 5 and 6 of the six cystines that form the cystine-knot. Cystine number 1 of the cystine-knot would be either cystine 52 or 66 for Cloaked-1 and cystine 57 or 71 for Cloaked-2. Cystine number 4 of the cystine-knot would be either cystine 110 or 124 for Cloaked-1 and cystine 111 or 125 for Cloaked-2. For all known cystine-knot polypeptides the six cystines form 3 disulfide bonds in the following pairings: cystine number 1 and 4, cystine number 2 and 5, cystine number 3 and 6.

The present invention thus relates to novel Cloaked-2 nucleic acid molecules and encoded polypeptides.

The invention provides for an isolated nucleic acid molecule comprising a nucleotide sequence selected from the group consisting of:

(a) the nucleotide sequence as set forth in SEQ ID NO:1 or SEQ ID NO:3;

(b) a nucleotide sequence encoding the polypeptide as set forth in SEQ ID NO:2 or SEQ ID NO:4;'

(c) a nucleotide sequence which hybridizes under moderately or highly stringent conditions to the complement of (a) or (b), wherein the encoded polypeptide has an activity of the polypeptide as set forth in SEQ ID NO:2 or SEQ ID NO:4; and

(d) a nucleotide sequence complementary to any of (a) - (c) .

The invention also provides for an isolated nucleic acid molecule comprising a nucleotide sequence selected from the group consisting of:

(a) a nucleotide sequence encoding a polypeptide that is at least about 70, 75, 80, 85, 90, 95, 96, 97, 98, or 99 percent identical to the polypeptide as set forth in SEQ ID NO:2 or SEQ ID NO:4, wherein the polypeptide has an activity of the polypeptide as set forth in SEQ ID NO:2 or SEQ ID NO:4;

(b) a nucleotide sequence encoding an allelic variant or splice variant of the nucleotide sequence as set forth in SEQ ID NO:1 or SEQ ID NO:3, wherein the encoded polypeptide has an activity of the polypeptide as set forth in SEQ ID NO:2 or SEQ ID NO:4;

(c) a nucleotide sequence of SEQ ID NO:1 or SEQ ID NO:3, (a), or (b) encoding a polypeptide fragment of at least about 25 amino acid residues, wherein the polypeptide has an activity of the polypeptide as set forth in SEQ ID NO: 2 or SEQ ID NO:4;

(d) a nucleotide sequence of SEQ ID NO:1 or SEQ ID NO:3, or (a)-(d) comprising a fragment of at least about 16 nucleotides;

(e) a nucleotide sequence which hybridizes under moderately or highly stringent conditions to the complement of any of (a)-(d), wherein the polypeptide has an activity of the polypeptide as set forth in SEQ ID NO:2 or SEQ ID NO: 4; and

(f) a nucleotide sequence complementary to any of (a) - (d) .

The invention further provides for an isolated nucleic acid molecule comprising a nucleotide sequence selected from the group consisting of:

(a) a nucleotide sequence encoding a polypeptide as set forth in SEQ ID NO:2 or SEQ ID NO:4 with at least one conservative amino acid substitution, wherein the polypeptide has an activity of the polypeptide as set forth in SEQ ID NO:2 or SEQ ID NO:4;

(b) a nucleotide sequence encoding a polypeptide as set forth in SEQ ID NO:2 or SEQ ID NO:4 with at least one amino acid insertion, wherein the polypeptide has an activity of the polypeptide as set forth in SEQ ID NO:2 or SEQ ID NO:4;

(c) a nucleotide sequence encoding a polypeptide as set forth in SEQ ID NO:2 or SEQ ID NO:4 with at least one amino acid deletion, wherein the polypeptide has an activity of the polypeptide as set forth in SEQ ID NO:2 or SEQ ID NO:4;

(d) a nucleotide sequence encoding a polypeptide as set forth in SEQ ID NO:2 or SEQ ID NO:4 which has a C- and/or N- terminal truncation, wherein the polypeptide has an activity of the polypeptide as set forth in SEQ ID NO: 2 or SEQ ID NO:4;

(e) a nucleotide sequence encoding a polypeptide as set forth in SEQ ID NO:2 or SEQ ID NO:4 with at least one modification selected from the group consisting of amino acid substitutions, amino acid insertions, amino acid deletions, C-terminal truncation, and N-terminal truncation, wherein the polypeptide has an activity of the polypeptide as set forth in SEQ ID NO:2 or SEQ ID NO:4;

(f) a nucleotide sequence of (a) - (e) comprising a fragment of at least about 16 nucleotides;

(g) a nucleotide sequence which hybridizes under moderately or highly stringent conditions to the complement of any of (a)-(f), wherein the polypeptide has an activity of the polypeptide as set forth in SEQ ID NO:2 or SEQ ID NO: 4; and

(h) a nucleotide sequence complementary to any of (a)-(e).

The invention also provides for an isolated polypeptide comprising the amino acid sequence selected from the group consisting of:

(a) The mature amino acid sequence as set forth in SEQ ID NO:2, and optionally further comprising an amino-terminal methionine; or the mature amino acid sequence as set forth in SEQ ID NO:4, and optionally further comprising an amino-terminal methionine;

(b) an amino acid sequence for an ortholog of SEQ ID NO:2 or SEQ ID NO:4, wherein the encoded polypeptide has an activity of the polypeptide as set forth in SEQ ID NO:2 or SEQ ID NO:4;

(c) an amino acid sequence that is at least about 70, 80, 85, 90, 95, 96, 97, 98, or 99 percent identical to the amino acid sequence of SEQ ID NO:2 or SEQ ID NO:4, wherein the polypeptide has an activity of the polypeptide as set forth in SEQ ID NO:2 or SEQ ID NO:4;

(d) a fragment of the amino acid sequence set forth in SEQ ID NO:2 or SEQ ID NO:4 comprising at least about 25 amino acid residues, wherein the polypeptide has an activity of the polypeptide as set forth in SEQ ID NO:2 or SEQ ID NO:4;

(e) an amino acid sequence for an allelic variant or splice variant of either the amino acid sequence as set forth in SEQ ID NO:2 or SEQ ID NO:4, or at least one of (a)-(c) wherein the polypeptide has an activity of the polypeptide as set forth in SEQ ID NO:2 or SEQ ID NO:4.

The invention further provides for an isolated polypeptide comprising the amino acid sequence selected from the group consisting of:

(a) the amino acid sequence as set forth in SEQ ID NO:2 or SEQ ID NO:4 with at least one conservative amino acid substitution, wherein the polypeptide has an activity of the polypeptide as set forth in SEQ ID NO:2 or SEQ ID NO:4;

(b) the amino acid sequence as set forth in SEQ ID NO:2 or SEQ ID NO:4 with at least one amino acid insertion, wherein the polypeptide has an activity of the polypeptide as set forth in SEQ ID NO:2 or SEQ ID NO:4;

(c) the amino acid sequence as set forth in SEQ ID NO:2 or SEQ ID NO:4 with at least one amino acid deletion, wherein the polypeptide has an activity of the polypeptide as set forth in SEQ ID NO:2 or SEQ ID NO:4;

(d) the amino acid sequence as set forth in SEQ ID NO:2 or SEQ ID NO:4 which has a C- and/or N-terminal truncation, wherein the polypeptide has an activity of the polypeptide as set forth in SEQ ID NO:2 or SEQ ID NO:4; and

(e) the amino acid sequence as set forth in SEQ ID NO:2 or SEQ ID NO:4, with at least one modification selected from the group consisting of amino acid substitutions, amino acid insertions, amino acid deletions, C-terminal truncation, and N-terminal truncation, wherein the polypeptide has an activity of the polypeptide as set forth in SEQ ID NO:2 or SEQ ID NO:4.

Also provided are fusion polypeptides comprising the amino acid sequences of (a)-(e) above.

The present invention also provides for an expression vector comprising the isolated nucleic acid molecules as set forth herein, recombinant host cells comprising recombinant nucleic acid molecules as set forth herein, and a method of producing a Cloaked-2 polypeptide comprising culturing the host cells and optionally isolating the polypeptide so produced.

A transgenic non-human animal comprising a nucleic acid molecule encoding a Cloaked-2 polypeptide is also encompassed by the invention. The Cloaked-2 nucleic acid molecules are introduced into the animal in a manner that allows expression and increased levels of the Cloaked-2 polypeptide, which may include increased circulating levels. The transgenic non-human animal is preferably a mammal.

Also provided are derivatives of the Cloaked-2 polypeptides of the present invention.

Analogs of Cloaked-2 are provided for in the present invention which result from conservative and non-conservative amino acids substitutions of the Cloaked-2 polypeptide of SEQ ID NO: 2. Such analogs include a Cloaked-2 polypeptide wherein the amino acid at position 9 is selected from the group consisting of aspartic acid or glutamic acid; a Cloaked-2 polypeptide wherein the amino acid at position 39 is selected from the group consisting of glycine; proline, or alanine; a Cloaked-2 polypeptide wherein the amino acid at position 58 is selected from the group consisting of arginine, lysine, glutamine or asparagine; a Cloaked-2 polypeptide wherein the amino acid at position 81 is selected from the group consisting of valine, isoleucine, methionine, leucine, phenylalanine, alanine, or norleucine; a Cloaked-2 polypeptide wherein the amino acid at position 102 is selected from the group consisting of tryptophan, tyrosine, or phenylalanine; a Cloaked-2 polypeptide wherein the amino acid at position 154 is selected from the group consisting of serine, threonine, or alanine.

Additionally provided are selective binding agents such as antibodies and peptides capable of specifically binding the Cloaked-2 polypeptides of the invention. Such antibodies and peptides may be agonistic or antagonistic.

Pharmaceutical compositions comprising the nucleotides, polypeptides, or selective binding agents of the present invention and one or more pharmaceutically acceptable formulation agents are also encompassed by the invention. The pharmaceutical compositions are used to provide therapeutically effective amounts of the nucleotides or polypeptides of the present invention. The invention is also directed to methods of using the polypeptides, nucleic acid molecules, and selective binding agents.

The Cloaked-2 polypeptides and nucleic acid molecules of the present invention may be used to treat, prevent, ameliorate, and/or detect diseases and disorders, including those recited herein.

The present invention also provides a method of assaying test molecules to identify a test molecule which binds to a Cloaked-2 polypeptide. The method comprises contacting a Cloaked-2 polypeptide with a test molecule and determining the extent of binding of the test molecule to the polypeptide. The method further comprises determining whether such test molecules are agonists or antagonists of a Cloaked-2 polypeptide. The present invention further provides a method of testing the impact of molecules on the expression of Cloaked-2 polypeptide or on the activity of Cloaked-2 polypeptide.

Methods of regulating expression and modulating (*i.e.*, increasing or decreasing) levels of a Cloaked-2 polypeptide are also encompassed by the invention. One method comprises administering to an animal a nucleic acid molecule encoding a Cloaked-2 polypeptide. In another method, a nucleic acid molecule comprising elements that regulate or modulate the expression of a Cloaked-2 polypeptide may be administered. Examples of these methods include gene therapy, cell therapy, and anti-sense therapy as further described herein.

In another aspect of the present invention, the Cloaked-2 polypeptides may be used for identifying receptors thereof ("Cloaked-2 receptors"). Various forms of "expression cloning" have been extensively used for cloning receptors for protein ligands. See for example, H. Simonsen and H.F. Lodish, Trends in Pharmacological Sciences, vol. 15, 437-441 (1994), and Tartaglia et al., Cell, 83:1263-1271 (1995). The isolation of the Cloaked-2 receptor(s) is useful for identifying or developing novel agonists and antagonists of the Cloaked-2 polypeptide-signaling pathway. Such agonists and antagonists include soluble Cloaked-2 receptor(s), anti-Cloaked-2 receptor selective binding agents (such as antibodies and derivatives thereof), small molecules, and antisense oligonucleotides, any of which can be used for treating one or more diseases or disorders, including those recited herein.

<u>Brief Description of the Figures</u>

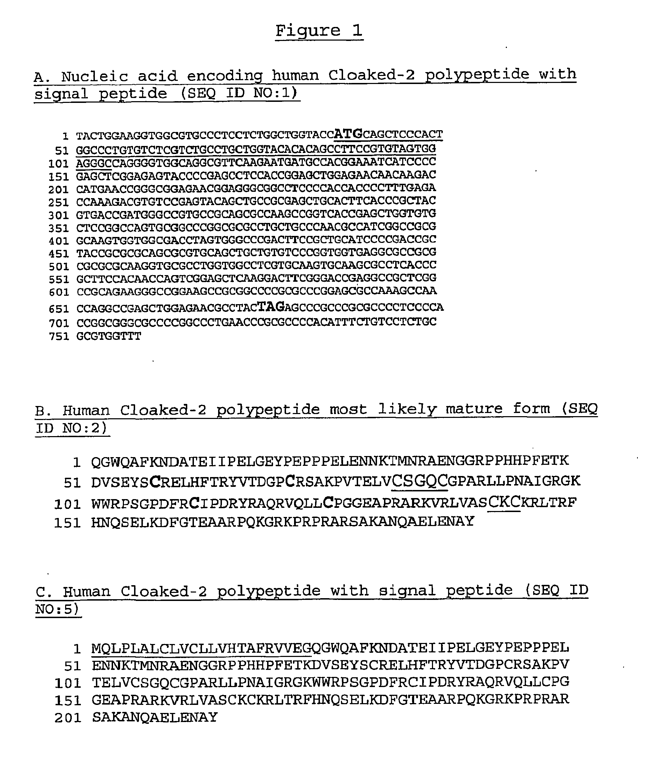

Figure 1A depicts the human Cloaked-2 cDNA sequence (SEQ ID NO:1) which encodes human Cloaked-2 polypeptide, including the predicted signal peptide. The start methionine codon (ATG) and the stop codon (TAG) are bolded and shown in larger font. The nucleotide sequence encoding the predicted signal peptide region is underlined.

Figure 1B depicts the amino acid sequence (SEQ ID NO:2) of the likely mature form *(i.e.,* predicted signal peptide cleaved off) of the human Cloaked-2 polypeptide. The asparagine (N) residues at positions 30 and 152 are located within classic NxS/T glycosylation motifs and are very likely to be glycosylated. The classic cystine-knot motifs, CxGxC and CxC, are shown in larger font and underlined. The four additional cystines are shown in larger font and bolded. The cystines in the CxGxC and CxC motifs represent cystines number 2 and 3, and, respectively, 5 and 6 of the six cystines that form the cystine-knot. Cystine number 1 of the cystine-knot would be either cystine 57 or 71 for human Cloaked-2. Cystine number 4 of the cystine-knot would be either cystine 111 or 125 for human Cloaked-2. For all known cystine-knot polypeptides the six cystines form 3 disulfide bonds in the following pairings: cystine number 1 and 4, cystine number 2 and 5, cystine number 3 and 6.

Figure 1C shows the full coding region of the human Cloaked-2 polypeptide (SEQ ID NO:5). The predicted signal peptide is underlined.

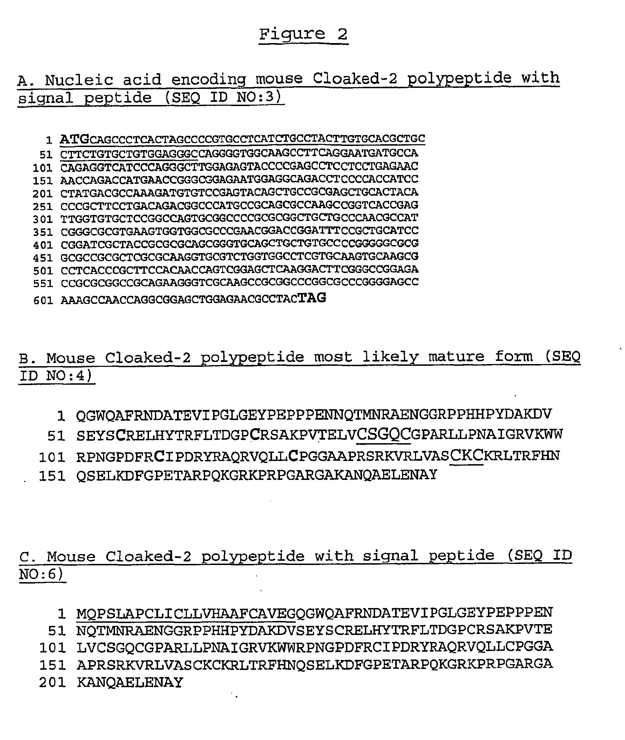

Figure 2A shows the mouse Cloaked-2 cDNA sequence (SEQ ID NO:3) which encodes mouse Cloaked-2 polypeptide including the predicted signal peptide. The start methionine codon (ATG) and the stop codon (TAG) are shown in larger font and bolded. The nucleotide sequence encoding the predicted signal peptide region is underlined.

Figure 2B depicts the amino acid sequence (SEQ ID NO:4) of the likely mature form (*i.e.*, predicted signal peptide cleaved off) of mouse Cloaked-2. The asparagine (N) residues at positions 28 and 150 are located within classic NxS/T glycosylation motifs and are very likely to be glycosylated. The classic cystine-knot motifs, CxGxC and CxC, are shown in larger font and underlined. The four additional cystines are shown in larger font and bolded. The cystines in the CxGxC and CxC motifs represent cystines number 2 and 3, and, respectively, 5 and 6 of the six cystines that form the cystine-knot. Cystine number 1 of the cystine-knot would be either cystine 55 or 69 for mouse Cloaked-2. Cystine number 4 of the cystine-knot would be either cystine 109 or 123 for mouse Cloaked-2. For all known cystine-knot polypeptides the six cystines form 3 disulfide bonds in the following pairings: cystine number 1 and 4, cystine number 2 and 5, cystine number 3 and 6.

Figure 2C depicts the full coding region of the mouse Cloaked-2 polypeptide (SEQ ID NO:6). The predicted signal peptide is underlined.

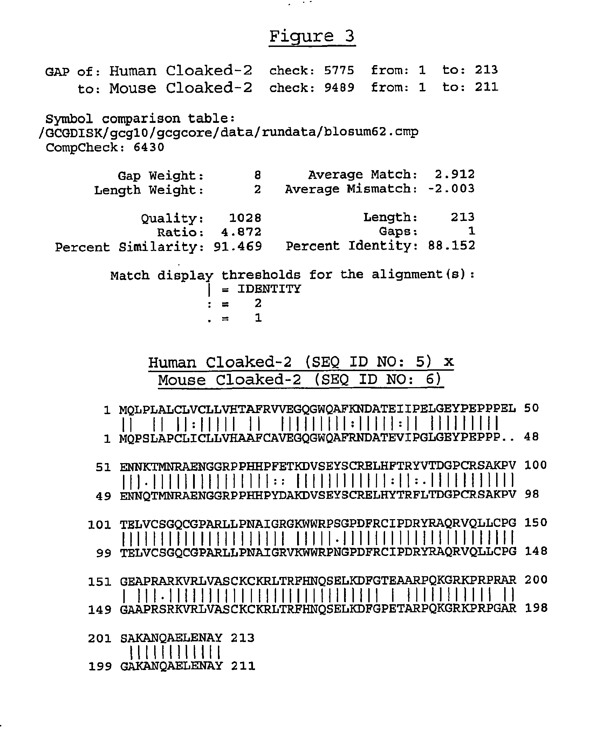

Figure 3 depicts the significant degree of homology (by GAP analysis) between the human and mouse Cloaked-2 polypeptides of SEQ ID NO:5 and SEQ ID NO:6.

Figure 4 depicts the significant degree of homology (by GAP analysis) between the most likely mature forms (*i.e.*, signal peptide cleaved off) of the human Cloaked-1 polypeptide (SEQ ID NO:25) and the human Cloaked-2 polypeptide (SEQ ID NO:2). The amino acid sequence of human Cloaked-1 can be found in *e.g.*, U.S. Patent No. 5,780,263 issued July 14, 1998 to Hastings, et al. The classic cystine-knot motifs, CxGxC and CxC, are conserved and are shown underlined. The four additional conserved cystines are shown in larger bold font.

Detailed Description of the Invention

The section headings used herein are for organizational purposes only and are not to be construed as limiting the subject matter described. All references cited in this application are expressly incorporated by reference herein.

Definitions

The terms "Cloaked-2 gene" or "Cloaked-2 nucleic acid molecule" or "polynucleotide" refers to a nucleic acid molecule comprising or consisting of a nucleotide sequence as set forth in SEQ ID NO:1 or SEQ ID NO:3, a nucleotide sequence encoding the polypeptide as set forth in SEQ ID NO:2 or SEQ ID NO:4, the nucleotide sequences of the DNA inserts in ATCC deposit no. PTA-1616 or PTA-1615, and nucleic acid molecules as defined herein.

The term "Cloaked-2 polypeptide" refers to a polypeptide comprising the amino acid sequence of SEQ ID NO: 2 or SEQ ID NO:4, and related polypeptides. Related polypeptides include: Cloaked-2 polypeptide allelic variants, Cloaked-2 polypeptide orthologs, Cloaked-2 polypeptide splice variants, Cloaked-2 polypeptide variants and Cloaked-2 polypeptide derivatives. Cloaked-2 polypeptides may be mature polypeptides, as defined herein, and may or may not have an amino terminal methionine residue, depending on the method by which they are prepared.

The term "Cloaked-2 polypeptide allelic variant" refers to one of several possible naturally occurring alternate forms of a gene occupying a given locus on a chromosome of an organism or a population of organisms.

The term "Cloaked-2 polypeptide derivatives" refers to the polypeptide as set forth in SEQ ID NO:2 or SEQ ID NO:4, Cloaked-2 polypeptide allelic variants, Cloaked-2 polypeptide orthologs, Cloaked-2 polypeptide splice variants, or Cloaked-2 polypeptide variants, as defined herein, that have been chemically modified.

The term "Cloaked-2 polypeptide fragment" refers to a polypeptide that comprises a truncation at the amino terminus (with or without a leader sequence) and/or a truncation at the carboxy terminus of the polypeptide as set forth in SEQ ID NO:2 or SEQ ID NO:4, Cloaked-2 polypeptide allelic variants, Cloaked-2 polypeptide orthologs, Cloaked-2 polypeptide splice variants and/or a Cloaked-2 polypeptide variant having one or more amino acid additions or substitutions or internal deletions (wherein the resulting polypeptide is at least 6 amino acids or more in length) as compared to the Cloaked-2 polypeptide amino acid sequence set forth in SEQ ID NO:2 or SEQ ID NO:4. Cloaked-2 polypeptide fragments may result from alternative RNA splicing or from *in vivo* protease activity. In preferred embodiments, truncations comprise about 10 amino acids, or about 20 amino acids, or about 50 amino acids, or about 75 amino acids, or about 100 amino acids, or more than about 100 amino acids. The polypeptide fragments so produced will comprise about 25 contiguous amino acids, or about 50 amino acids, or about 75 amino acids, or about 100 amino acids, or about 150 amino acids, or about 200 amino acids. Such Cloaked-2 polypeptide fragments may optionally comprise an amino terminal methionine residue. It will be appreciated that such fragments can be used, for example, to generate antibodies to Cloaked-2 polypeptides.

The term "Cloaked-2 fusion polypeptide" refers to a fusion of one or more amino acids (such as a heterologous peptide or polypeptide) at the amino or carboxy terminus of the polypeptide as set forth in SEQ ID NO:2 or SEQ ID NO: 4, Cloaked-2 polypeptide allelic variants, Cloaked-2 polypeptide orthologs, Cloaked-2 polypeptide splice variants, or Cloaked-2 polypeptide variants having one or more amino acid deletions, substitutions or internal additions as compared to the Cloaked-2 polypeptide amino acid sequence set forth in SEQ ID NO:2 or SEQ ID NO:4.

The term "Cloaked-2 polypeptide ortholog" refers to a polypeptide from another species that corresponds to Cloaked-2 polypeptide amino acid sequence as set forth in SEQ ID NO:2 or SEQ ID NO:4. For example, mouse and human Cloaked-2 polypeptides are considered orthologs of each other.

The term "Cloaked-2 polypeptide splice variant" refers to a nucleic acid molecule, usually RNA, which is generated by alternative processing of intron sequences in an RNA transcript of Cloaked-2 polypeptide amino acid sequence as set forth in SEQ ID NO:2 or SEQ ID NO:4.

The term "Cloaked-2 polypeptide variants" refers to Cloaked-2 polypeptides comprising amino acid sequences having one or more amino acid sequence substitutions, deletions (such as internal deletions and/or Cloaked-2 polypeptide fragments), and/or additions (such as internal additions and/or Cloaked-2 fusion polypeptides) as compared to the Cloaked-2 polypeptide amino acid sequence set forth in SEQ ID NO:2 or SEQ ID NO:4 (with or without a leader sequence). Variants may be naturally occurring (*e.g.*, Cloaked-2 polypeptide allelic variants, Cloaked-2 polypeptide orthologs and Cloaked-2 polypeptide splice variants) or artificially constructed. Such Cloaked-2 polypeptide variants may be prepared from the corresponding nucleic acid molecules having a DNA sequence that varies accordingly from the DNA sequence as set forth in SEQ ID NO:1 or SEQ ID NO:3. In preferred embodiments, the variants have from 1 to 3, or from 1 to 5, or from 1 to 10, or from 1 to 15, or from 1 to 20, or from 1 to 25, or from 1 to 50, or from 1 to 75, or from 1 to 100, or more than 100 amino acid substitutions, insertions, additions and/or deletions, wherein the substitutions may be conservative, or non-conservative, or any combination thereof.

The term "antigen" refers to a molecule or a portion of a molecule capable of being bound by a selective binding agent, such as an antibody, and additionally capable of being used in an animal to produce antibodies capable of binding to an epitope of that antigen. An antigen may have one or more epitopes.

The term "biologically active Cloaked-2 polypeptides" refers to Cloaked-2 polypeptides having at least one activity characteristic of the polypeptide comprising the amino acid sequence of SEQ ID NO:2 or SEQ ID NO:4.

The terms "effective amount" and "therapeutically effective amount" each refer to the amount of a Cloaked-2 polypeptide or Cloaked-2 nucleic acid molecule used to support an observable level of one or more biological activities of the Cloaked-2 polypeptides as set forth herein.

The term "expression vector" refers to a vector which is suitable for use in a host cell and contains nucleic acid sequences which direct and/or control the expression of heterologous nucleic acid sequences. Expression includes, but is not limited to, processes such as transcription, translation, and RNA splicing, if introns are present.

The term "host cell" is used to refer to a cell which has been transformed, or is capable of being transformed with a nucleic acid sequence and then of expressing a selected gene of interest. The term includes the progeny of the parent cell, whether or not the progeny is identical in morphology or in genetic make-up to the original parent, so long as the selected gene is present.

The term "identity" as known in the art, refers to a relationship between the sequences of two or more polypeptide molecules or two or more nucleic acid molecules, as determined by comparing the sequences. In the art, "identity" also means the degree of sequence relatedness between nucleic acid molecules or polypeptides, as the case may be, as determined by the match between strings of two or more nucleotide or two or more amino acid sequences. "Identity" measures the percent of identical matches between the smaller of two or more sequences with gap alignments (if any) addressed by a particular mathematical model or computer program (*i.e.,* "algorithms").

The term "similarity" is a related concept, but in contrast to "identity", refers to a measure of similarity which includes both identical matches and conservative substitution matches. If two polypeptide sequences have, for example, 10/20 identical amino acids, and the remainder are all non-conservative substitutions, then the percent identity and similarity would both be 50%. If in the same example, there are 5 more positions where there are conservative substitutions, then the percent identity remains 50%, but the per cent similarity would be 75% (15/20). Therefore, in cases where there are conservative substitutions, the degree of similarity between two polypeptides will be higher than the percent identity between those two polypeptides.

The term "isolated nucleic acid molecule" refers to a nucleic acid molecule of the invention that (1) has been separated from at least about 50 percent of proteins, lipids, carbohydrates or other materials with which it is naturally found when total DNA is isolated from the source cells, (2) is not linked to all or a portion of a polynucleotide to which the "isolated nucleic acid molecule" is linked in nature, (3) is operably linked to a polynucleotide which it is not linked to in nature, or (4) does not occur in nature as part of a larger polynucleotide sequence. Preferably, the isolated nucleic acid molecule of the present invention is substantially free from any other contaminating nucleic acid molecule (s) or other contaminants that are found in its natural environment that would interfere with its use in polypeptide production or its therapeutic, diagnostic, prophylactic or research use.

The term "isolated polypeptide" refers to a polypeptide of the present invention that (1) has been separated from at least about 50 percent of polynucleotides, lipids, carbohydrates or other materials with which it is naturally found when isolated from the source cell, (2) is not linked (by covalent or noncovalent interaction) to all or a portion of a polypeptide to which the "isolated polypeptide" is linked in nature, (3) is operably linked (by covalent or noncovalent interaction) to a polypeptide with which it is not linked in nature, or (4) does not occur in nature. Preferably, the isolated polypeptide is substantially free from any other contaminating polypeptides or other contaminants that are found in its natural environment that would interfere with its therapeutic, diagnostic, prophylactic or research use.

The term "mature Cloaked-2 polypeptide" refers to a Cloaked-2 polypeptide lacking a leader sequence. A mature Cloaked-2 polypeptide may also include other modifications such as proteolytic processing of the amino terminus (with or without a leader sequence) and/or the carboxy terminus, cleavage of a smaller polypeptide from a larger precursor, N-linked and/or O-linked glycosylation, and the like. An exemplary mature human Cloaked-2 polypeptide is depicted by SEQ ID NO:2. An exemplary mature mouse Cloaked-2 polypeptide is depicted by SEQ ID NO:4.

The term "nucleic acid sequence" or "nucleic acid molecule" refers to a DNA or RNA sequence. The term encompasses molecules formed from any of the known base analogs of DNA and RNA such as, but not limited to 4-acetylcytosine, 8-hydroxy-N6-methyladenosine, aziridinyl-cytosine, pseudoisocytosine, 5-(carboxyhydroxylmethyl) uracil, 5-fluorouracil, 5-bromouracil, 5-carboxymethylaminomethyl-2-thiouracil, 5-carboxy-methylaminomethyluracil, dihydrouracil, inosine, N6-iso-pentenyladenine, 1-methyladenine, 1-methylpseudouracil, 1-methylguanine, 1-methylinosine, 2,2-dimethyl-guanine, 2-methyladenine, 2-methylguanine, 3-methylcytosine, 5-methylcytosine, N6-methyladenine, 7-methylguanine, 5-methylaminomethyluracil, 5-methoxyamino-methyl-2-thiouracil, beta-D-mannosylqueosine, 5' -methoxycarbonyl-methyluracil, 5-methoxyuracil, 2-methylthio-N6-isopentenyladenine, uracil-5-oxyacetic acid methylester, uracil-5-oxyacetic acid, oxybutoxosine, pseudouracil, queosine, 2-thiocytosine, 5-methyl-2-thiouracil, 2-thiouracil, 4-thiouracil, 5-methyluracil, N-uracil-5-oxyacetic acid methylester, uracil-5-oxyacetic acid, pseudouracil, queosine, 2-thiocytosine, and 2,6-diaminopurine.

The term "naturally occurring" or "native" when used in connection with biological materials such as nucleic acid molecules, polypeptides, host cells, and the like, refers to materials which are found in nature and are not manipulated by man. Similarly, "non-naturally occurring" or "non-native" as used herein refers to a material that is not found in nature or that has been structurally modified or synthesized by man.

The term "operably linked" is used herein to refer to an arrangement of flanking sequences wherein the flanking sequences so described are configured or assembled so as to perform their usual function. Thus, a flanking sequence operably linked to a coding sequence may be capable of effecting the replication, transcription and/or translation of the coding sequence. For example, a coding sequence is operably linked to a promoter when the promoter is capable of directing transcription of that coding sequence. A flanking sequence need not be contiguous with the coding sequence, so long as it functions correctly. Thus, for example, intervening untranslated yet transcribed sequences can be present between a promoter sequence and the coding sequence and the promoter sequence can still be considered "operably linked" to the coding sequence.

The term "pharmaceutically acceptable carrier" or "physiologically acceptable carrier" as used herein refers to one or more formulation materials suitable for accomplishing or enhancing the delivery of the Cloaked-2 polypeptide, Cloaked-2 nucleic acid molecule or Cloaked-2 selective binding agent as a pharmaceutical composition.

The term "selective binding agent" refers to a molecule or molecules having specificity for a Cloaked-2 polypeptide. As used herein, the terms, "specific" and "specificity" refer to the ability of the selective binding agents to bind to human Cloaked-2 polypeptides and not to bind to human non-Cloaked-2 polypeptides. It will be appreciated, however, that the selective binding agents may also bind orthologs of the polypeptide as set forth in SEQ ID NO:2 or SEQ ID NO:4, that is, interspecies versions thereof, such as mouse and rat polypeptides.

The term "transduction" is used to refer to the transfer of genes from one bacterium to another, usually by a phage. "Transduction" also refers to the acquisition and transfer of eukaryotic cellular sequences by retroviruses.

The term "transfection" is used to refer to the uptake of foreign or exogenous DNA by a cell, and a cell has been "transfected" when the exogenous DNA has been introduced inside the cell membrane. A number of transfection techniques are well known in the art and are disclosed herein. See, for example, Graham et al., Virology, 52:456 (1973); Sambrook et al., Molecular Cloning, a laboratory Manual, Cold Spring Harbor Laboratories (New York, 1989); Davis et al., Basic Methods in Molecular Biology, Elsevier, 1986; and Chu et al., Gene, 13:197 (1981). Such techniques can be used to introduce one or more exogenous DNA moieties into suitable host cells.

The term "transformation" as used herein refers to a change in a cell's genetic characteristics, and a cell has been transformed when it has been modified to contain a new DNA. For example, a cell is transformed where it is genetically modified from its native state. Following transfection or transduction, the transforming DNA may recombine with that of the cell by physically integrating into a chromosome of the cell, may be maintained transiently as an episomal element without being replicated, or may replicate independently as a plasmid. A cell is considered to have been stably transformed when the DNA is replicated with the division of the cell.

The term "vector" is used to refer to any molecule (*e.g.*, nucleic acid, plasmid, or virus) used to transfer coding information to a host cell.

Relatedness of Nucleic Acid Molecules and/or Polypeptides

It is understood that related nucleic acid molecules include allelic or splice variants of the nucleic acid molecule of SEQ ID NO:1 or SEQ ID NO:3, and include sequences which are complementary to any of the above nucleotide sequences. Related nucleic acid molecules also include a nucleotide sequence encoding a polypeptide comprising or consisting essentially of a substitution, modification, addition and/or a deletion of one or more amino acid residues compared to the polypeptide in SEQ ID NO:2 or SEQ ID NO:4.

Fragments include molecules which encode a polypeptide of at least about 25 amino acid residues, or about 50, or about 75, or about 100, or greater than about 100 amino acid residues of the polypeptide of SEQ ID NO:2 or SEQ ID NO:4.

In addition, related Cloaked-2 nucleic acid molecules include those molecules which comprise nucleotide sequences which hybridize under moderately or highly stringent conditions as defined herein with the fully complementary sequence of the nucleic acid molecule of SEQ ID NO:1 or SEQ ID NO:3, or of a molecule encoding a polypeptide, which polypeptide comprises the amino acid sequence as shown in SEQ ID NO:2 or SEQ ID NO:4, or of a nucleic acid fragment as defined herein, or of a nucleic acid fragment encoding a polypeptide as defined herein. Hybridization probes may be prepared using the Cloaked-2 sequences provided herein to screen cDNA, genomic or synthetic DNA libraries for related sequences. Regions of the DNA and/or amino acid sequence of Cloaked-2 polypeptide that exhibit significant identity to known sequences are readily determined using sequence alignment algorithms as described herein and those regions may be used to design probes for screening.

The term "highly stringent conditions" refers to those conditions that are designed to permit hybridization of DNA strands whose sequences are highly complementary, and to exclude hybridization of significantly mismatched DNAs. Hybridization stringency is principally determined by temperature, ionic strength, and the concentration of denaturing agents such as formamide. Examples of "highly stringent conditions" for hybridization and washing are 0.015M sodium chloride, 0.0015M sodium citrate at 65-68°C or 0.015M sodium chloride, 0.0015M sodium citrate, and 50% formamide at 42°C. See Sambrook, Fritsch & Maniatis, Molecular Cloning: A Laboratory Manual, 2nd Ed., Cold Spring Harbor Laboratory, (Cold Spring Harbor, N.Y. 1989); Anderson et al., Nucleic Acid Hybridisation: a practical approach, Ch. 4, IRL Press Limited (Oxford, England).

More stringent conditions (such as higher temperature, lower ionic strength, higher formamide, or other denaturing agent) may also be used, however, the rate of hybridization will be affected. Other agents may be included in the hybridization and washing buffers for the purpose of reducing non-specific and/or background hybridization. Examples are 0.1% bovine serum albumin, 0.1% polyvinyl-pyrrolidone, 0.1% sodium pyrophosphate, 0.1% sodium dodecylsulfate ($NaDodSO_4$ or SDS), ficoll, Denhardt's solution, sonicated salmon sperm DNA (or other non-complementary DNA), and dextran sulfate, although other suitable agents can also be used. The concentration and types of these additives can be changed without substantially affecting the stringency of the hybridization conditions. Hybridization experiments are usually carried out at pH 6.8-7.4, however, at typical ionic strength conditions, the rate of hybridization is nearly independent of pH. See Anderson et al., Nucleic Acid Hybridisation: A Practical Approach, Ch. 4, IRL Press Limited (Oxford, England).

Factors affecting the stability of a DNA duplex include base composition, length, and degree of base pair mismatch. Hybridization conditions can be adjusted by one skilled in the art in order to accommodate these variables and allow DNAs of different sequence relatedness to form hybrids. The melting temperature of a perfectly matched DNA duplex can be estimated by the following equation:

$$T_m(^{\circ}C) = 81.5 + 16.6(\log[Na+]) + 0.41(\%G+C) - 600/N - 0.72(\%formamide)$$

where N is the length of the duplex formed, [Na+] is the molar concentration of the sodium ion in the hybridization or washing solution, %G+C is the percentage of (guanine+cytosine) bases in the hybrid. For imperfectly matched hybrids, the melting temperature is reduced by approximately 1°C for each 1% mismatch.

The term "moderately stringent conditions" refers to conditions under which a DNA duplex with a greater degree of base pair mismatching than could occur under "highly stringent conditions" is able to form. Examples of typical "moderately stringent conditions" are 0.015M sodium chloride, 0.0015M sodium citrate at 50-65°C or 0.015M sodium chloride, 0.0015M sodium citrate, and 20% formamide at 37-50°C. By way of example, a "moderately stringent" condition of 50°C in 0.015 M sodium ion will allow about a 21% mismatch.

It will be appreciated by those skilled in the art that there is no absolute distinction between "highly" and "moderately" stringent conditions. For example, at 0.015M sodium ion (no formamide), the melting temperature of perfectly matched long DNA is about 71°C. With a wash at 65°C (at the same ionic strength), this would allow for approximately a 6% mismatch. To capture more distantly related sequences, one skilled in the art can simply lower the temperature or raise the ionic strength.

A good estimate of the melting temperature in 1M NaCl* for oligonucleotide probes up to about 20nt is given by:

$$Tm = 2^{\circ}C \text{ per A-T base pair} + 4^{\circ}C \text{ per G-C base pair}$$

*The sodium ion concentration in 6X salt sodium citrate (SSC) is 1M. See Suggs et al., Developmental Biology Using Purified Genes, p. 683, Brown and Fox (eds.) (1981).

High stringency washing conditions for oligonucleotides are usually at a temperature of 0-5°C below the Tm of the oligonucleotide in 6X SSC, 0.1% SDS.

In another embodiment, related nucleic acid molecules comprise or consist of a nucleotide sequence that is about 70 percent identical to the nucleotide sequence as shown in SEQ ID N0:1 or SEQ ID N0:3, or comprise or consist essentially of a nucleotide sequence encoding a polypeptide that is about 70 percent identical to the polypeptide as set forth in SEQ ID NO:2 or SEQ ID NO:4. In preferred embodiments, the nucleotide sequences are about 75 percent, or about 80 percent, or about 85 percent, or about 90 percent, or about 95, 96, 97, 98, or 99 percent identical to the nucleotide sequence as shown in SEQ ID NO:1 or SEQ ID NO:3, or the nucleotide sequences encode a polypeptide that is about 75 percent, or about 80 percent, or about 85 percent, or about 90 percent, or about 95, 96, 97, 98, or 99 percent identical to the polypeptide sequence as set forth in SEQ ID NO:2 or SEQ ID NO:4.

Differences in the nucleic acid sequence may result in conservative and/or non-conservative modifications of the amino acid sequence relative to the amino acid sequence of SEQ ID NO:2 or SEQ ID NO:4.

Conservative modifications to the amino acid sequence of SEQ ID NO:2 or SEQ ID NO:4 (and the corresponding modifications to the encoding nucleotides) will produce Cloaked-2 polypeptides having functional and chemical characteristics similar to those of naturally occurring Cloaked-2 polypeptide. In contrast, substantial modifications in the functional and/or chemical characteristics of Cloaked-2 polypeptides may be accomplished by selecting substitutions in the amino acid sequence of SEQ ID NO:2 or SEQ ID NO:4 that differ significantly in their effect on maintaining (a) the structure of the molecular backbone in the area of the substitution, for example, as a sheet or helical conformation, (b) the charge or hydrophobicity of the molecule at the target site, or (c) the bulk of the side chain.

For example, a "conservative amino acid substitution" may involve a substitution of a native amino acid residue with a nonnative residue such that there is little or no effect on the polarity or charge of the amino acid residue at that position. Furthermore, any native residue in the polypeptide may also be substituted with alanine, as has been previously described for "alanine scanning mutagenesis."

Conservative amino acid substitutions also encompass non-naturally occurring amino acid residues which are typically incorporated by chemical peptide synthesis rather than by synthesis in biological systems. These include peptidomimetics, and other reversed or inverted forms of amino acid moieties.

Naturally occurring residues may be divided into classes based on common side chain properties:

1) hydrophobic: norleucine, Met, Ala, Val, Leu, Ile;
2) neutral hydrophilic: Cys, Ser, Thr, Asn, Gln;
3) acidic: Asp, Glu;
4) basic: His, Lys, Arg;
5) residues that influence chain orientation: Gly, Pro; and
6) aromatic: Trp, Tyr, Phe.

For example, non-conservative substitutions may involve the exchange of a member of one of these classes for a member from another class. Such substituted residues may be introduced into regions of the human Cloaked-2 polypeptide that are homologous with non-human Cloaked-2 polypeptide orthologs, or into the non-homologous regions of the molecule.

In making such changes, the hydropathic index of amino acids may be considered. Each amino acid has been assigned a hydropathic index on the basis of their hydrophobicity and charge characteristics, these are: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5).

The importance of the hydropathic amino acid index in conferring interactive biological function on a protein is understood in the art. Kyte et al., J. Mol. Biol., 157:105-131 (1982). It is known that certain amino acids may be substituted for other amino acids having a similar hydropathic index or score and still retain a similar biological activity. In making changes based upon the hydropathic index, the substitution of amino acids whose hydropathic indices are within $\pm 2$ is preferred, those which are within $\pm 1$ are particularly preferred, and those within $\pm 0.5$ are even more particularly preferred.

It is also understood in the art that the substitution of like amino acids can be made effectively on the basis of hydrophilicity, particularly where the biologically functionally equivalent protein or peptide thereby created is intended for use in immunological embodiments, as in the present case. The greatest local average hydrophilicity of a protein, as governed by the hydrophilicity of its adjacent amino acids, correlates with its immunogenicity and antigenicity, *i.e.*, with a biological property of the protein.

The following hydrophilicity values have been assigned to amino acid residues: arginine (+3.0); lysine (+3.0); aspartate (+3.0 $\pm$ 1); glutamate (+3.0 $\pm$ 1); serine (+0.3); asparagine (+0.2); glutamine (+0.2); glycine (0); threonine (-0.4); proline (-0.5 $\pm$ 1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4). In making changes based upon similar hydrophilicity values, the substitution of amino acids whose hydrophilicity values are within $\pm 2$ is preferred, those which are within $\pm 1$ are particularly preferred, and those within $\pm 0.5$ are even more particularly preferred. One may also identify epitopes from primary amino acid sequences on the basis of hydrophilicity. These regions are also referred to as "epitopic core regions."

Desired amino acid substitutions (whether conservative or non-conservative) can be determined by those skilled in the art at the time such substitutions are desired. For example, amino acid substitutions can be used to identify important residues of the Cloaked-2 polypeptide, or to increase or decrease the affinity of the Cloaked-2 polypeptides described herein.

Exemplary amino acid substitutions are set forth in Table I.

Table I

| Amino Acid Substitutions | | |
|---|---|---|
| Original Residues | Exemplary Substitutions | Preferred Substitutions |
| Ala | Val, Leu, Ile | Val |
| Arg | Lys, Gln, Asn | Lys |
| Asn | Gln | Gln |
| Asp | Glu | Glu |
| Cys | Ser, Ala | Ser |
| Gln | Asn | Asn |
| Glu | Asp | Asp |
| Gly | Pro, Ala | Ala |

(continued)

| Amino Acid Substitutions | | |
|---|---|---|
| Original Residues | Exemplary Substitutions | Preferred Substitutions |
| His | Asn, Gln, Lys, Arg | Arg |
| Ile | Leu, Val, Met, Ala, Phe, Norleucine | Leu |
| Leu | Norleucine, Ile, Val, Met, Ala, Phe | Ile |
| Lys | Arg, 1,4 Diamino-butyric Acid, Gln, Asn | Arg |
| Met | Leu, Phe, Ile | Leu |
| Phe | Leu, Val, Ile, Ala, Tyr | Leu |
| Pro | Ala | Gly |
| Ser | Thr, Ala, Cys | Thr |
| Thr | Ser | Ser |
| Trp | Tyr, Phe | Tyr |
| Tyr | Trp, Phe, Thr, Ser | Phe |
| Val | Ile, Met, Leu, Phe, Ala, Norleucine | Leu |

A skilled artisan will be able to determine suitable variants of the polypeptide as set forth in SEQ ID NO:2 or SEQ ID NO:4 using well known techniques. For identifying suitable areas of the molecule that may be changed without destroying activity, one skilled in the art may target areas not believed to be important for activity. For example, when similar polypeptides with similar activities from the same species or from other species are known, one skilled in the art may compare the amino acid sequence of a Cloaked-2 polypeptide to such similar polypeptides. With such a comparison, one can identify residues and portions of the molecules that are conserved among similar polypeptides. It will be appreciated that changes in areas of a Cloaked-2 polypeptide that are not conserved relative to such similar polypeptides would be less likely to adversely affect the biological activity and/or structure of the Cloaked-2 polypeptide. One skilled in the art would also know that, even in relatively conserved regions, one may substitute chemically similar amino acids for the naturally occurring residues while retaining activity (conservative amino acid residue substitutions). Therefore, even areas that may be important for biological activity or for structure may be subject to conservative amino acid substitutions without destroying the biological activity or without adversely affecting the polypeptide structure.

Additionally, one skilled in the art can review structure-function studies identifying residues in similar polypeptides that are important for activity or structure. In view of such a comparison, one can predict the importance of amino acid residues in a Cloaked-2 polypeptide that correspond to amino acid residues that are important for activity or structure in similar polypeptides. One skilled in the art may opt for chemically similar amino acid substitutions for such predicted important amino acid residues of Cloaked-2 polypeptides.

One skilled in the art can also analyze the three-dimensional structure and amino acid sequence in relation to that structure in similar polypeptides. In view of that information, one skilled in the art may predict the alignment of amino acid residues of a Cloaked-2 polypeptide with respect to its three dimensional structure. One skilled in the art may choose not to make radical changes to amino acid residues predicted to be on the surface of the protein, since such residues may be involved in important interactions with other molecules. Moreover, one skilled in the art may generate test variants containing a single amino acid substitution at each desired amino acid residue. The variants can then be screened using activity assays know to those skilled in the art. Such variants could be used to gather information about suitable variants. For example, if one discovered that a change to a particular amino acid residue resulted in destroyed, undesirably reduced, or unsuitable activity, variants with such a change would be avoided. In other words, based on information gathered from such routine experiments, one skilled in the art can readily determine the amino acids where further substitutions should be avoided either alone or in combination with other mutations.

Cloaked-2 polypeptide analogs of the invention can be determined by comparing the amino acid sequence of Cloaked-2 polypeptide with related family members. Exemplary Cloaked-2 polypeptide related family members include, but are not limited to Cloaked-1. This comparison can be accomplished by using a Pileup alignment (Wisconsin GCG Program Package) or an equivalent (overlapping) comparison with multiple family members within conserved and non-conserved regions.

As shown in Figure 4, the predicted amino acid sequence of Cloaked-2 polypeptide (SEQ ID NO: 2) is aligned with human Cloaked-1 polypeptide (SEQ ID NO 25). Other Cloaked-2 polypeptide analogs can be determined using these or other methods known to those of skill in the art. These overlapping sequences provide guidance for conservative and non-conservative amino acid substitutions resulting in additional Cloaked-2 analogs. It will be appreciated that these amino acid substitutions can consist of naturally occurring or non-naturally occurring amino acids. For example, as depicted in Figure 4, alignment of these related polypeptides indicates that potential Cloaked-2 analogs may have the aspartic acid residue at position 9 of SEQ ID NO: 2 substituted with a glutamic acid; the glycine residue at position 39 of SEQ ID NO: 2 substituted with a proline, or alanine; the arginine residue at position 58 of SEQ ID NO: 2 substituted with a lysine, glutamine, or asparagine; the valine residue at position 81 of SEQ ID NO: 2 substituted with an isoleucine, methionine, leucine, phenylalanine, alanine, or norleucine; the tryptophan residue at position 102 of SEQ ID NO: 2 substituted with a tyrosine or phenylalanine; and the serine residue at position 154 of SEQ ID NO: 2 substituted with a threonine, or alanine.

A number of scientific publications have been devoted to the prediction of secondary structure. See Moult J., Curr. Op. in Biotech., 7(4):422-427 (1996), Chou et al., Biochemistry, 13(2):222-245 (1974); Chou et al., Biochemistry, 113 (2) :211-222 (1974); Chou et al., Adv. Enzymol. Relat. Areas Mol. Biol., 47:45-148 (1978); Chou et al., Ann. Rev. Biochem., 47:251-276 and Chou et al., Biophys. J., 26:367-384 (1979). Moreover, computer programs are currently available to assist with predicting secondary structure. One method of predicting secondary structure is based upon homology modeling. For example, two polypeptides or proteins which have a sequence identity of greater than 30%, or similarity greater than 40% often have similar structural topologies. The recent growth of the protein structural data base (PDB) has provided enhanced predictability of secondary structure, including the potential number of folds within a polypeptide's or protein's structure. *See* Holm et al., Nucl. Acid. Res., 27(1):244-247 (1999). It has been suggested (Brenner et al., Curr. Op. Struct. Biol., 7(3):369-376 (1997)) that there are a limited number of folds in a given polypeptide *or* protein and that once a critical number of structures have been resolved, structural prediction will gain dramatically in accuracy.

Additional methods of predicting secondary structure include "threading" (Jones, D., Curr. Opin. Struct. Biol., 7(3):377-87 (1997); Sippl et al., Structure, 4(1):15-9 (1996)), "profile analysis" (Bowie et al., Science, 253:164-170 (1991); Gribskov et al., Meth. Enzym., 183:146-159 (1990); Gribskov et al., Proc. Nat. Acad. Sci., 84(13):4355-4358 (1987)), and "evolutionary linkage" (See Home, *supra,* and Brenner, *supra*).

Preferred Cloaked-2 polypeptide variants include glycosylation variants wherein the number and/or type of glycosylation sites has been altered compared to the amino acid sequence set forth in SEQ ID NO:2 or SEQ ID NO:4. In one embodiment, Cloaked-2 polypeptide variants comprise a greater or a lesser number of N-linked glycosylation sites than the amino acid sequence set forth in SEQ ID NO:2 or SEQ ID NO:4. An N-linked glycosylation site is characterized by the sequence: Asn-X-Ser or Asn-X-Thr, wherein the amino acid residue designated as X may be any amino acid residue except proline. The substitution(s) of amino acid residues to create this sequence provides a potential new site for the addition of an N-linked carbohydrate chain. Alternatively, substitutions which eliminate this sequence will remove an existing N-linked carbohydrate chain. Also provided is a rearrangement of N-linked carbohydrate chains wherein one or more N-linked glycosylation sites (typically those that are naturally occurring) are eliminated and one or more new N-linked sites are created. Additional preferred Cloaked-2 variants include cysteine variants, wherein one or more cysteine residues are deleted from or substituted for another amino acid (*e.g.*, serine) as compared to the amino acid sequence set forth in SEQ ID NO:2 or SEQ ID NO:4. Cysteine variants are useful when Cloaked-2 polypeptides must be refolded into a biologically active conformation such as after the isolation of insoluble inclusion bodies. Cysteine variants generally have fewer cysteine residues than the native protein, and typically have an even number to minimize interactions resulting from unpaired cysteines.

In addition, the polypeptide comprising the amino acid sequence of SEQ ID NO:2 or SEQ ID NO:4, or a Cloaked-2 polypeptide variant may be fused to a homologous polypeptide to form a homodimer or to a heterologous polypeptide to form a heterodimer. Heterologous peptides and polypeptides include, but are not limited to: an epitope to allow for the detection and/or isolation of a Cloaked-2 fusion polypeptide; a transmembrane receptor protein or a portion thereof, such as an extracellular domain, or a transmembrane and intracellular domain; a ligand or a portion thereof which binds to a transmembrane receptor, protein; an enzyme or portion thereof which is catalytically active; a polypeptide or peptide which promotes oligomerization, such as a leucine zipper domain; a polypeptide or peptide which increases stability, such as an immunoglobulin constant region; and a polypeptide which has a therapeutic activity different from the polypeptide comprising the amino acid sequence as set forth in SEQ ID NO:2 or SEQ ID NO:4, or a Cloaked-2 polypeptide variant.

Fusions can be made either at the amino terminus or at the carboxy terminus of the polypeptide comprising the amino acid sequence set forth in SEQ ID NO:2 or SEQ ID NO:4, or a Cloaked-2 polypeptide variant. Fusions may be direct with no linker or adapter molecule or indirect using a linker or adapter molecule. A linker or adapter molecule may be one or more amino acid residues, typically up to about 20 to about 50 amino acid residues. A linker or adapter molecule may also be designed with a cleavage site for a DNA restriction endonuclease or for a protease to allow for the separation of the fused moieties. It will be appreciated that once constructed, the fusion polypeptides can be derivatized according to the methods described herein.

In a further embodiment of the invention, the polypeptide comprising the amino acid sequence of SEQ ID NO:

2 or SEQ ID NO:4, or a Cloaked-2 polypeptide variant is fused to one or more domains of an Fc region of human IgG. Antibodies comprise two functionally independent parts, a variable domain known as "Fab", which binds antigen, and a constant domain known as "Fc", which is involved in effector functions such as complement activation and attack by phagocytic cells. An Fc has a long serum half-life, whereas an Fab is short-lived. Capon et al., Nature, 337:525-31 (1989). When constructed together with a therapeutic protein, an Fc domain can provide longer half-life or incorporate such functions as Fc receptor binding, protein A binding, complement fixation and perhaps even placental transfer. *Id.* Table II summarizes the use of certain Fc fusions known in the art.

Table II

| Fc Fusion with Therapeutic Proteins | | | |
|---|---|---|---|
| **Form of Fc** | **Fusion partner** | **Therapeutic implications** | **Reference** |
| IgG1 | N-terminus of CD30-L | Hodgkin's disease; anaplastic lymphoma; T-cell leukemia | U.S. Patent No. 5,480,981 |
| Murine Fcy2a | IL-10 | anti-inflammatory; transplant rejection | Zheng et al. (1995), J. Immunol., 154: 5590-5600 |
| IgG1 | TNF receptor | septic shock | Fisher et al. (1996), N. Engl. J. Med., 334: 1697-1702; Van Zee et al., (1996), J. Immunol., 156 : 2221-2230 |
| IgG, IgA, IgM, or receptor IgE (excludin g the first domain) | TNF | inflammation, autoimmune disorders | U.S. Pat. No. 5,808,029, issued September 15, 1998 |
| IgG1 | CD4 receptor | AIDS | Capon et al. (1989), Nature 337: 525-531 |
| IgG1, IgG3 of IL-2 | N-terminus | anti-cancer, antiviral | Harvill et al. (1995), Immunotech., 1: 95-105 |
| IgG1 | C-terminus of OPG | osteoarthritis; bone density | WO 97/23614, published July 3, 1997 |
| IgG1 | N-terminus of leptin | anti-obesity | PCT/US 97/23183, filed December 11, 1997 |
| Human Ig Cγ1 | CTLA-4 | autoimmune disorders | Linsley (1991), J. Exp. Med., 174:561-569 |

In one example, all or a portion of the human IgG hinge, CH2 and CH3 regions may be fused at either the N-terminus or C-terminus of the Cloaked-2 polypeptides using methods known to the skilled artisan. The resulting Cloaked-2 fusion polypeptide may be purified by use of a Protein A affinity column. Peptides and proteins fused to an Fc region have been found to exhibit a substantially greater half-life *in vivo* than the unfused counterpart. Also, a fusion to an Fc region allows for dimerization/multimerization of the fusion polypeptide. The Fc region may be a naturally occurring Fc region, or may be altered to improve certain qualities, such as therapeutic qualities, circulation time, reduce aggregation, etc.

Identity and similarity of related nucleic acid molecules and polypeptides can be readily calculated by known methods. Such methods include, but are not limited to, those described in Computational Molecular Biology, Lesk, A.M., ed., Oxford University Press, New York, 1988; Biocomputing: Informatics and Genome Projects, Smith, D.W., ed., Academic Press, New York, 1993; Computer Analysis of Sequence Data, Part 1, Griffin, A.M., and Griffin, H.G., eds., Humana Press, New Jersey, 1994; Sequence Analysis in Molecular Biology, von Heinje, G., Academic Press, 1987; Sequence Analysis Primer, Gribskov, M. and Devereux, J., eds., M. Stockton Press, New York, 1991; and Carillo et al., SIAM J. Applied Math., 48:1073 (1988).

Preferred methods to determine identity and/or similarity are designed to give the largest match between the sequences tested. Methods to determine identity and similarity are described in publicly available computer programs. Preferred computer program methods to determine identity and similarity between two sequences include, but are not limited to, the GCG program package, including GAP (Devereux et al., Nucl. Acid. Res., 12:387 (1984); Genetics Computer Group, University of Wisconsin, Madison, WI), BLASTP, BLASTN, and FASTA (Altschul et al., J. Mol. Biol., 215: 403-410 (1990)). The BLASTX program is publicly available from the National Center for Biotechnology Information (NCBI) and other sources *(*BLAST Manual, Altschul et al. NCB/NLM/NIH Bethesda, MD 20894; Altschul *et al., supra).* The well known Smith Waterman algorithm may also be used to determine identity.

Certain alignment schemes for aligning two amino acid sequences may result in the matching of only a short region of the two sequences, and this small aligned region may have very high sequence identity even though there is no significant relationship between the two full length sequences. Accordingly, in a preferred embodiment, the selected alignment method (GAP program) will result in an alignment that spans at least 50 contiguous amino acids of the target polypeptide.

For example, using the computer algorithm GAP (Genetics Computer. Group, University of Wisconsin, Madison, WI), two polypeptides for which the percent sequence identity is to be determined are aligned for optimal matching of their respective amino acids (the "matched span", as determined by the algorithm). A gap opening penalty (which is calculated as 3X the average diagonal; the "average diagonal" is the average of the diagonal of the comparison matrix being used; the "diagonal" is the score or number assigned to each perfect amino acid match by the particular comparison matrix) and a gap extension penalty (which is usually 1/10 times the gap opening penalty), as well as a comparison matrix such as PAM 250 or BLOSUM 62 are used in conjunction with the algorithm. A standard comparison matrix (see Dayhoff et al., Atlas of Protein Sequence and Structure, vol. 5, supp.3 (1978) for the PAM 250 comparison matrix; Henikoff et al., Proc. Natl. Acad. Sci USA, 89:10915-10919 (1992) for the BLOSUM 62 comparison matrix) is also used by the algorithm.

Preferred parameters for a polypeptide sequence comparison include the following:

Algorithm: Needleman et al., J. Mol. Biol., 48:443-453 (1970);
Comparison matrix: BLOSUM 62 from Henikoff et al., Proc. Natl. Acad. Sci. USA, 89:10915-10919 (1992);
Gap Penalty: 12
Gap Length Penalty: 4
Threshold of Similarity: 0

The GAP program is useful with the above parameters. The aforementioned parameters are the default parameters for polypeptide comparisons (along with no penalty for end gaps) using the GAP algorithm.

Preferred parameters for nucleic acid molecule sequence comparisons include the following:

Algorithm: Needleman et al., J. Mol Biol., 48:443-453 (1970);
Comparison matrix: matches = +10, mismatch = 0
Gap Penalty: 50
Gap Length Penalty: 3

The GAP program is also useful with the above parameters. The aforementioned parameters are the default parameters for nucleic acid molecule comparisons.

Other exemplary algorithms, gap opening penalties, gap extension penalties, comparison matrices, thresholds of similarity, etc. may be used" including those set forth in the Program Manual, Wisconsin Package, Version 9, September, 1997. The particular choices to be made will be apparent to those of skill in the art and will depend on the specific comparison to be made, such as DNA to DNA, protein to protein, protein to DNA; and additionally, whether the comparison is between given pairs of sequences (in which case GAP or BestFit are generally preferred) or between one sequence and a large database of sequences (in which case FASTA or BLASTA are preferred).

Synthesis

It will be appreciated by those skilled in the art the nucleic acid and polypeptide molecules described herein may be produced by recombinant and other means.

Nucleic Acid Molecules

The nucleic acid molecules encode a polypeptide comprising the amino acid sequence of a Cloaked-2 polypeptide can readily be obtained in a variety of ways including, without limitation, chemical synthesis, cDNA or genomic library screening, expression library screening and/or PCR amplification of cDNA.

Recombinant DNA methods used herein are generally those set forth in Sambrook et al., Molecular Cloning: A Laboratory Manual, Cold Spring Harbor Laboratory Press, Cold Spring Harbor, NY (1989), and/or Ausubel et al., eds., Current Protocols in Molecular Biology, Green Publishers Inc. and Wiley and Sons, NY (1994). The present invention provides for nucleic acid molecules as described herein and methods for obtaining the molecules.

Where a gene encoding the amino acid sequence of a Cloaked-2 polypeptide has been identified from one species, all or a portion of that gene may be used as a probe to identify orthologs or related genes from the same species. The probes or primers may be used to screen cDNA libraries from various tissue sources believed to express the Cloaked-2 polypeptide. In addition, part or all of a nucleic acid molecule having the sequence as set forth in SEQ ID NO:1 or SEQ ID NO:3 may be used to screen a genomic library to identify and isolate a gene encoding the amino acid sequence of a Cloaked-2 polypeptide. Typically, conditions of moderate or high stringency will be employed for screening to minimize the number of false positives obtained from the screen.

Nucleic acid molecules encoding the amino acid sequence of Cloaked-2 polypeptides may also be identified by expression cloning which employs the detection of positive clones based upon a property of the expressed protein. Typically, nucleic acid libraries are screened by the binding of an antibody or other binding partner (*e.g.*, receptor or ligand) to cloned proteins which are expressed and displayed on a host cell surface. The antibody or binding partner is modified with a detectable label to identify those cells expressing the desired clone.

Recombinant expression techniques conducted in accordance with the descriptions set forth below may be followed to produce these polynucleotides and to express the encoded polypeptides. For example, by inserting a nucleic acid sequence which encodes the amino acid sequence of a Cloaked-2 polypeptide into an appropriate vector, one skilled in the art can readily produce large quantities of the desired nucleotide sequence. The sequences can then be used to generate detection probes or amplification primers. Alternatively, a polynucleotide encoding the amino acid sequence of a Cloaked-2 polypeptide can be inserted into an expression vector. By introducing the expression vector into an appropriate host, the encoded Cloaked-2 polypeptide may be produced in large amounts.

Another method for obtaining a suitable nucleic acid sequence is the polymerase chain reaction (PCR). In this method, cDNA is prepared from poly(A)$^+$ RNA or total RNA using the enzyme reverse transcriptase. Two primers, typically complementary to two separate regions of cDNA (oligonucleotides) encoding the amino acid sequence of a Cloaked-2 polypeptide, are then added to the cDNA along with a polymerase such as *Taq* polymerase, and the polymerase amplifies the cDNA region between the two primers.

Another means of preparing a nucleic acid molecule encoding the amino acid sequence of a Cloaked-2 polypeptide is chemical synthesis using methods well known to the skilled artisan such as those described by Engels et al., Angew. Chem. Intl. Ed., 28:716-734 (1989). These methods include, *inter alia,* the phosphotriester, phosphoramidite, and H-phosphonate methods for nucleic acid synthesis. A preferred method for such chemical synthesis is polymer-supported synthesis using standard phosphoramidite chemistry. Typically, the DNA encoding the amino acid sequence of a Cloaked-2 polypeptide will be several hundred nucleotides in length. Nucleic acids larger than about 100 nucleotides can be synthesized as several fragments using these methods. The fragments can then be ligated together to form the full length nucleotide sequence of a Cloaked-2 polypeptide. Usually, the DNA fragment encoding the amino terminus of the polypeptide will have an ATG, which encodes a methionine residue. This methionine may or may not be present on the mature form of the Cloaked-2 polypeptide, depending on whether the polypeptide produced in the host cell is designed to be secreted from that cell. Other methods known to the skilled artisan may be used as well.

In certain embodiments, nucleic acid variants contain codons which have been altered for the optimal expression of a Cloaked-2 polypeptide in a given host cell. Particular codon alterations will depend upon the Cloaked-2 polypeptide (s) and host cell(s) selected for expression. Such "codon optimization" can be carried out by a variety of methods, for example, by selecting codons which are preferred for use in highly expressed genes in a given host cell. Computer algorithms which incorporate codon frequency tables such as "Ecohigh.cod" for codon preference of highly expressed bacterial genes may be used and are provided by the University of Wisconsin Package Version 9.0, Genetics Computer Group, Madison, WI. Other useful codon frequency tables include "Celegans_high.cod", "Celegans_low.cod", "Drosophila_high.cod", "Human_high.cod", "Maize_high.cod", and "Yeast_high.cod".

<u>Vectors and Host Cells</u>

A nucleic acid molecule encoding the amino acid sequence of a Cloaked-2 polypeptide may be inserted into an appropriate expression vector using standard ligation techniques. The vector is typically selected to be functional in the particular host cell employed (*i.e.*, the vector is compatible with the host cell machinery such that amplification of the gene and/or expression of the gene can occur). A nucleic acid molecule encoding the amino acid sequence of a Cloaked-2 polypeptide may be amplified/expressed in prokaryotic, yeast, insect (baculovirus systems), and/or eukaryotic host cells. Selection of the host cell will depend in part on whether a Cloaked-2 polypeptide is to be post-translationally modified (*e.g.,* glycosylated and/or phosphorylated). If so, yeast, insect, or mammalian host cells are preferable. For a review of expression vectors, see Meth. Enz., v.185, D.V. Goeddel, ed. Academic Press Inc., San Diego, CA (1990).

Typically, expression vectors used in any of the host cells will contain sequences for plasmid maintenance and for cloning and expression of exogenous nucleotide sequences. Such sequences, collectively referred to as "flanking sequences" in certain embodiments will typically include one or more of the following nucleotide sequences: a promoter, one or more enhancer sequences, an origin of replication, a transcriptional termination sequence, a complete intron sequence containing a donor and acceptor splice site, a sequence encoding a leader sequence for polypeptide secretion, a ribosome binding site, a polyadenylation sequence, a polylinker region for inserting the nucleic acid encoding the polypeptide to be expressed, and a selectable marker element. Each of these sequences is discussed below.

Optionally, the vector may contain a "tag"-encoding sequence, *i.e.*, an oligonucleotide molecule located at the 5' or 3' end of the Cloaked-2 polypeptide coding sequence; the oligonucleotide sequence encodes polyHis (such as hexaHis), or other "tag" such as FLAG, HA (hemaglutinin Influenza virus) or *myc* for which commercially available antibodies exist. This tag is typically fused to the polypeptide upon expression of the polypeptide, and can serve as a means for affinity purification of the Cloaked-2 polypeptide from the host cell. Affinity purification can be accomplished, for example, by column chromatography using antibodies against the tag as an affinity matrix. Optionally, the tag can subsequently be removed from the purified Cloaked-2 polypeptide by various means such as using certain peptidases for cleavage.

Flanking sequences may be homologous (*i.e.*, from the same species and/or strain as the host cell), heterologous (*i.e.*, from a species other than the host cell species or strain), hybrid (*i.e.*, a combination of flanking sequences from more than one source) or synthetic, or the flanking sequences may be native sequences which normally function to regulate Cloaked-2 polypeptide expression. As such, the source of a flanking sequence may be any prokaryotic or eukaryotic organism, any vertebrate or invertebrate organism, or any plant, provided that the flanking sequence is functional in, and can be activated by, the host cell machinery.

The flanking sequences useful in the vectors of this invention may be obtained by any of several methods well known in the art. Typically, flanking sequences useful herein other than the Cloaked-2 gene flanking sequences will have been previously identified by mapping and/or by restriction endonuclease digestion and can thus be isolated from the proper tissue source using the appropriate restriction endonucleases. In some cases, the full nucleotide sequence of a flanking sequence may be known. Here, the flanking sequence may be synthesized using the methods described herein for nucleic acid synthesis or cloning.

Where all or only a portion of the flanking sequence is known, it may be obtained using PCR and/or by screening a genomic library with suitable oligonucleotide and/or flanking sequence fragments from the same or another species. Where the flanking sequence is not known, a fragment of DNA containing a flanking sequence may be isolated from a larger piece of DNA that may contain, for example, a coding sequence or even another gene or genes. Isolation may be accomplished by restriction endonuclease digestion to produce the proper DNA fragment followed by isolation using agarose gel purification, Qiagen® column chromatography (Chatsworth, CA), or other methods known to the skilled artisan. The selection of suitable enzymes to accomplish this purpose will be readily apparent to one of ordinary skill in the art.

An origin of replication is typically a part of those prokaryotic expression vectors purchased commercially, and the origin aids in the amplification of the vector in a host cell. Amplification of the vector to a certain copy number can, in some cases, be important for the optimal expression of a Cloaked-2 polypeptide. If the vector of choice does not contain an origin of replication site, one may be chemically synthesized based on a known sequence, and ligated into the vector. For example, the origin of replication from the plasmid pBR322 (Product No. 303-3s, New England Biolabs, Beverly, MA) is suitable for most Gram-negative bacteria and various origins (*e.g.*, SV40, polyoma, adenovirus, vesicular stomatitus virus (VSV) or papillomaviruses such as HPV or BPV) are useful for cloning vectors in mammalian cells. Generally, the origin of replication component is not needed for mammalian expression vectors (for example, the SV40 origin is often used only because it contains the early promoter).

A transcription termination sequence is typically located 3' of the end of a polypeptide coding region and serves to terminate transcription. Usually, a transcription termination sequence in prokaryotic cells is a G-C rich fragment followed by a poly T sequence. While the sequence is easily cloned from a library or even purchased commercially as part of a vector, it can also be readily synthesized using methods for nucleic acid synthesis such as those described herein.

A selectable marker gene element encodes a protein necessary for the survival and growth of a host cell grown in a selective culture medium. Typical selection marker genes encode proteins that (a) confer resistance to antibiotics or other toxins, *e.g.*, ampicillin, tetracycline, or kanamycin for prokaryotic host cells, (b) complement auxotrophic deficiencies of the cell; or (c) supply critical nutrients not available from complex media. Preferred selectable markers are the kanamycin resistance gene, the ampicillin resistance gene, and the tetracycline resistance gene. A neomycin resistance gene may also be used for selection in prokaryotic and eukaryotic host cells.

Other selection genes may be used to amplify the gene which will be expressed. Amplification is the process wherein genes which are in greater demand for the production of a protein critical for growth are reiterated in tandem within the chromosomes of successive generations of recombinant cells. Examples of suitable selectable markers for mammalian cells include dihydrofolate reductase (DHFR) and thymidine kinase. The mammalian cell transformants are placed under selection pressure which only the transformants are uniquely adapted to survive by virtue of the selection gene present in the vector. Selection pressure is imposed by culturing the transformed cells under conditions in which the concentration of selection agent in the medium is successively changed, thereby leading to the amplification of both the selection gene and the DNA that encodes a Cloaked-2 polypeptide. As a result, increased quantities of Cloaked-2 polypeptide are synthesized from the amplified DNA.

A ribosome binding site is usually necessary for translation initiation of mRNA and is characterized by a Shine-Dalgarno sequence (prokaryotes) or a Kozak sequence (eukaryotes). The element is typically located 3' to the promoter and 5' to the coding sequence of a Cloaked-2 polypeptide to be expressed. The Shine-Dalgarno sequence is varied but is typically a polypurine (*i.e.*, having a high A-G content). Many Shine-Dalgarno sequences have been identified, each of which can be readily synthesized using methods set forth herein and used in a prokaryotic vector.

A leader, or signal, sequence may be used to direct a Cloaked-2 polypeptide out of the host cell. Typically, a nucleotide sequence encoding the signal sequence is positioned in the coding region of a Cloaked-2 nucleic acid molecule, or directly at the 5' end of a Cloaked-2 polypeptide coding region. Many signal sequences have been identified, and any of those that are functional in the selected host cell may be used in conjunction with a Cloaked-2 nucleic acid molecule. Therefore, a signal sequence may be homologous (naturally occurring) or heterologous to a Cloaked-2 gene or cDNA. Additionally, a signal sequence may be chemically synthesized using methods described herein. In most cases, the secretion of a Cloaked-2 polypeptide from the host cell via the presence of a signal peptide will result in the removal of the signal peptide from the secreted Cloaked-2 polypeptide. The signal sequence may be a component of the vector, or it may be a part of a Cloaked-2 nucleic acid molecule that is inserted into the vector.

Included within the scope of this invention is the use of either a nucleotide sequence encoding a native Cloaked-2 polypeptide signal sequence joined to a Cloaked-2 polypeptide coding region or a nucleotide sequence encoding a heterologous signal sequence joined to a Cloaked-2 polypeptide coding region. The heterologous signal sequence selected should be one that is recognized and processed, *i.e.*, cleaved by a signal peptidase, by the host cell. For prokaryotic host cells that do not recognize and process the native Cloaked-2 polypeptide signal sequence, the signal sequence is substituted by a prokaryotic signal sequence selected, for example, from the group of the alkaline phosphatase, penicillinase, or heat-stable enterotoxin II leaders. For yeast secretion, the native Cloaked-2 polypeptide signal sequence may be substituted by the yeast invertase, alpha factor, or acid phosphatase leaders. In mammalian cell expression the native signal sequence is satisfactory, although other mammalian signal sequences may be suitable.

In some cases, such as where glycosylation is desired in a eukaryotic host cell expression system, one may manipulate the various presequences to improve glycosylation or yield. For example, one may alter the peptidase cleavage site of a particular signal peptide, or add presequences, which also may affect glycosylation. The final protein product may have, in the -1 position (relative to the first amino acid of the mature protein) one or more additional amino acids incident to expression, which may not have been totally removed. For example, the final protein product may have one or two amino acid residues found in the peptidase cleavage site, attached to the N-terminus. Alternatively, use of some enzyme cleavage sites may result in a slightly truncated form of the desired Cloaked-2 polypeptide, if the enzyme cuts at such area within the mature polypeptide.

In many cases, transcription of a nucleic acid molecule is increased by the presence of one or more introns in the vector; this is particularly true where a polypeptide is produced in eukaryotic host cells, especially mammalian host cells. The introns used may be naturally occurring within the Cloaked-2 gene, especially where the gene used is a full length genomic sequence or a fragment thereof. Where the intron is not naturally occurring within the gene (as for most cDNAs), the intron(s) may be obtained from another source. The position of the intron with respect to flanking sequences and the Cloaked-2 gene is generally important, as the intron must be transcribed to be effective. Thus, when a Cloaked-2 cDNA molecule is being transcribed, the preferred position for the intron is 3' to the transcription start site, and 5' to the polyA transcription termination sequence. Preferably, the intron or introns will be located on one side or the other (*i.e.*, 5' or 3') of the cDNA such that it does not interrupt the coding sequence. Any intron from any source, including any viral, prokaryotic and eukaryotic (plant or animal) organisms, may be used to practice this invention, provided that it is compatible with the host cell(s) into which it is inserted. Also included herein are synthetic introns. Optionally, more than one intron may be used in the vector.

The expression and cloning vectors of the present invention will each typically contain a promoter that is recognized by the host organism and operably linked to the molecule encoding a Cloaked-2 polypeptide. Promoters are untranscribed sequences located upstream (5') to the start codon of a structural gene (generally within about 100 to 1000 bp) that control the transcription of the structural gene. Promoters are conventionally grouped into one of two classes, inducible promoters and constitutive promoters. Inducible promoters initiate increased levels of transcription from DNA under their control in response to some change in culture conditions, such as the presence or absence of a nutrient or a change in temperature. Constitutive promoters, on the other hand, initiate continual gene product production; that is, there is little or no control over gene expression. A large number of promoters, recognized by a variety of potential host cells, are well known. A suitable promoter is operably linked to the DNA encoding a Cloaked-2 polypeptide by removing the promoter from the source DNA by restriction enzyme digestion and inserting the desired promoter sequence into the vector. The native Cloaked-2 gene promoter sequence may be used to direct amplification and/or expression of a Cloaked-2 nucleic acid molecule. A heterologous promoter is preferred, however, if it permits greater transcription and higher yields of the expressed protein as compared to the native promoter, and if it is compatible with the host cell system that has been selected for use.

Promoters suitable for use with prokaryotic hosts include the beta-lactamase and lactose promoter systems; alkaline phosphatase, a tryptophan (trp) promoter system; and hybrid promoters such as the tac promoter. Other known bacterial promoters are also suitable. Their sequences have been published, thereby enabling one skilled in the art to ligate them to the desired DNA sequence(s), using linkers or adapters as needed to supply any useful restriction sites.

Suitable promoters for use with yeast hosts are also well known in the art. Yeast enhancers are advantageously used with yeast promoters. Suitable promoters for use with mammalian host cells are well known and include, but are not limited to, those obtained from the genomes of viruses such as polyoma virus, fowlpox virus, adenovirus (such as Adenovirus 2), bovine papilloma virus, avian sarcoma virus, cytomegalovirus (CMV), a retrovirus, hepatitis-B virus and most preferably Simian Virus 40 (SV40). Other suitable mammalian promoters include heterologous mammalian promoters, *e.g.*, heat-shock promoters and the actin promoter.

Additional promoters which may be of interest in controlling Cloaked-2 gene transcription include, but are not limited to: the SV40 early promoter region (Bernoist and Chambon, Nature, 290:304-310, 1981); the CMV promoter; the promoter contained in the 3' long terminal repeat of Rous sarcoma virus (Yamamoto et al., Cell, 22:787-797, 1980); the herpes thymidine kinase promoter (Wagner et al., Proc. Natl. Acad. Sci. USA, 78:144-1445, 1981); the regulatory sequences of the metallothionine gene (Brinster et al., Nature, 296:39-42, 1982); prokaryotic expression vectors such as the beta-lactamase promoter (Villa-Kamaroff, et al., Proc. Natl. Acad. Sci. USA, 75:3727-3731, 1978); or the tac promoter (DeBoer, et al., Proc. Natl. Acad. Sci. USA, 80:21-25, 1983). Also of interest are the following animal transcriptional control regions, which exhibit tissue specificity and have been utilized in transgenic animals: the elastase I gene control region which is active in pancreatic acinar cells (Swift et al., Cell, 38:639-646, 1984; Ornitz et al., Cold Spring Harbor Symp. Quant. Biol., 50:399-409 (1986); MacDonald, Hepatology, 7:425-515, 1987); the insulin gene control region which is active in pancreatic beta cells (Hanahan, Nature, 315:115-122, 1985); the immunoglobulin gene control region which is active in lymphoid cells (Grosschedl et al., Cell, 38:647-658 (1984); Adames et al., Nature, 318:533-538 (1985); Alexander et al., Mol. Cell. Biol., 7:1436-1444, 1987); the mouse mammary tumor virus control region which is active in testicular, breast, lymphoid and mast cells (Leder et al., Cell, 45:485-495, 1986); the albumin gene control region which is active in liver (Pinkert et al., Genes and Devel., 1:268-276, 1987); the alphafetoprotein gene control region which is active in liver (Krumlauf et al., Mol. Cell. Biol., 5:1639-1648, 1985; Hammer et al., Science, 235:53-58, 1987); the alpha 1-antitrypsin gene control region which is active in the liver (Kelsey et al., Genes and Devel., 1:161-171, 1987); the beta-globin gene control region which is active in myeloid cells (Mogram et al., Nature, 315:338-340, 1985; Kollias et al., Cell, 46:89-94, 1986); the myelin basic protein gene control region which is active in oligodendrocyte cells in the brain (Readhead et al., Cell, 48:703-712, 1987); the myosin light chain-2 gene control region which is active in skeletal muscle (Sani, Nature, 314:283-286, 1985); and the gonadotropic releasing hormone gene control region which is active in the hypothalamus (Mason et al., Science, 234:1372-1378, 1986).

An enhancer sequence may be inserted into the vector to increase the transcription of a DNA encoding a Cloaked-2 polypeptide of the present invention by higher eukaryotes. Enhancers are cis-acting elements of DNA, usually about 10-300 bp in length, that act on the promoter to increase transcription. Enhancers are relatively orientation and position independent. They have been found 5' and 3' to the transcription unit. Several enhancer sequences available from mammalian genes are known (*e.g.*, globin, elastase, albumin, alpha-feto-protein and insulin). Typically, however, an enhancer from a virus will be used. The SV40 enhancer, the cytomegalovirus early promoter enhancer, the polyoma enhancer, and adenovirus enhancers are exemplary enhancing elements for the activation of eukaryotic promoters. While an enhancer may be spliced into the vector at a position 5' or 3' to a Cloaked-2 nucleic acid molecule, it is typically located at a site 5' from the promoter.

Expression vectors of the invention may be constructed from a starting vector such as a commercially available vector. Such vectors may or may not contain all of the desired flanking sequences. Where one or more of the desired flanking sequences are not already present in the vector, they may be individually obtained and ligated into the vector. Methods used for obtaining each of the flanking sequences are well known to one skilled in the art.

Preferred vectors for practicing this invention are those which are compatible with bacterial, insect, and mammalian host cells. Such vectors include, *inter alia.,* pCRII, pCR3, and pcDNA3.1 (Invitrogen Company, Carlsbad, CA), pBSII (Stratagene Company, La Jolla, CA), pET15 (Novagen, Madison, WI), pGEX (Pharmacia Biotech, Piscataway, NJ), pEGFP-N2 (Clontech, Palo Alto, CA), pETL (BlueBacII; Invitrogen), pDSR-alpha (PCT Publication No. WO90/14363) and pFastBacDual (Gibco/BRL, Grand Island, NY).

Additional suitable vectors include, but are not limited to, cosmids, plasmids or modified viruses, but it will be appreciated that the vector system must be compatible with the selected host cell. Such vectors include, but are not limited to plasmids such as Bluescript plasmid derivatives (a high copy number ColE1-based phagemid, Stratagene Cloning Systems Inc., La Jolla CA), PCR cloning plasmids designed for cloning Taq-amplified PCR products (*e.g.*, TOPO™ TA Cloning® Kit, PCR2.1 plasmid derivatives, Invitrogen, Carlsbad, CA), and mammalian, yeast, or virus vectors such as a baculovirus expression system (pBacPAK plasmid derivatives, Clontech, Palo Alto, CA).

After the vector has been constructed and a nucleic acid molecule encoding a Cloaked-2 polypeptide has been inserted into the proper site of the vector, the completed vector may be inserted into a suitable host cell for amplification and/or polypeptide expression. The transformation of an expression vector for a Cloaked-2 polypeptide into a selected host cell may be accomplished by well known methods including methods such as transfection, infection, calcium chloride, electroporation, microinjection, lipofection or the DEAE-dextran method or other known techniques. The method selected will in part be a function of the type of host cell to be used. These methods and other suitable methods are well known to the skilled artisan, and are set forth, for example, in Sambrook *et al., supra.*

Host cells may.be prokaryotic host cells (such as *E. coli*) or eukaryotic host cells (such as a yeast cell, an insect cell or a vertebrate cell). The host cell, when cultured under appropriate conditions, synthesizes a Cloaked-2 polypeptide which can subsequently be collected from the culture medium (if the host cell secretes it into the medium) or directly from the host cell producing it (if it is not secreted). The selection of an appropriate host cell will depend upon various factors, such as desired expression levels, polypeptide modifications that are desirable or necessary for activity, such as glycosylation or phosphorylation, and ease of folding into a biologically active molecule.

A number of suitable host cells are known in the art and many are available from the American Type Culture Collection (ATCC), 10801 University Boulevard, Manassas, VA 20110-2209. Examples include, but are not limited to, mammalian cells, such as Chinese hamster ovary cells (CHO) (ATCC No. CCL61) CHO DHFR-cells (Urlaub et al., Proc. Natl. Acad. Sci. USA, 97:4216-4220 (1980)), human embryonic kidney (HEK) 293 or 293T cells (ATCC No. CRL1573), or 3T3 cells (ATCC No. CCL92). The selection of suitable mammalian host cells and methods for transformation, culture, amplification, screening and product production and purification are known in the art. Other suitable mammalian cell lines, are the monkey COS-1 (ATCC No. CRL1650) and COS-7 cell lines (ATCC No. CRL1651), and the CV-1 cell line (ATCC No. CCL70). Further exemplary mammalian host cells include primate cell lines and rodent cell lines, including transformed cell lines. Normal diploid cells, cell strains derived from *in vitro* culture of primary tissue, as well as primary explants, are also suitable. Candidate cells may be genotypically deficient in the selection gene, or may contain a dominantly acting selection gene. Other suitable mammalian cell lines include but are not limited to, mouse neuroblastoma N2A cells, HeLa, mouse L-929 cells, 3T3 lines derived from Swiss, Balb-c or NIH mice, BHK or HaK hamster cell lines, which are available from the ATCC. Each of these cell lines is known by and available to those skilled in the art of protein expression.

Similarly useful as host cells suitable for the present invention are bacterial cells. For example, the various strains of *E. coli* (*e.g.*, HB101, (ATCC No. 33694) DH5$\alpha$, DH10, and MC1061 (ATCC No. 53338)) are well-known as host cells in the field of biotechnology. Various strains of *B. subtilis, Pseudomonas spp.,* other *Bacillus spp., Streptomyces spp.*, and the like may also be employed in this method.

Many strains of yeast cells known to those skilled in the art are also available as host cells for the expression of the polypeptides of the present invention. Preferred yeast cells include, for example, *Saccharomyces cerivisae and Pichia pastoris.*

Additionally, where desired, insect cell systems may be utilized in the methods of the present invention. Such systems are described for example in Kitts et al., Biotechniques, 14:810-817 (1993); Lucklow, Curr. Opin. Biotechnol., 4:564-572 (1993); and Lucklow et al. (J. Virol., 67:4566-4579 (1993). Preferred insect cells are Sf-9 and Hi5 (Invitrogen, Carlsbad, CA).

One may also use transgenic animals to express glycosylated Cloaked-2 polypeptides. For example, one may use a transgenic milk-producing animal (a cow or goat, for example) and obtain the present glycosylated polypeptide in the animal milk. One may also use plants to produce Cloaked-2 polypeptides, however, in general, the glycosylation occurring in plants is different from that produced in mammalian cells, and may result in a glycosylated product which is not suitable for human therapeutic use.

Polypeptide Production

Host cells comprising a Cloaked-2 polypeptide expression vector may be cultured using standard media well known to the skilled artisan. The media will usually contain all nutrients necessary for the growth and survival of the cells. Suitable media for culturing *E. coli* cells include, for example, Luria Broth (LB) and/or Terrific Broth (TB). Suitable media for culturing eukaryotic cells include Roswell Park Memorial Institute medium 1640 (RPMI 1640), Minimal Essential Medium (MEM) and/or Dulbecco's Modified Eagle Medium (DMEM), all of which may be supplemented with serum and/or growth factors as indicated by the particular cell line being cultured. A suitable medium for insect cultures is Grace's medium supplemented with yeastolate, lactalbumin hydrolysate and/or fetal calf serum, as necessary.

Typically, an antibiotic or other compound useful for selective growth of transformed cells is added as a supplement to the media. The compound to be used will be dictated by the selectable marker element present on the plasmid with which the host cell was transformed. For example, where the selectable marker element is kanamycin resistance, the compound added to the culture medium will be kanamycin. Other compounds for selective growth include ampicillin, tetracycline, and neomycin.

The amount of a Cloaked-2 polypeptide produced by a host cell can be evaluated using standard methods known in the art. Such methods include, without limitation, Western blot analysis, SDS-polyacrylamide gel electrophoresis, non-denaturing gel electrophoresis, HPLC separation, immunoprecipitation, and/or activity assays such as DNA binding gel shift assays.

If a Cloaked-2 polypeptide has been designed to be secreted from the host cells, the majority of polypeptide may be found in the cell culture medium. If however, the Cloaked-2 polypeptide is not secreted from the host cells, it will be present in the cytoplasm and/or the nucleus (for eukaryotic host cells) or in the cytosol (for bacterial host cells).

For a Cloaked-2 polypeptide situated in the host cell, cytoplasm and/or the nucleus (for eukaryotic host cells) or in the cytosol (for bacterial host cells), intracellular material (including inclusion bodies for gram-negative bacteria) can be extracted from the host cell using any standard technique known to the skilled artisan. For example, the host cells can be lysed to release the contents of the periplasm/cytoplasm by French press, homogenization, and/or sonication followed by centrifugation.

If a Cloaked-2 polypeptide has formed inclusion bodies in the cytosol, the inclusion bodies can often bind to the inner and/or outer cellular membranes and thus will be found primarily in the pellet material after centrifugation. The pellet material can then be treated at pH extremes or with a chaotropic agent such as a detergent, guanidine, guanidine derivatives, urea, or urea derivatives in the presence of a reducing agent such as dithiothreitol at alkaline pH or tris carboxyethyl phosphine at acid pH to release, break apart, and solubilize the inclusion bodies. The Cloaked-2 polypeptide in its now soluble form can then be analyzed using gel electrophoresis, immunoprecipitation or the like. If it is desired to isolate the Cloaked-2 polypeptide, isolation may be accomplished using standard methods such as those described herein and in Marston et al., Meth. Enz., 182:264-275 (1990).

In some cases, a Cloaked-2 polypeptide may not be biologically active upon isolation. Various methods for "refolding" or converting the polypeptide to its tertiary structure and generating disulfide linkages can be used to restore biological activity. Such methods include exposing the solubilized polypeptide to a pH usually above 7 and in the presence of a particular concentration of a chaotrope. The selection of chaotrope is very similar to the choices used for inclusion body solubilization, but usually the chaotrope is used at a lower concentration and is not necessarily the same as chaotropes used for the solubilization. In most cases the refolding/oxidation solution will also contain a reducing agent or the reducing agent plus its oxidized form in a specific ratio to generate a particular redox potential allowing for disulfide shuffling to occur in the formation of the protein's cysteine bridge(s). Some of the commonly used redox couples include cysteine/cystamine, glutathione (GSH)/dithiobis GSH, cupric chloride, dithiothreitol(DTT)/ dithiane DTT, and 2-2mercaptoethanol(bME)/dithio-b(ME). A cosolvent may be used to increase the efficiency of the refolding, and the more common reagents used for this purpose include glycerol, polyethylene glycol of various molecular weights, arginine and the like.

If inclusion bodies are not formed to a significant degree upon expression of a Cloaked-2 polypeptide, then the polypeptide will be found primarily in the supernatant after centrifugation of the cell homogenate. The polypeptide may be further isolated from the supernatant using methods such as those described herein.

The purification of a Cloaked-2 polypeptide from solution can be accomplished using a variety of techniques. If the polypeptide has been synthesized such that it contains a tag such as Hexahistidine (Cloaked-2 polypeptide/hexaHis) or other small peptide such as FLAG (Eastman Kodak Co., New Haven, CT) or *myc* (Invitrogen, Carlsbad, CA) at either its carboxyl or amino terminus, it may be purified in a one-step process by passing the solution through an affinity column where the column matrix has a high affinity for the tag.

For example, polyhistidine binds with great affinity and specificity to nickel, thus an affinity column of nickel (such as the Qiagen® nickel columns) can be used for purification of Cloaked-2 polypeptide/polyHis. See for example, Ausubel et al., eds., Current Protocols in Molecular Biology, Section 10.11.8, John Wiley & Sons, New York (1993).

Additionally, the Cloaked-2 polypeptide may be purified through the use of a monoclonal antibody which is capable of specifically recognizing and binding to the Cloaked-2 polypeptide.

Suitable procedures for purification thus include, without limitation, affinity chromatography, immunoaffinity chromatography, ion exchange chromatography, molecular sieve chromatography, High Performance Liquid Chromatography (HPLC), electrophoresis (including native gel electrophoresis) followed by gel elution, and preparative isoelectric focusing ("Isoprime" machine/technique, Hoefer Scientific, San Francisco, CA). In some cases, two or more purification techniques may be combined to achieve increased purity.

Cloaked-2 polypeptides may also be prepared by chemical synthesis methods (such as solid phase peptide synthesis) using techniques known in the art, such as those set forth by Merrifield et al., J. Am. Chem. Soc., 85 2149 (1963), Houghten et al., Proc. Natl. Acad. Sci. USA, 82:5132 (1985), and Stewart and Young, Solid Phase Peptide Synthesis, Pierce Chemical Co., Rockford, IL (1984). Such polypeptides may be synthesized with or without a methionine on the amino terminus. Chemically synthesized Cloaked-2 polypeptides may be oxidized using methods set forth in these references to form disulfide bridges. Chemically synthesized Cloaked-2 polypeptides are expected to have comparable.biological activity to the corresponding Cloaked-2 polypeptides produced recombinantly or purified from natural sources, and thus may be used interchangeably with a recombinant or natural Cloaked-2 polypeptide.

Another means of obtaining a Cloaked-2 polypeptide is via purification from biological samples such as source tissues and/or fluids in which the Cloaked-2 polypeptide is naturally found. Such purification can be conducted using methods for protein purification as described herein. The presence of the Cloaked-2 polypeptide during purification may be monitored using, for example, an antibody prepared against recombinantly produced Cloaked-2 polypeptide or peptide fragments thereof.

A number of additional methods for producing nucleic acids and polypeptides are known in the art, and can be used to produce polypeptides having specificity for Cloaked-2. See for example, Roberts et al., Proc. Natl. Acad. Sci., 94:12297-12303 (1997), which describes the production of fusion proteins between an mRNA and its encoded peptide. *See also* Roberts, R., Curr. Opin. Chem. Biol., 3:-268-273 (1999). Additionally, U.S. patent No. 5,824,469 describes methods of obtaining oligonucleotides capable of carrying out a specific biological function. The procedure involves generating a heterogeneous pool of oligonucleotides, each having a 5' randomized sequence, a central preselected sequence, and a 3' randomized sequence. The resulting heterogeneous pool is introduced into a population of cells that do not exhibit the desired biological function. Subpopulations of the cells are then screened for those which exhibit a predetermined biological function. From that subpopulation, oligonucleotides capable of carrying out the desired biological function are isolated.

U.S. Patent Nos. 5,763,192, 5,814,476, 5,723,323, and 5,817,483 describe processes for producing peptides or polypeptides. This is done by producing stochastic genes or fragments thereof, and then introducing these genes into host cells which produce one or more proteins encoded by the stochastic genes. The host cells are then screened to identify those clones producing peptides or polypeptides having the desired activity.

Chemical Derivatives

Chemically modified derivatives of the Cloaked-2 polypeptides may be prepared by one skilled in the art, given the disclosures set forth hereinbelow. Cloaked-2 polypeptide derivatives are modified in a manner that is different, either in the type or location of the molecules naturally attached to the polypeptide. Derivatives may include molecules formed by the deletion of one or more naturally-attached chemical groups. The polypeptide comprising the amino acid sequence of SEQ ID NO:2 or SEQ ID NO:4, or a Cloaked-2 polypeptide variant may be modified by the covalent attachment of one or more polymers. For example, the polymer selected is typically water soluble so that the protein to which it is attached does not precipitate in an aqueous environment, such as a physiological environment. Included within the scope of suitable polymers is a mixture of polymers. Preferably, for therapeutic use of the end-product preparation, the polymer will be pharmaceutically acceptable.

The polymers each may be of any molecular weight and may be branched or unbranched. The polymers each typically have an average molecular weight of between about 2kDa to about 10okDa (the term "about" indicating that in preparations of a water soluble polymer, some molecules will weigh more, some less, than the stated molecular weight). The average molecular weight of each polymer preferably is between about 5kDa and about 50kDa, more preferably between about 12kDa and about 40kDa and most preferably between about 20kDa and about 35kDa.

Suitable water soluble polymers or mixtures thereof include, but are not limited to, N-linked or O-linked carbohydrates, sugars, phosphates, polyethylene glycol (PEG) (including the forms of PEG that have been used to derivatize proteins, including mono-($C_1$-$C_{10}$) alkoxy- or aryloxy-polyethylene glycol), monomethoxypolyethylene glycol, dextran (such as low molecular weight dextran, of, for example about 6 kD), cellulose, or other carbohydrate based polymers, poly-(N-vinyl pyrrolidone) polyethylene glycol, propylene glycol homopolymers, a polypropylene oxide/ethylene oxide copolymer, polyoxyethylated polyols (*e.g.*, glycerol) and polyvinyl alcohol. Also encompassed by the present invention are bifunctional crosslinking molecules which may be used to prepare covalently attached multimers of the polypeptide comprising the amino acid sequence of SEQ ID NO:2 or SEQ ID NO:4 or a Cloaked-2 polypeptide variant.

In general, chemical derivatization may be performed under..any suitable condition used to react a protein with an activated polymer molecule. Methods for preparing chemical derivatives of polypeptides will generally comprise the steps of (a) reacting the polypeptide with the activated polymer molecule (such as a reactive ester or aldehyde derivative of the polymer molecule) under conditions whereby the polypeptide comprising the amino acid sequence of SEQ ID NO: 2 or SEQ ID NO:4, or a Cloaked-2 polypeptide variant becomes attached to one or more polymer molecules, and (b) obtaining the reaction product(s). The optimal reaction conditions will be determined based on known parameters and the desired result. For example, the larger the ratio of polymer molecules:protein, the greater the percentage of attached polymer molecule. In one embodiment, the Cloaked-2 polypeptide derivative may have a single polymer molecule moiety at the amino terminus. See, for example, U.S. Patent No. 5,234,784.

The pegylation of the polypeptide specifically may be carried out by any of the pegylation reactions known in the art, as described for example in the following references: Francis et al., Focus on Growth Factors, 3:4-10 (1992); EP 0154316; EP 0401384 and U.S. Patent No. 4,179,337. For example, pegylation may be carried out via an acylation reaction or an alkylation reaction with a reactive polyethylene glycol molecule (or an analogous reactive water-soluble polymer) as described herein. For the acylation reactions, the polymer(s) selected should have a single reactive ester group. For reductive alkylation, the polymer(s) selected should have a single reactive aldehyde group. A reactive aldehyde is, for example, polyethylene glycol propionaldehyde, which is water stable, or mono $C_1$-$C_{10}$ alkoxy or aryloxy derivatives thereof (see U.S. Patent No. 5,252,714).

In another embodiment, Cloaked-2 polypeptides may be chemically coupled to biotin, and the biotin/Cloaked-2 polypeptide molecules which are conjugated are then allowed to bind to avidin, resulting in tetravalent avidin/biotin/Cloaked-2 polypeptide molecules. Cloaked-2 polypeptides may also be covalently coupled to dinitrophenol (DNP) or trinitrophenol (TNP) and the resulting conjugates precipitated with anti-DNP or anti-TNP-IgM to form decameric conjugates with a valency of 10.

Generally, conditions which may be alleviated or modulated by the administration of the present Cloaked-2 polypeptide derivatives include those described herein for Cloaked-2 polypeptides. However, the Cloaked-2 polypeptide derivatives disclosed herein may have additional activities, enhanced or reduced biological activity, or other characteristics, such as increased or decreased half-life, as compared to the non-derivatized molecules.

Genetically Engineered Non-Human Animals

Additionally included within the scope of the present invention are non-human animals such as mice, rats, or other rodents, rabbits, goats, or sheep, or other farm animals, in which the gene (or genes) encoding the native Cloaked-2 polypeptide has (have) been disrupted ("knocked out") such that the level of expression of this gene or genes is (are) significantly decreased or completely abolished. Such animals may be prepared using techniques and methods such as those described in U.S. Patent No. 5,557,032.

The present invention further includes non-human animals such as mice, rats, or other rodents, rabbits, goats, sheep, or other farm animals, in which either the native form of the Cloaked-2 gene(s) for that animal or a heterologous Cloaked-2 gene(s) is (are) over-expressed by the animal, thereby creating a "transgenic" animal. Such transgenic animals may be prepared using well known methods such as those described in U.S. Patent No 5,489,743 and PCT application No. WO94/28122.

The present invention further includes non-human animals in which the promoter for one or more of the Cloaked-2 polypeptides of the present invention is either activated or inactivated (*e.g.*, by using homologous recombination methods) to alter the level of expression of one or more of the native Cloaked-2 polypeptides.

These non-human animals may be used for drug candidate screening. In such screening, the impact of a drug candidate on the animal may be measured. For example, drug candidates may decrease or increase the expression of the Cloaked-2 gene. In certain embodiments, the amount of Cloaked-2 polypeptide, that is produced may be measured after the exposure of the animal to the drug candidate. Additionally, in certain embodiments, one may detect the actual impact of the drug candidate on the animal. For example, the overexpression of a particular gene may result in, or be associated with, a disease or pathological condition. In such cases, one may test a drug candidate's ability to decrease expression of the gene or its ability to prevent or inhibit a pathological condition. In other examples, the production of a particular metabolic product such as a fragment of a polypeptide, may result in, or be associated with, a disease or pathological condition. In such cases, one may test a drug candidate's ability to decrease the production of such a metabolic product or its ability to prevent or inhibit a pathological condition.

Microarray

It will be appreciated that DNA microarray technology can be utilized in accordance with the present invention. DNA microarrays are miniature, high density arrays of nucleic acids positioned on a solid support, such as glass. Each cell or element within the array has numerous copies of a single species of DNA which acts as a target for hybridization for its cognate mRNA. In expression profiling using DNA microarray technology, mRNA is first extracted from a cell or tissue sample and then converted enzymatically to fluorescently labeled cDNA. This material is hybridized to the microarray and unbound cDNA is removed by washing. The expression of discrete genes represented on the array is then visualized by quantitating the amount of labeled cDNA which is specifically bound to each target DNA. In this way, the expression of thousands of genes can be quantitated in a high throughput, parallel manner from a single sample of biological material.

This high throughput expression profiling has a broad' range of applications with respect to the Cloaked-2 molecules of the invention, including, but not limited to: the identification and validation of Cloaked-2 disease-related genes as targets for therapeutics; molecular toxicology of Cloaked-2 molecules and inhibitors thereof; stratification of populations and generation of surrogate markers for clinical trials; and enhancing Cloaked-2-related small molecule drug discovery by aiding in the identification of selective compounds in high throughput screens (HTS).

Selective Binding Agents

As used herein, the term "selective binding agent" refers to a molecule which has specificity for one or more Cloaked-2 polypeptides. Suitable selective binding agents include, but are not limited to, antibodies and derivatives thereof, polypeptides, and small molecules. Suitable selective binding agents may be prepared using methods known in the art. An exemplary Cloaked-2 polypeptide selective binding agent of the present invention is capable of binding a certain portion of the Cloaked-2 polypeptide thereby inhibiting the binding of the polypeptide to the Cloaked-2 polypeptide receptor(s).

Selective binding agents such as antibodies and antibody fragments that bind Cloaked-2 polypeptides are within the scope of the present invention. The antibodies may be polyclonal including monospecific polyclonal, monoclonal (MAbs), recombinant, chimeric, humanized such as CDR-grafted, human, single chain, and/or bispecific, as well as fragments, variants or derivatives thereof. Antibody fragments include those portions of the antibody which bind to an epitope on the CLOAKED-2 polypeptide. Examples of such fragments include Fab and F(ab') fragments generated by enzymatic cleavage of full-length antibodies. Other binding fragments include those generated by recombinant DNA techniques, such as the expression of recombinant plasmids containing nucleic acid sequences encoding antibody variable regions.

Polyclonal antibodies directed toward a Cloaked-2 polypeptide generally are produced in animals (*e.g.,* rabbits or mice) by means of multiple subcutaneous or intraperitoneal injections of Cloaked-2 polypeptide and an adjuvant. It may be useful to conjugate a Cloaked-2 polypeptide to a carrier protein that is immunogenic in the species to be immunized, such as keyhole limpet heocyanin, serum, albumin, bovine thyroglobulin, or soybean trypsin inhibitor. Also, aggregating agents such as alum are used to enhance the immune response. After immunization, the animals are bled and the serum is assayed for anti-Cloaked-2 polypeptide antibody titer.

Monoclonal antibodies directed toward a Cloaked-2 polypeptide are produced using any method which provides for the production of antibody molecules by continuous cell lines in culture. Examples of suitable methods for preparing monoclonal antibodies include the hybridoma methods of Kohler et al., Nature, 256:495-497 (1975) and the human B-cell hybridoma method, Kozbor, J. Immunol., 133:3001 (1984); Brodeur et al., Monoclonal Antibody Production Techniques and Applications, pp. 51-63 (Marcel Dekker, Inc., New York, 1987). Also provided by the invention are hybridoma cell lines which produce monoclonal antibodies reactive with Cloaked-2 polypeptides.

Monoclonal antibodies of the invention may be modified for use as therapeutics. One embodiment is a "chimeric" antibody in which a portion of the heavy and/or light chain is identical with or homologous to a corresponding sequence in antibodies derived from a particular species or belonging to a particular antibody class or subclass, while the remainder of the chain(s) is identical with or homologous to a corresponding sequence in antibodies derived from another species or belonging to another antibody class or subclass. Also included are fragments of such antibodies, so long as they exhibit the desired biological activity. See, U.S. Patent No. 4,816,567; Morrison et al., Proc. Natl. Acad. Sci., 81:6851-6855 (1985).

In another embodiment, a monoclonal antibody of the invention is a "humanized" antibody. Methods for humanizing non-human antibodies are well known in the art. See U.S. Patent Nos. 5,585,089, and 5,693,762. Generally, a humanized antibody has one or more amino acid residues introduced into it from a source which is non-human. Humanization can be performed, for example, using methods described in the art (Jones et al., Nature 321:522-525 (1986); Riechmann et al., Nature, 332:323-327 (1988); Verhoeyen et al., Science 239:1534-1536 (1988)), by substituting at least a portion of a rodent complementarity-determining region (CDR) for the corresponding regions of a human antibody.

Also encompassed by the invention are human antibodies which bind Cloaked-2 polypeptides. Using transgenic animals (*e.g.*, mice) that are capable of producing a repertoire of human antibodies in the absence of endogenous immunoglobulin production such antibodies are produced by immunization with a Cloaked-2 antigen (*i.e.*, having at least 6 contiguous amino acids), optionally conjugated to a carrier. See, for example, Jakobovits et al., Proc. Natl. Acad. Sci., 90:2551-2555 (1993); Jakobovits et al., Nature 362:255-258 (1993); Bruggermann et al., Year in Immuno., 7:33 (1993). In one method, such transgenic animals are produced by incapacitating the endogenous loci encoding the heavy and light immunoglobulin chains therein, and inserting loci encoding human heavy and light chain proteins into the genome thereof. Partially modified animals, that' is those having less than the full complement of modifications, are then cross-bred to obtain an animal having all of the desired immune system modifications. When administered an immunogen, these transgenic animals produce antibodies with human (rather than *e.g.*, murine) amino acid sequences, including variable regions which are immunospecific for these antigens. See PCT application nos. PCT/US96/05928 and PCT/US93/06926. Additional methods are described in U.S. Patent No. 5,545,807, PCT application nos. PCT/US91/245, PCT/GB89/01207, and in EP 546073B1 and EP 546073A1. Human antibodies may also be produced by the expression of recombinant DNA in host cells or by expression in hybridoma cells as described herein.

In an alternative embodiment, human antibodies can be produced from phage-display libraries (Hoogenboom et al., J. Mol. Biol. 227:381 (1991); Marks et al., J. Mol. Biol. 222:581 (1991). These processes mimic immune selection through the display of antibody repertoires on the surface of filamentous bacteriophage, and subsequent selection of phage by their binding to an antigen of choice. One such technique is described in PCT Application no. PCT/US98/17364, which describes the isolation of high affinity and functional agonistic antibodies for MPL-and msk- receptors using such an approach.

Chimeric, CDR grafted, and humanized antibodies are typically produced by recombinant methods. Nucleic acids encoding the antibodies are introduced into host cells and expressed using materials and procedures described herein. In a preferred embodiment, the antibodies are produced in mammalian host cells, such as CHO cells. Monoclonal (*e.g.*, human) antibodies may be produced by the expression of recombinant DNA in host cells or by expression in hybridoma cells as described herein.

The anti-Cloaked-2 antibodies of the invention may be employed in any known assay method, such as competitive binding assays, direct and indirect sandwich assays, and immunoprecipitation assays (Sola, Monoclonal Antibodies: A Manual of Techniques, pp. 147-158 (CRC Press, Inc., 1987)) for the detection and quantitation of Cloaked-2 polypeptides. The antibodies will bind Cloaked-2 polypeptides with an affinity which is appropriate for the assay method being employed.

For diagnostic applications, in certain embodiments, anti-Cloaked-2 antibodies may be labeled with a detectable moiety. The detectable moiety can be any one which is capable of producing, either directly or indirectly, a detectable signal. For example, the detectable moiety may be a radioisotope, such as $^{3}H$, $^{14}C$, $^{32}P$, $^{35}S$, or $^{125}I$, a fluorescent or chemiluminescent compound, such as fluorescein isothiocyanate, rhodamine, or luciferin; or an enzyme, such as alkaline phosphatase, β-galactosidase, or horseradish peroxidase (Bayer et al., Meth. Enz., 184:138-163 (1990)).

Competitive binding assays rely on the ability of a labeled standard (*e.g.*, a Cloaked-2 polypeptide, or an immunologically reactive portion thereof) to compete with the test sample analyte (an Cloaked-2 polypeptide) for binding with a limited amount of anti Cloaked-2 antibody. The amount of a Cloaked-2 polypeptide in the test sample is inversely proportional to the amount of standard that becomes bound to the antibodies. To facilitate determining the amount of standard that becomes bound, the antibodies typically are insolubilized before or after the competition, so that the standard and analyte that are bound to the antibodies may conveniently be separated from the standard and analyte which remain unbound.

Sandwich assays typically involve the use of two antibodies, each capable of binding to a different immunogenic portion, or epitope, of the protein to be detected and/or quantitated. In a sandwich assay, the test sample analyte is typically bound by a first antibody which is immobilized on a solid support, and thereafter a second antibody binds to the analyte, thus forming an insoluble three part complex. See, *e.g.*, U.S. Patent No. 4,376,110. The second antibody may itself be labeled with a detectable moiety (direct sandwich assays) or may be measured using an anti-immunoglobulin antibody that is labeled with a detectable moiety (indirect sandwich assays). For example, one type of sandwich assay is an enzyme-linked immunosorbent assay (ELISA), in which case the detectable moiety is an enzyme.

The selective binding agents, including anti-Cloaked-2 antibodies, also are useful for *in vivo* imaging. An antibody labeled with a detectable moiety may be administered to an animal, preferably into the bloodstream, and the presence and location of the labeled antibody in the host is assayed. The antibody may be labeled with any moiety that is detectable in an animal, whether by nuclear magnetic resonance, radiology, or other detection means known in the art.

Selective binding agents of the invention, including antibodies, may be used as therapeutics. These therapeutic agents are generally agonists or antagonists, in that they either enhance or reduce, respectively, at least one of the biological activities of a Cloaked-2 polypeptide. In one embodiment, antagonist antibodies of the invention are antibodies or binding fragments thereof which are capable of specifically binding to a Cloaked-2 polypeptide and which are capable of inhibiting or eliminating the functional activity of a Cloaked-2 polypeptide *in vivo* or *in vitro*. In preferred embodiments, the selective binding agent, *e.g.*, an antagonist antibody, will inhibit the functional activity of a Cloaked-2 polypeptide by at least about 50%, and preferably by at least about 80%. In another embodiment, the selective binding agent may be an antibody that is capable of interacting with a Cloaked-2 binding partner (a ligand or receptor) thereby inhibiting or eliminating Cloaked-2 activity *in vitro* or *in vivo.* Selective binding agents, including agonist and antagonist anti-Cloaked-2 antibodies, are identified by screening assays which are well known in the art.

The invention also relates to a kit comprising Cloaked-2 selective binding agents (such as antibodies) and other reagents useful for detecting Cloaked-2 polypeptide levels in biological samples. Such reagents may include, a detectable label, blocking serum, positive and negative control samples, and detection reagents.

The Cloaked-2 polypeptides of the present invention can be used to clone Cloaked-2 receptors, using an expression cloning strategy. Radiolabeled (125-Iodine) Cloaked-2 polypeptide or affinity/activity-tagged Cloaked-2 polypeptide (such as an Fc fusion or an alkaline phosphatase fusion) can be used in binding assays to identify a cell type or cell line or tissue that expresses Cloaked-2 receptor(s). RNA isolated from such cells or tissues can be converted to cDNA, cloned into a mammalian expression vector, and transfected into mammalian cells (such as COS or 293 cells) to create an expression library. A radiolabeled or tagged Cloaked-2 polypeptide can then be used as an affinity ligand to identify and isolate from this library the subset of cells which express the Cloaked-2 receptor(s) on their surface. DNA can then be isolated from these cells and transfected into mammalian cells to create a secondary expression library in which the fraction of cells expressing Cloaked-2 receptor(s) is many-fold higher than in the original library. This enrichment process can be repeated iteratively until a single recombinant clone containing a Cloaked-2 receptor is isolated. Isolation of the Cloaked-2 receptor(s) is useful for identifying or developing novel agonists and antagonists of the Cloaked-2 polypeptide signaling pathway. Such agonists and antagonists include soluble Cloaked-2 receptor(s), anti-Cloaked-2 receptor antibodies, small molecules, or antisense oligonucleotides, and they may be used for treating, preventing, or diagnosing one or more disease or disorder, including those described herein.

Assaying for Other Modulators of Cloaked-2 Polypeptide Activity

In some situations, it may be desirable to identify molecules that are modulators, *i.e.*, agonists or antagonists, of the activity of Cloaked-2 polypeptide. Natural or synthetic molecules that modulate Cloaked-2 polypeptide may be identified using one or more screening assays, such as those described herein. Such molecules may be administered either in an *ex vivo* manner, or in an *in vivo* manner by injection, or by oral delivery, implantation device, or the like.

"Test molecule (s)" refers to the molecule (s) that is/are under evaluation for the ability to modulate (*i.e.,* increase or decrease) the activity of a Cloaked-2 polypeptide. Most commonly, a test molecule will interact directly with a Cloaked-2 polypeptide. However, it is also contemplated that a test molecule may also modulate Cloaked-2 polypeptide activity indirectly, such as by affecting Cloaked-2 gene expression, or by binding to a Cloaked-2 binding partner (*e.g.*, receptor or ligand). In one embodiment, a test molecule will bind to a Cloaked-2 polypeptide with an affinity constant of at least about $10^{-6}$ M, preferably about $10^{-8}$ M, more preferably about $10^{-9}$ M, and even more preferably about $10^{-10}$ M.

Methods for identifying compounds which interact with Cloaked-2 polypeptides are encompassed by the present invention. In certain embodiments, a Cloaked-2 polypeptide is incubated with a test molecule under conditions which permit the interaction of the test molecule with a Cloaked-2 polypeptide, and the extent of the interaction can be measured. The test molecule(s) can be screened in a substantially purified form or in a crude mixture.

In certain embodiments, a Cloaked-2 polypeptide agonist or antagonist may be a protein, peptide, carbohydrate, lipid, or small molecular weight molecule which interacts with Cloaked-2 polypeptide to regulate its activity. Molecules which regulate Cloaked-2 polypeptide expression include nucleic acids which are complementary to nucleic acids encoding a Cloaked-2 polypeptide, or are complementary to nucleic acids sequences which direct or control the expression of Cloaked-2 polypeptide, and which act as anti-sense regulators of expression.

Once a set of test molecules has been identified as interacting with a Cloaked-2 polypeptide, the molecules may be further evaluated for their ability to increase or decrease Cloaked-2 polypeptide activity. The measurement of the interaction of test molecules with Cloaked-2 polypeptides may be carried out in several formats, including cell-based binding assays, membrane binding assays, solution-phase assays and immunoassays. In general, test molecules are incubated with a Cloaked-2 polypeptide for a specified period of time, and Cloaked-2 polypeptide activity is determined by one or more assays for measuring biological activity.

The interaction of test molecules with Cloaked-2 polypeptides may also be assayed directly using polyclonal or monoclonal antibodies in an immunoassay. Alternatively, modified forms of Cloaked-2 polypeptides containing epitope tags as described herein may be used in immunoassays.

In the event that Cloaked-2 polypeptides display biological activity through an interaction with a binding partner (*e.g.*, a receptor or a ligand), a variety of *in vitro* assays may be used to measure the binding of a Cloaked-2 polypeptide to the corresponding binding partner (such as a selective binding agent, receptor, or ligand). These assays may be used to screen test molecules for their ability to increase or decrease the rate and/or the extent of binding of a Cloaked-2 polypeptide to its binding partner. In one assay, a Cloaked-2 polypeptide is immobilized in the wells of a microtiter plate. Radiolabeled Cloaked-2 binding partner (for example, iodinated Cloaked-2 binding partner) and the test molecule(s) can then be added either one at a time (in either order) or simultaneously to the wells. After incubation, the wells can be washed and counted, using a scintillation counter, for radioactivity to determine the extent to which the binding partner bound to Cloaked-2 polypeptide. Typically, the molecules will be tested over a range of concentrations, and a series of control wells lacking one or more elements of the test assays can be used for accuracy in the evaluation of the results. An alternative to this method involves reversing the "positions" of the proteins, *i.e.*, immobilizing Cloaked-2 binding partner to the microtiter plate wells, incubating with the test molecule and radiolabeled Cloaked-2 polypeptide, and determining the extent of Cloaked-2 polypeptide binding. See, for example, chapter 18, Current Protocols in Molecular Biology, Ausubel et al., eds., John Wiley & Sons, New York, NY (1995).

As an alternative to radiolabelling, a Cloaked-2 polypeptide or its binding partner may be conjugated to biotin and the presence of biotinylated protein can then be detected using streptavidin linked to an enzyme, such as horseradish peroxidase (HRP) or alkaline phosphatase (AP), that can be detected colorometrically, or by fluorescent tagging of streptavidin. An antibody directed to a Cloaked-2 polypeptide or to a Cloaked-2 binding partner and conjugated to biotin may also be used and can be detected after incubation with enzyme-linked streptavidin linked to AP or HRP.

An Cloaked-2 polypeptide or a Cloaked-2 binding partner can also be immobilized by attachment to agarose beads, acrylic beads or other types of such inert solid phase substrates. The substrate-protein complex can be placed in a solution containing the complementary protein and the test compound. After incubation, the beads can be precipitated by centrifugation, and the amount of binding between a Cloaked-2 polypeptide and its binding partner can be assessed using the methods described herein. Alternatively, the substrate-protein complex can be immobilized in a column, and the test molecule and complementary protein are passed through the column. The formation of a complex between a Cloaked-2 polypeptide and its binding partner can then be assessed using any of the techniques set forth herein, *i.e.*, radiolabelling, antibody binding, or the like.

Another *in vitro* assay that is useful for identifying a test molecule which increases or decreases the formation of a complex between a Cloaked-2 polypeptide and a Cloaked-2 binding partner is a surface plasmon resonance detector system such as the BIAcore assay system (Pharmacia, Piscataway, NJ). The BIAcore system may be carried out using the manufacturer's protocol. This assay essentially involves the covalent binding of either Cloaked-2 polypeptide or a Cloaked-2 binding partner to a dextran-coated sensor chip which is located in a detector. The test compound and the other complementary protein can then be injected, either simultaneously or sequentially, into the chamber containing the sensor chip. The amount of complementary protein that binds can be assessed based on the change in molecular mass which is physically associated with the dextran-coated side of the sensor chip; the change in molecular mass can be measured by the detector system.

In some cases, it may be desirable to evaluate two or more test compounds together for their ability to increase or decrease the formation of a complex between a Cloaked-2 polypeptide and a Cloaked-2 binding partner. In these cases, the assays set forth herein can be readily modified by adding such additional test compound(s) either simultaneous with, or subsequent to, the first test compound. The remainder of the steps in the assay are as set forth herein.

*In vitro* assays such as those described herein may be used advantageously to screen large numbers of compounds for effects on complex formation by Cloaked-2 polypeptide and Cloaked-2 binding partner. The assays may be automated to screen compounds generated in phage display, synthetic peptide, and chemical synthesis libraries.

Compounds which increase or decrease the formation of a complex between a Cloaked-2 polypeptide and a Cloaked-2 binding partner may also be screened in cell culture using cells and cell lines expressing either Cloaked-2 polypeptide or Cloaked-2 binding partner. Cells and cell lines may be obtained from any mammal, but preferably will be from human or other primate, canine, or rodent sources. The binding of a Cloaked-2 polypeptide to cells expressing Cloaked-2 binding partner at the surface is evaluated in the presence or absence of test molecules, and the extent of binding may be determined by, for example, flow cytometry using a biotinylated antibody to a Cloaked-2 binding partner. Cell culture assays can be used advantageously to further evaluate compounds that score positive in protein binding assays described herein.

Cell cultures can also be used to screen the impact of a drug candidate. For example, drug candidates may decrease or increase the expression of the Cloaked-2 gene. In certain embodiments, the amount of Cloaked-2 polypeptide that is produced may be measured after exposure of the cell culture to the drug candidate. In certain embodiments, one may detect the actual impact of the drug candidate on the cell culture. For example, the overexpression of a particular gene may have a particular impact on the cell culture. In such cases, one may test a drug candidate's ability to increase or decrease the expression of the gene or its ability to prevent or inhibit a particular impact on the cell culture. In other examples, the production of a particular metabolic product such as a fragment of a polypeptide, may result in, or be associated with, a disease or pathological condition. In such cases, one may test a drug candidate's ability to decrease the production of such a metabolic product in a cell culture.

Internalizing Proteins

The tat protein sequence (from HIV) can be used to internalize proteins into a cell. See *e.g.*, Falwell et al., Proc. Natl. Acad. Sci., 91:664-668 (1994). For example, an 11 amino acid sequence (YGRKKRRQRRR; SEQ ID NO: 23) of the HIV tat protein (termed the "protein transduction domain", or TAT PDT) has been described as mediating delivery across the cytoplasmic membrane and the nuclear membrane of a cell. See Schwarze et al., Science, 285:1569-1572 (1999); and Nagahara et al., Nature Medicine, 4:1449-1452 (1998). In these procedures, FITC-constructs (FITC-GGGGYGRKKRRQRRR; SEQ IS NO: 24) are prepared which bind to cells as observed by fluorescence-activated cell sorting (FACS) analysis, and these constructs penetrate tissues after i.p. administration. Next, tat-βgal fusion proteins are constructed. Cells treated with this construct demonstrated b-gal activity. Following injection, a number of tissues, including liver, kidney, lung, heart, and brain tissue have been found to demonstrate expression using these procedures. It is believed that these constructions underwent some degree of unfolding in order to enter the cell; as such, refolding may be required after entering the cell.

It will thus be appreciated that the tat protein sequence may be used to internalize a desired protein or polypeptide into a cell. For example, using the tat protein sequence, a Cloaked-2 antagonist (such as an anti-Cloaked-2 selective binding agent, small molecule, soluble receptor, or antisense oligonucleotide) can be administered intracellularly to inhibit the activity of a Cloaked-2 molecule. As used herein, the term "Cloaked-2 molecule" refers to both Cloaked-2 nucleic acid molecules and Cloaked-2 polypeptides as defined herein. Where desired, the Cloaked-2 protein itself may also be internally administered to a cell using these procedures. *See also,* Strauss, E., "Introducing Proteins Into the Body's Cells", Science, 285:1466-1467 (1999).

Therapeutic Uses

Members of the cystine-knot growth factor structural superfamily are important regulators of one or more of the following biological processes: cell proliferation, cell survival, cell differentiation, cell-cell communication and cellular function. See *e.g.,* Sun and Davies, Annual Review of Biophysics and Biomolecular Structure, vol. 24, pp. 269-291 (1995).
A non-exclusive list of acute and chronic diseases which can be treated, diagnosed, ameliorated, or prevented with the polypeptides and nucleic acids of the invention include:

- Diseases or disorders involving the kidney. Examples of such diseases or disorders include, but are not limited to, anemia, hypertension and low blood pressure. Other kidney associated diseases or disorders are encompassed within the scope of the invention.

- Diseases or disorders involving the heart. Examples of such diseases or disorders include, but are not limited to, cardiac hypertrophy, congestive heart failure, myocardial infarction, arrhythmias, atherosclerosis, hypertension and low blood pressure. Other heart associated diseases or disorders are encompassed within the scope of the invention.

- Diseases or disorders involving skeletal muscle. Examples of such diseases or disorders include, but are not limited to, muscular dystrophy and cachexia. Other skeletal muscle associated diseases or disorders are encompassed within the scope of the invention.

- Diseases or disorders involving the placenta. Examples of such diseases or disorders include, but are not limited to, miscarriage and congenital abnormalities. Other placenta associated diseases or disorders are encompassed within the scope of the invention.

- Diseases or disorders involving the liver. Examples of such diseases or disorders include, but are not limited to, hepatitis and cirrhosis. Other liver associated diseases or disorders are encompassed within the scope of the invention.

- Diseases or disorders involving the pancreas. Examples of such diseases or disorders include, but are not limited to, diabetes and pancreatitis. Other pancreas associated diseases or disorders are encompassed within the scope of the invention.

- Diseases or disorders involving the thyroid. Examples of such diseases or disorders include, but are not limited to, Graves' disease and myxedema. Other thyroid associated diseases or disorders are encompassed within the scope of the invention.

- Diseases or disorders involving the adrenal cortex. Examples of such diseases or disorders include, but are not limited to, Cushing's disease and Addison's disease. Other adrenal cortex associated diseases or disorders are encompassed within the scope of the invention.

Additionally, the expression of Cloaked-2 in kidney, heart, placenta, skeletal muscle, liver, pancreas, thyroid, and adrenal cortex indicates a possible role for Cloaked-2 in the common function of these organs or tissues, namely, energy utilization/homeostasis and metabolism. As such, Cloaked-2 polypeptide and/or Cloaked-2 polypeptide agonists or antagonists (such as selective binding agents) may be useful for the treatment and/or diagnosis of energy utilization/homeostasis diseases or disorders and metabolic diseases or disorders. Examples of such diseases or disorders include, but are not limited to, obesity, wasting syndromes (for example, cancer associated cachexia), myopathies, gastrointestinal diseases or disorders, diabetes, growth failure, hypercholesterolemia, atherosclerosis and aging. Other energy utilization/homeostasis and metabolism associated diseases or disorders are encompassed within the scope of the invention.
Expression of Cloaked-2 in the brain has been detected, specifically in the amygdala and thalamus. These regions of the brain are both components of the limbic system which is involved in processing and relaying information from the conscious senses. Lesions in the amygdala can lead to docile behavior, emotional instability, increased fighting, and increased eating. As such, Cloaked-2 polypeptide and/or Cloaked-2 polypeptide agonists or antagonists may be useful for the treatment and/or diagnosis of emotion-related diseases or disorders. Examples of such diseases or disorders include, but are not limited to, depression, obesity, obsessive-compulsive disorder, psychosis, anxiety, schizophrenia, bipolar disorder and stress related diseases or disorders. Other emotion related diseases or disorders are encompassed within the scope of the invention.
Cloaked-2 polypeptide may also act as a growth factor involved in the regeneration (proliferation and differentiation) of tissues or specialized cell types present in kidney, heart, placenta, skeletal muscle, liver, pancreas, thyroid, adrenal cortex, amygdala and thalamus.

Because Cloaked-2 polypeptide is likely to have hormonal activities or growth-factor activities, Cloaked-2 polypeptide and/or Cloaked-2 polypeptide agonists or antagonists may be useful for the treatment and/or diagnosis of diseases or disorders that could be treated by increasing cell proliferation and/or differentiation. Examples of such diseases or disorders include, but are not limited to, tissue damage/degeneration (such as caused by cancer treatments, infections, autoimmune diseases or disorders), aging and wound healing. Other diseases or disorders that can be treated by increasing cell proliferation and/or differentiation are also encompassed within the therapeutic and diagnostic utilities that are part of the invention.

Because the Cloaked-2 polypeptide is likely to have hormonal activities or growth-factor activities, Cloaked-2 polypeptide and/or Cloaked-2 polypeptide agonists or antagonists may be useful for the treatment and/or diagnosis of diseases or disorders that can be treated by decreasing cell proliferation and/or differentiation. Examples of such diseases or disorders include, but are not limited to, cancers, hyperplasias and hypertrophies. Other diseases or disorders that could be treated by decreasing cell proliferation and/or differentiation are also encompassed within the therapeutic and diagnostic utilities that are part of the invention.

Other diseases or disorders caused or mediated by undesirable levels of Cloaked-2 polypeptide are encompassed within the therapeutic and diagnostic utilities that are part of the invention. By way of illustration, such undesirable levels include excessively elevated levels and sub-normal levels.

Cloaked-2 Compositions and Administration

Therapeutic compositions are within the scope of the present invention. Such Cloaked-2 pharmaceutical compositions may comprise a therapeutically effective amount of a Cloaked-2 polypeptide or a Cloaked-2 nucleic acid molecule in admixture with a pharmaceutically or physiologically acceptable formulation agent selected for suitability with the mode of administration. Pharmaceutical compositions may comprise a therapeutically effective amount of one or more Cloaked-2 selective binding agents in admixture with a pharmaceutically or physiologically acceptable formulation agent selected for suitability with the mode of administration.

Acceptable formulation materials preferably are nontoxic to recipients at the dosages and concentrations employed.

The pharmaceutical composition may contain formulation materials for modifying, maintaining or preserving, for example, the pH, osmolarity, viscosity, clarity, color, isotonicity, odor, sterility, stability, rate of dissolution or release, adsorption or penetration of the composition. Suitable formulation materials include, but are not limited to, amino acids (such as glycine, glutamine, asparagine, arginine or lysine), antimicrobials, antioxidants (such as ascorbic acid, sodium sulfite or sodium hydrogensulfite), buffers (such as borate, bicarbonate, Tris-HCl, citrates, phosphates, other organic acids), bulking agents (such as mannitol or glycine), chelating agents (such as ethylenediamine tetraacetic acid (EDTA)), complexing agents (such as caffeine, polyvinylpyrrolidone, beta-cyclodextrin or hydroxypropyl-beta-cyclodextrin), fillers, monosaccharides, disaccharides, and other carbohydrates (such as glucose, mannose, or dextrins), proteins (such as serum albumin, gelatin or immunoglobulins), coloring, flavoring and diluting agents, emulsifying agents, hydrophilic polymers (such as polyvinylpyrrolidone), low molecular weight polypeptides, salt-forming counterions (such as sodium), preservatives (such as benzalkonium chloride, benzoic acid, salicylic acid, thimerosal, phenethyl alcohol, methylparaben, propylparaben, chlorhexidine, sorbic acid or hydrogen peroxide), solvents (such as glycerin, propylene glycol or polyethylene glycol), sugar alcohols (such as mannitol or sorbitol), suspending agents, surfactants or wetting agents (such as pluronics, PEG, sorbitan esters, polysorbates such as polysorbate 20, polysorbate 80, triton, tromethamine, lecithin, cholesterol, tyloxapal), stability enhancing agents (sucrose or sorbitol), tonicity enhancing agents (such as alkali metal halides (preferably sodium or potassium chloride), mannitol sorbitol), delivery vehicles, diluents, excipients and/or pharmaceutical adjuvants. *(*Remington's Pharmaceutical Sciences, 18th Edition, A.R. Gennaro, ed., Mack Publishing Company [1990]).

The optimal pharmaceutical composition will be determined by one skilled in the art depending upon, for example, the intended route of administration, delivery format, and desired dosage. See for example, *Remington's Pharmaceutical Sciences, supra.* Such compositions may influence the physical state, stability, rate of *in vivo* release, and rate of *in vivo* clearance of the Cloaked-2 molecule.

The primary vehicle or carrier in a pharmaceutical composition may be either aqueous or non-aqueous in nature. For example, a suitable vehicle or carrier may be water for injection, physiological saline solution, or artificial cerebrospinal fluid, possibly supplemented with other materials common in compositions for parenteral administration. Neutral buffered saline or saline mixed with serum albumin are further exemplary vehicles. Other exemplary pharmaceutical compositions comprise Tris buffer of about pH 7.0-8.5, or acetate buffer of about pH 4.0-5.5, which may further include sorbitol or a suitable substitute therefor. In one embodiment of the present invention, Cloaked-2 polypeptide compositions may be prepared for storage by mixing the selected composition having the desired degree of purity with optional formulation agents (*Remington's Pharmaceutical Sciences, supra)* in the form of a lyophilized cake or an aqueous solution. Further, the Cloaked-2 polypeptide product may be formulated as a lyophilizate using appropriate excipients such as sucrose.

The Cloaked-2 pharmaceutical compositions can be selected for parenteral delivery. Alternatively, the compositions may be selected for inhalation or for delivery through the digestive tract, such as orally. The preparation of such pharmaceutically acceptable compositions is within the skill of the art.

The formulation components are present in concentrations that are acceptable to the site of administration. For example, buffers are used to maintain the composition at physiological pH or at slightly lower pH, typically within a pH range of from about 5 to about 8.

When parenteral administration is contemplated, the therapeutic compositions for use in this invention may be in the form of a pyrogen-free, parenterally acceptable aqueous solution comprising the desired Cloaked-2 molecule in a pharmaceutically acceptable vehicle. A particularly suitable vehicle for parenteral injection is sterile distilled water in which a Cloaked-2 molecule is formulated as a sterile, isotonic solution, properly preserved. Yet another preparation can involve the formulation of the desired molecule with an agent, such as injectable microspheres, bio-erodible particles, polymeric compounds (polylactic acid, polyglycolic acid), or beads, or liposomes, that provides for the controlled or sustained release of the product which may then be delivered as a depot injection. Hyaluronic acid may also be used, and this may have the effect of promoting sustained duration in the circulation. Other suitable means for the introduction of the desired molecule include implantable drug delivery devices.

In one embodiment, a pharmaceutical composition may be formulated for inhalation. For example, a Cloaked-2 molecule may be formulated as a dry powder for inhalation. Cloaked-2 polypeptide or Cloaked-2 nucleic acid molecule inhalation solutions may also be formulated with a propellant for aerosol delivery. In yet another embodiment, solutions may be nebulized. Pulmonary administration is further described in PCT application no. PCT/US94/001875, which describes pulmonary delivery of chemically modified proteins.

It is also contemplated that certain formulations may be administered orally. In one embodiment of the present invention, Cloaked-2 molecules which are administered in this fashion can be formulated with or without those carriers customarily used in the compounding of solid dosage forms such as tablets and capsules. For example, a capsule may be designed to release the active portion of the formulation at the point in the gastrointestinal tract when bioavailability is maximized and pre-systemic degradation is minimized. Additional agents can be included to facilitate absorption of the Cloaked-2 molecule. Diluents, flavorings, low melting point waxes, vegetable oils, lubricants, suspending agents, tablet disintegrating agents, and binders may also be employed.

Another pharmaceutical composition may involve an effective quantity of Cloaked-2 molecules in a mixture with non-toxic excipients which are suitable for the manufacture of tablets. By dissolving the tablets in sterile water, or other appropriate vehicle, solutions can be prepared in unit dose form. Suitable excipients include, but are not limited to, inert diluents, such as calcium carbonate, sodium carbonate or bicarbonate, lactose, or calcium phosphate; or binding agents, such as starch, gelatin, or acacia; or lubricating agents such as magnesium stearate, stearic acid, or talc.

Additional Cloaked-2 pharmaceutical compositions will be evident to those skilled in the art, including formulations involving Cloaked-2 polypeptides in sustained- or controlled-delivery formulations. Techniques for formulating a variety of other sustained- or controlled-delivery means, such as liposome carriers, bio-erodible microparticles or porous beads and depot injections, are also known to those skilled in the art. See for example, PCT/US93/00829 which describes controlled release of porous polymeric microparticles for the delivery of pharmaceutical compositions. Additional examples of sustained-release preparations include semipermeable polymer matrices in the form of shaped articles, *e.g.* films, or microcapsules. Sustained release matrices may include polyesters, hydrogels, polylactides (U.S. 3,773,919, EP 58,481), copolymers of L-glutamic acid and gamma ethyl-L-glutamate (Sidman et al., Biopolymers, 22:547-556 (1983)), poly (2-hydroxyethylmethacrylate) (Langer et al., J. Biomed. Mater. Res., 15:167-277 (1981) and Langer, Chem. Tech., 12:98-105 (1982)), ethylene vinyl acetate (Langer *et al., supra*) or poly-D(-)-3-hydroxybutyric acid (EP 133,988). Sustained-release compositions also may include liposomes, which can be prepared by any of several methods known in the art. See *e.g.*, Eppstein et al., Proc. Natl. Acad. Sci. USA, 82:3688-3692 (1985); EP 36,676; EP 88,046; EP 143,949.

The Cloaked-2 pharmaceutical composition to be used for *in vivo* administration typically must be sterile. This may be accomplished by filtration through sterile filtration membranes. Where the composition is lyophilized, sterilization using these methods may be conducted either prior to, or following, lyophilization and reconstitution. The composition for parenteral administration may be stored in lyophilized form or in solution. In addition, parenteral compositions generally are placed into a container having a sterile access port, for example, an intravenous solution bag or vial having a stopper pierceable by a hypodermic injection needle.

Once the pharmaceutical composition has been formulated, it may be stored in sterile vials as a solution, suspension, gel, emulsion, solid, or a dehydrated or lyophilized powder. Such formulations may be stored either in a ready-to-use form or in a form *(e.g.,* lyophilized) requiring reconstitution prior to administration.

In a specific embodiment, the present invention is directed to kits for producing a single-dose administration unit. The kits may each contain both a first container having a dried protein and a second container having an aqueous formulation. Also included within the scope of this invention are kits containing single and multi-chambered pre-filled syringes (*e.g.*, liquid syringes and lyosyringes).

An effective amount of a Cloaked-2 pharmaceutical composition to be employed therapeutically will depend, for example, upon the therapeutic context and objectives. One skilled in the art will appreciate that the appropriate dosage levels for treatment will thus vary depending, in part, upon the molecule delivered, the indication for which the Cloaked-2 molecule is being used, the route of administration, and the size (body weight, body surface or organ size) and condition (the age and general health) of the patient. Accordingly, the clinician may titer the dosage and modify the route of administration to obtain the optimal therapeutic effect. A typical dosage may range from about 0.1 μg/kg to up to about 100 mg/kg or more, depending on the factors mentioned above. In other embodiments, the dosage may range from 0.1 μg/kg up to about 100 mg/kg; or 1 μg/kg up to about 100 mg/kg; or 5 μg/kg up to about 100 mg/kg.

The frequency of dosing will depend upon the pharmacokinetic parameters of the Cloaked-2 molecule in the formulation used. Typically, a clinician will administer the composition until a dosage is reached that achieves the desired effect. The composition may therefore be administered as a single dose, or as two or more doses (which may or may not contain the same amount of the desired molecule) over time, or as a continuous infusion via implantation device or catheter. Further refinement of the appropriate dosage is routinely made by those of ordinary skill in the art and is within the ambit of tasks routinely performed by them. Appropriate dosages may be ascertained through use of appropriate dose-response data.

The route of administration of the pharmaceutical composition is in accord with known methods, *e.g.* oral, injection by intravenous, intraperitoneal, intracerebral (intra-parenchymal), intracerebroventricular, intramuscular, intraocular, intraarterial, intraportal, or intralesional routes, or by sustained release systems or implantation device. Where desired, the compositions may be administered by bolus injection or continuously by infusion, or by implantation device.

Alternatively or additionally, the composition may be administered locally via implantation of a membrane, sponge, or other appropriate material on to which the desired molecule has been absorbed or encapsulated. Where an implantation device is used, the device may be implanted into any suitable tissue or organ, and delivery of the desired molecule may be via diffusion, timed release bolus, or continuous administration.

In some cases, it may be desirable to use Cloaked-2 pharmaceutical compositions in an ex *vivo* manner. In such instances, cells, tissues, or organs that have been removed from the patient are exposed to Cloaked-2 pharmaceutical compositions after which the cells, tissues and/or organs are subsequently implanted back into the patient.

In other cases, a Cloaked-2 polypeptide can be delivered by implanting certain cells that have been genetically engineered, using methods such as those described herein, to express and secrete the polypeptide. Such cells may be animal or human cells, and may be autologous, heterologous, or xenogeneic. Optionally, the cells may be immortalized. In order to decrease the chance of an immunological response, the cells may be encapsulated to avoid infiltration of surrounding tissues. The encapsulation materials are typically biocompatible, semi-permeable polymeric enclosures or membranes that allow the release of the protein product(s) but prevent the destruction of the cells by the patient's immune system or by other detrimental factors from the surrounding tissues.

Additional embodiments of the present invention relate to cells and methods (*e.g.*, homologous recombination and/or other recombinant production methods) for both the *in vitro* production of therapeutic polypeptides and for the production and delivery of therapeutic polypeptides by gene therapy or cell therapy. Homologous and other recombination methods may be used to modify a cell that contains a normally transcriptionally silent Cloaked-2 gene, or an under expressed gene, and thereby produce a cell which expresses therapeutically efficacious amounts of Cloaked-2 polypeptides.

Homologous recombination is a technique originally developed for targeting genes to induce or correct mutations in transcriptionally active genes (Kucherlapati, Prog. in Nucl. Acid Res. & Mol. Biol., 36:301, 1989). The basic technique was developed as a method for introducing specific mutations into specific regions of the mammalian genome (Thomas et al., Cell, 44:419-428, 1986; Thomas and Capecchi, Cell, 51:503-512, 1987; Doetschman et al., Proc. Natl. Acad. Sci., 85:8583-8587, 1988) or to correct specific mutations within defective genes (Doetschman et al., Nature, 330:576-578, 1987). Exemplary homologous recombination techniques are described in U.S. Patent No. 5,272,071 (EP 9193051, EP Publication No. 505500; PCT/US90/07642, International Publication No. WO 91/09955).

Through homologous recombination, the DNA sequence to be inserted into the genome can be directed to a specific region of the gene of interest by attaching it to targeting DNA. The targeting DNA is a nucleotide sequence that is complementary (homologous) to a region of the genomic DNA. Small pieces of targeting DNA that are complementary to a specific region of the genome are put in contact with the parental strand during the DNA replication process. It is a general property of DNA that has been inserted into a cell to hybridize, and therefore, recombine with other pieces of endogenous DNA through shared homologous regions. If this complementary strand is attached to an oligonucleotide that contains a mutation or a different sequence or an additional nucleotide, it too is incorporated into the newly synthesized strand as a result of the recombination. As a result of the proofreading function, it is possible for the new sequence of DNA to serve as the template. Thus, the transferred DNA is incorporated into the genome.

Attached to these pieces of targeting DNA are regions of DNA which may interact with or control the expression of a Cloaked-2 polypeptide, *e.g.*, flanking sequences. For example, a promoter/enhancer element, a suppresser, or an exogenous transcription modulatory element is inserted in the genome of the intended host cell in proximity and orientation sufficient to influence the transcription of DNA encoding the desired Cloaked-2 polypeptide. The control element controls a portion of the DNA present in the host cell genome. Thus, the expression of the desired Cloaked-2 polypeptide may be achieved not by transfection of DNA that encodes the Cloaked-2 gene itself, but rather by the use of targeting DNA (containing regions of homology with the endogenous gene of interest) coupled with DNA regulatory segments that provide the endogenous gene sequence with recognizable signals for transcription of a Cloaked-2 polypeptide.

In an exemplary method, the expression of a desired targeted gene in a cell (*i.e.*, a desired endogenous cellular gene) is altered via homologous recombination into the cellular genome at a preselected site, by the introduction of DNA which includes at least a regulatory sequence, an exon and a splice donor site. These components are introduced into the chromosomal (genomic) DNA in such a manner that this, in effect, results in the production of a new transcription unit (in which the regulatory sequence, the exon and the splice donor site present in the DNA construct are operatively linked to the endogenous gene). As a result of the introduction of these components into the chromosomal DNA, the expression of the desired endogenous gene is altered.

Altered gene expression, as described herein, encompasses activating (or causing to be expressed) a gene which is normally silent (unexpressed) in the cell as obtained, as well as increasing the expression of a gene which is not expressed at physiologically significant levels in the cell as obtained. The embodiments further encompass changing the pattern of regulation or induction such that it is different from the pattern of regulation or induction that occurs in the cell as obtained, and reducing (including eliminating) the expression of a gene which is expressed in the cell as obtained.

One method by which homologous recombination can be used to increase, or cause, Cloaked-2 polypeptide production from a cell's endogenous Cloaked-2 gene involves first using homologous recombination to place a recombination sequence from a site-specific recombination system (*e.g.*, Cre/loxP, FLP/FRT) (Sauer, Current Opinion In Biotechnology, 5:521-527, 1994; Sauer, Methods In Enzymology, 225:890-900, 1993) upstream (that is, 5' to) of the cell's endogenous genomic Cloaked-2 polypeptide coding region. A plasmid containing a recombination site homologous to the site that was placed just upstream of the genomic Cloaked-2 polypeptide coding region is introduced into the modified cell line along with the appropriate recombinase enzyme. This recombinase causes the plasmid to integrate, via the plasmid's recombination site, into the recombination site located just upstream of the genomic Cloaked-2 polypeptide coding region in the cell line (Baubonis and Sauer, Nucleic Acids Res., 21:2025-2029, 1993; O'Gorman et al., Science, 251:1351-1355, 1991). Any flanking sequences known to increase transcription (*e.g.*, enhancer/promoter, intron, translational enhancer), if properly positioned in this plasmid, would integrate in such a manner as to create a new or modified transcriptional unit resulting in de novo or increased Cloaked-2 polypeptide production from the cell's endogenous Cloaked-2 gene.

A further method to use the cell line in which the site specific recombination sequence had been placed just upstream of the cell's endogenous genomic Cloaked-2 polypeptide coding region is to use homologous recombination to introduce a second recombination site elsewhere in the cell line's genome. The appropriate recombinase enzyme is then introduced into the two-recombination-site cell line, causing a recombination event (deletion, inversion, translocation) (Sauer, *Current Opinion In Biotechnology, supra,* 1994; Sauer, *Methods In Enzymology, supra,* 1993) that would create a new or modified transcriptional unit resulting in de novo or increased Cloaked-2 polypeptide production from the cell's endogenous Cloaked-2 gene.

An additional approach for increasing, or causing, the expression of Cloaked-2 polypeptide from a cell's endogenous Cloaked-2 gene involves increasing, or causing, the expression of a gene or genes (*e.g.*, transcription factors) and/or decreasing the expression of a gene or genes (*e.g.*, transcriptional repressors) in a manner which results in de novo or increased Cloaked-2 polypeptide production from the cell's endogenous Cloaked-2 gene. This method includes the introduction of a non-naturally occurring polypeptide (*e.g.*, a polypeptide comprising a site specific DNA binding domain fused to a transcriptional factor domain) into the cell such that de novo or increased Cloaked-2 polypeptide production from the cell's endogenous Cloaked-2 gene results.

The present invention further relates to DNA constructs useful in the method of altering expression of a target gene. In certain embodiments, the exemplary DNA constructs comprise: (a) one or more targeting sequences; (b) a regulatory sequence; (c) an exon; and (d) an unpaired splice-donor site. The targeting sequence in the DNA construct directs the integration of elements (a)-(d) into a target gene in a cell such that the elements (b)-(d) are operatively linked to sequences of the endogenous target gene. In another embodiment, the DNA constructs comprise: (a) one or more targeting sequences, (b) a regulatory sequence, (c) an exon, (d) a splice-donor site, (e) an intron, and (f) a splice-acceptor site, wherein the targeting sequence directs the integration of elements (a)-(f) such that the elements of (b)-(f) are operatively linked to the endogenous gene. The targeting sequence is homologous to the preselected site in the cellular chromosomal DNA with which homologous recombination is to occur. In the construct, the exon is generally 3' of the regulatory sequence and the splice-donor site is 3' of the exon.

If the sequence of a particular gene is known, such as the nucleic acid sequence of Cloaked-2 polypeptide presented herein, a piece of DNA that is complementary to a selected region of the gene can be synthesized or otherwise obtained, such as by appropriate restriction of the native DNA at specific recognition sites bounding the region of interest.

This piece serves as a targeting sequence(s) upon insertion into the cell and will hybridize to its homologous region within the genome. If this hybridization occurs during DNA replication, this piece of DNA, and any additional sequence attached thereto, will act as an Okazaki fragment and will be incorporated into the newly synthesized daughter strand of DNA. The present invention, therefore, includes nucleotides encoding a Cloaked-2 polypeptide, which nucleotides may be used as targeting sequences.

Cloaked-2 polypeptide cell therapy, *e.g.*, the implantation of cells producing Cloaked-2 polypeptides, is also contemplated. This embodiment involves implanting cells capable of synthesizing and secreting a biologically active form of Cloaked-2 polypeptide. Such Cloaked-2 polypeptide-producing cells can be cells that are natural producers of Cloaked-2 polypeptides or may be recombinant cells whose ability to produce . Cloaked-2 polypeptides has been augmented by transformation with a gene encoding the desired Cloaked-2 polypeptide or with a gene augmenting the expression of Cloaked-2 polypeptide. Such a modification may be accomplished by means of a vector suitable for delivering the gene as well as promoting its expression and secretion. In order to minimize a potential immunological reaction in patients being administered a Cloaked-2 polypeptide, as may occur with the administration of a polypeptide of a foreign species, it is preferred that the natural cells producing Cloaked-2 polypeptide be of human origin and produce human Cloaked-2 polypeptide. Likewise, it is preferred that the recombinant cells producing Cloaked-2 polypeptide be transformed with an expression vector containing a gene encoding a human Cloaked-2 polypeptide.

Implanted cells may be encapsulated to avoid the infiltration of surrounding tissue. Human or non-human animal cells may be implanted in patients in biocompatible, semipermeable polymeric enclosures or membranes that allow the release of Cloaked-2 polypeptide, but that prevent the destruction of the cells by the patient's immune system or by other detrimental factors from the surrounding tissue. Alternatively, the patient's own cells, transformed to produce Cloaked-2 polypeptides *ex vivo,* may be implanted directly into the patient without such encapsulation.

Techniques for the encapsulation of living cells are known in the art, and the preparation of the encapsulated cells and their implantation in patients may be routinely accomplished. For example, Baetge *et al.* (WO95/05452; PCT/US94/09299) describe membrane capsules containing genetically engineered cells for the effective delivery of biologically active molecules. The capsules are biocompatible and are easily retrievable. The capsules encapsulate cells transfected with recombinant DNA molecules comprising DNA sequences coding for biologically active molecules operatively linked to promoters that are not subject to down regulation *in vivo* upon implantation into a mammalian host. The devices provide for the delivery of the molecules from living cells to specific sites within a recipient. In addition, see U.S. Patent Nos. 4,892,538, 5,011,472, and 5,106,627. A system for encapsulating living cells is described in PCT Application no. PCT/US91/00157 of Aebischer *et al.* See also, PCT Application no. PCT/US91/00155 of Aebischer *et al.*, Winn et al., Exper. Neurol., 113:322-329 (1991), Aebischer et al., Exper. Neurol., 111:269-275 (1991); and Tresco et al., ASAIO, 38:17-23 (1992).

*In vivo* and *in vitro* gene therapy delivery of Cloaked-2 polypeptides is also envisioned. One example of a gene therapy technique is to use the Cloaked-2 gene (either genomic DNA, cDNA, and/or synthetic DNA) encoding a Cloaked-2 polypeptide which may be operably linked to a constitutive or inducible promoter to form a "gene therapy DNA construct". The promoter may be homologous or heterologous to the endogenous Cloaked-2 gene, provided that it is active in the cell or tissue type into which the construct will be inserted. Other components of the gene therapy DNA construct may optionally include, DNA molecules designed for site-specific integration (*e.g.*, endogenous sequences useful for homologous recombination), tissue-specific promoter, enhancer(s) or silencer(s), DNA molecules capable of providing a selective advantage over the parent cell, DNA molecules useful as labels to identify transformed cells, negative selection systems, cell specific binding agents (as, for example, for cell targeting), cell-specific internalization factors, and transcription factors to enhance expression by a vector as well as factors to enable vector manufacture.

A gene therapy DNA construct can then be introduced into cells (either *ex vivo* or *in vivo*) using viral or non-viral vectors. One means for introducing the gene therapy DNA construct is by means of viral vectors as described herein. Certain vectors, such as retroviral vectors, will deliver the DNA construct to the chromosomal DNA of the cells, and the gene can integrate into the chromosomal DNA. Other vectors will function as episomes, and the gene therapy DNA construct will remain in the cytoplasm.

In yet other embodiments, regulatory elements can be included for the controlled expression of the Cloaked-2 gene in the target cell. Such elements are turned on in response to an appropriate effector. In this way, a therapeutic polypeptide can be expressed when desired. One conventional control means involves the use of small molecule dimerizers or rapalogs (as described in WO9641865 (PCT/US96/099486); W09731898 (PCT/US97/03137) and WO9731899 (PCT/US95/03157) used to dimerize chimeric proteins which contain a small molecule-binding domain and a domain capable of initiating biological process, such as a DNA-binding protein or transcriptional activation protein. The dimerization of the proteins can be used to initiate transcription of the transgene.

An alternative regulation technology uses a method of storing proteins expressed from the gene of interest inside the cell as an aggregate or cluster. The gene of interest is expressed as a fusion protein that includes a conditional aggregation domain which results in the retention of the aggregated protein in the endoplasmic reticulum. The stored proteins are stable and inactive inside the cell. The proteins can be released, however, by administering a drug (*e.g.*,

small molecule ligand) that removes the conditional aggregation domain and thereby specifically breaks apart the aggregates or clusters so that the proteins may be secreted from the cell. See, Science 287:816-817, and 826-830 (2000).

Other suitable control means or gene switches include, but are not limited to, the following systems. Mifepristone (RU486) is used as a progesterone antagonist. The binding of a modified progesterone receptor ligand-binding domain to the progesterone antagonist activates transcription by forming a dimer of two transcription factors which then pass into the nucleus to bind DNA. The ligand binding domain is modified to eliminate the ability of the receptor to bind to the natural ligand. The modified steroid hormone receptor system is further described in U.S. 5,364,791; W09640911, and WO9710337.

Yet another control system uses ecdysone (a fruit fly steroid hormone) which binds to and activates an ecdysone receptor (cytoplasmic receptor). The receptor then translocates to the nucleus to bind a specific DNA response element (promoter from ecdysone-responsive gene). The ecdysone receptor includes a transactivation domain/DNA-binding domain/ligand-binding domain to initiate transcription. The ecdysone system is further described in U.S. 5,514,578; WO9738117; WO9637609; and WO9303162.

Another control means uses a positive tetracycline-controllable transactivator. This system involves a mutated tet repressor protein DNA-binding domain (mutated tet R-4 amino acid changes which resulted in a reverse tetracycline-regulated transactivator protein, *i.e.*, it binds to a tet operator in the presence of tetracycline) linked to a polypeptide which activates transcription. Such systems are described in U.S. Patent Nos. 5,464,758; 5,650,298 and 5,654,168.

Additional expression control systems and nucleic acid constructs are described in U.S. Patent Nos. 5,741,679 and 5,834,186, to Innovir Laboratories Inc.

In *vivo* gene therapy may be accomplished by introducing the gene encoding a Cloaked-2 polypeptide into cells via local injection of a Cloaked-2 nucleic acid molecule or by other appropriate viral or non-viral delivery vectors. Hefti, Neurobiology, 25:1418-1435 (1994). For example, a nucleic acid molecule encoding a Cloaked-2 polypeptide may be contained in an adeno-associated virus (AAV) vector for delivery to the targeted cells (*e.g.*, Johnson, International Publication No. WO95/34670; International Application No. PCT/US95/07178). The recombinant AAV genome typically contains AAV inverted terminal repeats flanking a DNA sequence encoding a Cloaked-2 polypeptide operably linked to functional promoter and polyadenylation sequences.

Alternative suitable viral vectors include, but are not limited to, retrovirus, adenovirus, herpes simplex virus, lentivirus, hepatitis virus, parvovirus, papovavirus, poxvirus, alphavirus, coronavirus, rhabdovirus, paramyxovirus, and papilloma virus vectors. U.S. Patent No. 5,672,344 describes an *in vivo* viral-mediated gene transfer system involving a recombinant neurotrophic HSV-1 vector. U.S. Patent No. 5,399,346 provides examples of a process for providing a patient with a therapeutic protein by the delivery of human cells which have been treated *in vitro* to insert a DNA segment encoding a therapeutic protein. Additional methods and materials for the practice of gene therapy techniques are described in U.S. Patent No. 5,631,236 involving adenoviral vectors; U.S. Patent No. 5,672,510 involving retroviral vectors; and U.S. 5,635,399 involving retroviral vectors expressing cytokines.

Nonviral delivery methods include, but are not limited to, liposome-mediated transfer, naked DNA delivery (direct injection), receptor-mediated transfer (ligand-DNA complex), electroporation, calcium phosphate precipitation, and microparticle bombardment (*e.g.*, gene gun). Gene therapy materials and methods may also include the use of inducible promoters, tissue-specific enhancer-promoters, DNA sequences designed for site-specific integration, DNA sequences capable of providing a selective advantage over the parent cell, labels to identify transformed cells, negative selection systems and expression control systems (safety measures), cell-specific binding agents (for cell targeting), cell-specific internalization factors, and transcription factors to enhance expression by a vector as well as methods of vector manufacture. Such additional methods and materials for the practice of gene therapy techniques are described in U.S. Patent No. 4,970,154 involving electroporation techniques; WO96/40958 involving nuclear ligands; U.S. Patent No. 5,679,559 describing a lipoprotein-containing system for gene delivery; U.S. Patent No. 5,676,954 involving liposome carriers; U.S. Patent No. 5,593,875 concerning methods for calcium phosphate transfection; and U.S. Patent No. 4,945,050 wherein biologically active particles are propelled at cells at a speed whereby the particles penetrate the surface of the cells and become incorporated into the interior of the cells.

It is also contemplated that Cloaked-2 gene therapy or cell therapy can further include the delivery of one or more additional polypeptide(s) in the same or a different cell(s). Such cells may be separately introduced into the patient, or the cells may be contained in a single implantable device, such as the encapsulating membrane described above, or the cells may be separately modified by means of viral vectors.

A means to increase endogenous Cloaked-2 polypeptide expression in a cell via gene therapy is to insert one or more enhancer elements into the Cloaked-2 polypeptide promoter, where the enhancer element(s) can serve to increase transcriptional activity of the Cloaked-2 gene. The enhancer element(s) used will be selected based on the tissue in which one desires to activate the gene(s); enhancer elements known to confer promoter activation in that tissue will be selected. For example, if a gene encoding a Cloaked-2 polypeptide is to be "turned on" in T-cells, the *lck* promoter enhancer element may be used. Here, the functional portion of the transcriptional element to be added may be inserted into a fragment of DNA containing the Cloaked-2 polypeptide promoter (and optionally, inserted into a vector and/or 5' and/or 3' flanking sequence(s), etc.) using standard cloning techniques. This construct, known as a "homologous recombination construct", can then be introduced into the desired cells either *ex vivo* or *in vivo*.

Gene therapy also can be used to decrease Cloaked-2 polypeptide expression by modifying the nucleotide sequence of the endogenous promoter(s). Such modification is typically accomplished via homologous recombination methods. For example, a DNA molecule containing all or a portion of the promoter of the Cloaked-2 gene(s) selected for inactivation can be engineered to remove and/or replace pieces of the promoter that regulate transcription. For example the TATA box and/or the binding site of a transcriptional activator of the promoter may be deleted using standard molecular biology techniques; such deletion can inhibit promoter activity thereby repressing the transcription of the corresponding Cloaked-2 gene. The deletion of the TATA box or the transcription activator binding site in the promoter may be accomplished by generating a DNA construct comprising all or the relevant portion of the Cloaked-2 polypeptide promoter(s) (from the same or a related species as the Cloaked-2 gene(s) to be regulated) in which one or more of the TATA box and/or transcriptional activator binding site nucleotides are mutated via substitution, deletion and/or insertion of one or more nucleotides. As a result, the TATA box and/or activator binding site has decreased activity or is rendered completely inactive. The construct will typically contain at least about 500 bases of DNA that correspond to the native (endogenous) 5' and 3' DNA sequences adjacent to the promoter segment that has been modified. The construct may be introduced into the appropriate cells (either *ex vivo* or *in vivo*) either directly or via a viral vector as described herein. Typically, the integration of the construct into the genomic DNA of the cells will be via homologous recombination, where the 5' and 3' DNA sequences in the promoter construct can serve to help integrate the modified promoter region via hybridization to the endogenous chromosomal DNA.

Additional Uses of Cloaked-2 Nucleic Acids and Polypeptides

Nucleic acid molecules of the present invention (including those that do not themselves encode biologically active polypeptides) may be used to map the locations of the Cloaked-2 gene and related genes on chromosomes. Mapping may be done by techniques known in the art, such as PCR amplification and *in situ* hybridization.

Cloaked-2 nucleic acid molecules (including those that do not themselves encode biologically active polypeptides), may be useful as hybridization probes in diagnostic assays to test, either qualitatively or quantitatively, for the presence of a Cloaked-2 DNA or corresponding RNA in mammalian tissue or bodily fluid samples.

The Cloaked-2 polypeptides may be used (simultaneously or sequentially) in combination with one or more cytokines, growth factors, antibiotics, anti-inflammatories, and/or chemotherapeutic agents as is appropriate for the indication being treated.

Other methods may also be employed where it is desirable to inhibit the activity of one or more Cloaked-2 polypeptides. Such inhibition may be effected by nucleic acid molecules which are complementary to and hybridize to expression control sequences (triple helix formation) or to Cloaked-2 mRNA. For example, antisense DNA or RNA molecules, which have a sequence that is complementary to at least a portion of the selected Cloaked-2 gene(s) can be introduced into the cell. Anti-sense probes may be designed by available techniques using the sequence of Cloaked-2 polypeptide disclosed herein. Typically, each such antisense molecule will be complementary to the start site (5' end) of each selected Cloaked-2 gene. When the antisense molecule then hybridizes to the corresponding Cloaked-2 mRNA; translation of this mRNA is prevented or reduced. Anti-sense inhibitors provide information relating to the decrease or absence of a Cloaked-2 polypeptide in a cell or organism.

Alternatively, gene therapy may be employed to create a dominant-negative inhibitor of one or more Cloaked-2 polypeptides. In this situation, the DNA encoding a mutant polypeptide of each selected Cloaked-2 polypeptide can be prepared and introduced into the cells of a patient using either viral or non-viral methods as described herein. Each such mutant is typically designed to compete with endogenous polypeptide in its biological role.

In addition, a Cloaked-2 polypeptide, whether biologically active or not, may be used as an immunogen, that is, the polypeptide contains at least one epitope to which antibodies may be raised. Selective binding agents that bind to a Cloaked-2 polypeptide (as described herein) may be used for *in vivo* and *in vitro* diagnostic purposes, including, but not limited to, use in labeled form to detect the presence of Cloaked-2 polypeptide in a body fluid or cell sample. The antibodies may also be used to prevent, treat, or diagnose a number of diseases and disorders, including those recited herein. The antibodies may bind to a Cloaked-2 polypeptide so as to diminish or block at least one activity characteristic of a Cloaked-2 polypeptide, or may bind to a polypeptide to increase at least one activity characteristic of a Cloaked-2 polypeptide (including by increasing the pharmacokinetics of the Cloaked-2 polypeptide).

cDNAs encoding human and mouse Cloaked-2 polypeptide were deposited with the ATCC on March 31, 2000 having accession nos. PTA-1616 and PTA-1615, respectively.

The following examples are intended for illustration purposes only, and should not be construed as limiting the scope of the invention in any way.

EXAMPLE 1

Cloning Human Cloaked-2 cDNA

A sequence containing the full coding region of Cloaked-2 was assembled by computer from human genomic sequences. PCR primers were designed from this sequence and a sequence containing the full coding region of Cloaked-2 was amplified from cDNA using the following reaction mix and PCR conditions:

Template: ten microliters of Human Kidney Marathon Ready cDNA (Clontech Laboratories, Inc., Palo Alto, CA; catalog no. 7405-1).

Forward primer: 5'- tactggaaggtggcgtgccctcct -3' (SEQ ID NO:7).

Reverse primer: 5'- aaaccacgcgcagaggacagaaatgt -3' (SEQ ID NO:8).

Final concentration of each primer: 1.0 micromolar.

Final concentration of dNTPs: 200 micromolar.

Five units of Pfu polymerase (Stratagene Inc., La Jolla, CA).

Ten microliters of 10x Pfu reaction buffer (Stratagene Inc., La Jolla, CA).

Twenty microliters of GC melt (Clontech Laboratories, Inc., Palo Alto, CA; Advantage GC cDNA PCR kit; catalog no. K1907-1).

Final reaction volume: 100 microliters.

Cycling conditions: 94°C for forty-five seconds followed by 38 cycles of 94°C (twelve seconds), 62°C (thirty seconds), 72°C (fifty seconds), and then at the end of the 38th cycle an incubation at 72°C for three minutes.

The PCR reaction was run on an agarose gel and the 759-base pair Cloaked-2 PCR product was isolated from the agarose gel and blunt end cloned into pPCR-Script Amp SK(+) (Stratagene Inc., La Jolla, CA). A number of clones were sequenced and all were found to contain the full coding region of human Cloaked-2.

EXAMPLE 2

Cloning Mouse Cloaked-2 cDNA

Using the sequences of 3 rat Cloaked-2 ESTs, PCR primers homologous to regions of the rat Cloaked-2 coding region sequence were designed and used to amplify a region of the mouse Cloaked-2 coding region using the following reaction mix and PCR conditions:

Template: five microliters of Mouse Testis Marathon Ready cDNA (Clontech Laboratories, Inc., Palo Alto, CA; catalog no. 7455-1).

Forward primer: 5'- gccaggggtggcaagccttcaagaatgat -3' (SEQ ID NO:9).

Reverse primer: 5'- cgatccgggatgcagcggaagtcg -3' (SEQ ID NO:10).

Final concentration of each primer: 1.0 micromolar.

Final concentration of dNTPs: 200 micromolar.

One microliter of Advantage cDNA Polymerase Mix (Clontech Laboratories, Inc., Palo Alto, CA; catalog no. 8417-1).

Five microliters of 10x cDNA PCR Reaction Buffer (Clontech Laboratories, Inc., Palo Alto, CA; catalog no. 8417-1).

Final reaction volume: 50 microliters.

Cycling conditions: 94°C for sixty seconds followed by 35 cycles of 94°C (twenty-five seconds), 60°C (thirty seconds), 72°C (forty-five seconds), and then at the end of the 35th cycle an incubation at 72°C for seven minutes.

The PCR reaction was run on an agarose gel and a 340-base pair product was isolated from the agarose gel and cloned into pCR-TOPO 2.1 (Invitrogen Inc., Carlsbad, CA; TOPO TA Cloning Kit, catalog no. 45-0641). Sequencing identified several clones containing sequences which encoded a polypeptide highly related to human Cloaked-2, thus identifying the inserts in the clones in question as containing part of the mouse Cloaked-2 cDNA sequence. Primers were designed from this mouse sequence for use in 5' and 3' RACE reactions with the goal of obtaining the sequence of the full coding region of mouse Cloaked-2.

5' RACE was done using the following reaction mix and PCR conditions:

5' RACE Primary PCR:

Template: five microliters of Mouse Testis Marathon Ready cDNA (Clontech Laboratories, Inc., Palo Alto, CA; catalog no. 7455-1).

Forward primer: 5'- ccatcctaatacgactcactatagggc - 3' (SEQ ID NO:11).

Reverse primer: 5'- tgtcaggaagcgggtgtagtgcag -3' (SEQ ID NO:12).

Final concentration of each primer: 1.0 micromolar.

Final concentration of dNTPs: 200 micromolar.

One microliter of Advantage GC cDNA Polymerase Mix (Clontech Laboratories, Inc., Palo Alto, CA; Advantage GC cDNA PCR Kit, catalog no. K1907-1).

Ten microliters of 5x GC cDNA PCR Reaction Buffer (Clontech Laboratories, Inc., Palo Alto, CA; Advantage GC cDNA PCR Kit, catalog no. K1907-1).

Two microliters of GC-Melt (Clontech Laboratories, Inc., Palo Alto, CA; Advantage GC cDNA PCR Kit, catalog no. K1907-1)

Final reaction volume: 50 microliters.

Cycling conditions: 94°C for sixty seconds followed by 5 cycles of 94°C (fifteen seconds), 70°C (ninety seconds), and then 5 cycles of 94°C (fifteen seconds), 68°C (ninety seconds), and then 30 cycles of 94°C (fifteen seconds), 66°C (ninety seconds) and then at the end of the 30th cycle an incubation at 70°C for five minutes.

5' RACE Nested PCR:

Template: one microliters of the above described primary 5'RACE Primary PCR.

Forward primer: 5'- actcactatagggctcgagcggc -3' (SEQ ID NO:13).

Reverse primer: 5'- ggacacatctttggcgtcataggga -3' (SEQ ID NO:14).

Final concentration of each primer: 1.0 micromolar.

Final concentration of dNTPs: 200 micromolar.

One microliter of Advantage GC cDNA Polymerase Mix (Clontech Laboratories, Inc., Palo Alto, CA; Advantage GC cDNA PCR Kit, catalog no. K1907-1).

Ten microliters of 5x GC cDNA PCR Reaction Buffer (Clontech Laboratories, Inc., Palo Alto, CA; Advantage GC cDNA PCR Kit, catalog no. K1907-1).

Two microliters of GC-Melt (Clontech Laboratories, Inc., Palo Alto, CA; Advantage GC cDNA PCR Kit, catalog no. K1907-1).

Final reaction volume: 50 microliters.

Cycling conditions: 94°C for sixty seconds followed by 3 cycles of 94°C (fifteen seconds), 70°C (ninety seconds), and then 3 cycles of 94°C (fifteen seconds), 68°C (ninety seconds), and then 25 cycles of 94°C (fifteen seconds), 66°C (ninety seconds) and then at the end of the 25th cycle an incubation at 70°C for five minutes.

The 5' RACE Nested PCR reaction was purified and cloned into pCR4-TOPO (Invitrogen Inc., Carlsbad, CA; TOPO TA Cloning Kit for Sequencing, catalog no. 45-0030). Sequencing identified a clone containing sequences homologous to mouse Cloaked-2.

3' RACE was done using the following reaction mix and PCR conditions:

3' RACE Primary PCR:

Template: five microliters of Mouse Testis Marathon Ready cDNA (Clontech Laboratories, Inc., Palo Alto, CA; catalog no. 7455-1).

Forward primer: 5'- tacacccgcttcctgacagac -3' (SEQ ID NO:15).

Reverse primer: 5'- ccatcctaatacgactcactatagggc - 3' (SEQ ID NO:16).

Final concentration of each primer: 1.0 micromolar.

Final concentration of dNTPs: 200 micromolar.

One microliter of Advantage GC cDNA Polymerase Mix (Clontech Laboratories, Inc., Palo Alto, CA; Advantage GC cDNA PCR Kit, catalog no. K1907-1).

Ten microliters of 5x GC cDNA PCR Reaction Buffer (Clontech Laboratories, Inc., Palo Alto, CA; Advantage GC cDNA PCR Kit, catalog no. K1907-1).

Five microliters of GC-Melt (Clontech Laboratories, Inc., Palo Alto, CA; Advantage GC cDNA PCR Kit, catalog no. K1907-1).

Final reaction volume: 50 microliters.

Cycling conditions: 94°C for sixty seconds followed by 5 cycles of 94°C (fifteen seconds), 70°C (ninety seconds), and then 5 cycles of 94°C (fifteen seconds), 68°C (ninety seconds), and then 30 cycles of 94°C (fifteen seconds), 66°C (ninety seconds) and then at the end of the 30th cycle an incubation at 70°C for five minutes.

<u>3' RACE Nested PCR:</u>

Template: one microliters of the above described 3'RACE Primary PCR.

Forward primer: 5'- ggtcaccgagttggtgtgctc -3' (SEQ ID NO:17).

Reverse primer: 5'- actcactatagggctcgagcggc -3' (SEQ ID NO:18).

Final concentration of each primer: 1.0 micromolar.

Final concentration of dNTPs: 200 micromolar.

One microliter of Advantage GC cDNA Polymerase Mix (Clontech Laboratories, Inc., Palo Alto, CA; Advantage GC cDNA PCR Kit, catalog no. K1907-1).

Ten microliters of 5x GC cDNA PCR Reaction Buffer (Clontech Laboratories, Inc., Palo Alto, CA; Advantage GC cDNA PCR Kit, catalog no. K1907-1).

Five microliters of GC-Melt (Clontech Laboratories, Inc., Palo Alto, CA; Advantage GC cDNA PCR Kit, catalog no. K1907-1).

Final reaction volume: 50 microliters. Cycling conditions: 94°C for sixty seconds followed by 3 cycles of 94°C (fifteen seconds), 70°C (ninety seconds), and then 3 cycles of 94°C (fifteen seconds), 68°C (ninety seconds), and then 25 cycles of 94°C (fifteen seconds), 66°C (ninety seconds) and then at the end of the 25th cycle an incubation at 70°C for five minutes.

The 3' RACE Nested PCR reaction was purified and cloned into pCR4-TOPO (Invitrogen Inc., Carlsbad, CA; TOPO TA Cloning Kit for Sequencing, catalog no. 45-0030). Sequencing identified several clones containing sequences homologous to mouse Cloaked-2.

Sequences from the 5' RACE, the 340-base pair above mentioned mouse Cloaked-2 PCR product and the 3' RACE were assembled by computer into a contig. Comparison of this mouse contig sequence with the sequence of human Cloaked-2 indicated that the full coding region of mouse Cloaked-2 was present in the mouse contig.

PCR primers were designed from the mouse contig in order to clone the full coding region of mouse Cloaked-2 as a single fragment using the following reaction mix and PCR conditions:

Template: ten microliters of Mouse Testis Marathon Ready cDNA (Clontech Laboratories, Inc., Palo Alto, CA; catalog no. 7455-1).

<u>Forward primer:</u>

5'-cgtactagtaagcttccaccatgcagccctcactagccccgtgcc-3' (SEQ ID NO:19)

<u>Reverse primer</u>:

5'-tttggatcccgatcgctagtaggcgttctccagctccgcct-3' (SEQ ID NO:20).

Final concentration of each primer: 1.0 micromolar.

Final concentration of dNTPs: 200 micromolar.

Five units of Pfu polymerase (Stratagene, La Jolla, CA).

Ten microliters of 10x Pfu reaction buffer (Stratagene, La Jolla, CA).

Twenty microliters of GC melt (Clontech Laboratories, Inc., Palo Alto, CA; Advantage GC cDNA PCR kit; catalog no. K1907-1).

Final reaction volume: 100 microliters.

Cycling conditions: 95°C for ninety seconds followed by five cycles of 94°C (twelve seconds), 65°C (thirty seconds), 72°C (fifty seconds), and then followed by 35 cycles of 94°C (twelve seconds), 60°C (thirty seconds), 72°C (fifty seconds), and then at the end of the 35th cycle an incubation at 72°C for five minutes.

The PCR reaction was purified, cut with SpeI and BamHI and run on an agarose gel. The 665-base pair band was isolated from the gel and cloned into SpeI-BamHI double digested pBluescript II (KS-). A number of clones were sequenced and all were found to contain the full coding region of mouse Cloaked-2.

EXAMPLE 3

Presence and Distribution of mRNA in Different Tissues

A sequence containing the full coding region of Cloaked-2 was assembled by computer from human genomic sequences. PCR primers were designed from this sequence to amplify a 376-base pair coding region subfragment from cDNA using the following reaction mix and PCR conditions:

Template: ten microliters of Human Prostate Marathon Ready cDNA (Clontech Laboratories, Inc., Palo Alto, CA; catalog no. 7418-1).

Forward primer: 5'- tgtgtctcgtctgcctgctggtacaca - 3' (SEQ ID NO:21).

Reverse primer: 5'- gaagtcgggcccactaggtcgcc -3' (SEQ ID NO:22).

Final concentration of each primer: 1.0 micromolar.

Final concentration of dNTPs: 200 micromolar. One microliter of Advantage cDNA Polymerase Mix (Clontech Laboratories, Inc., Palo Alto, CA; catalog no. 8417-1).

Five microliters of 10x cDNA PCR Reaction Buffer (Clontech Laboratories, Inc., Palo Alto, CA; catalog no. 8417-1).

Final reaction volume: 50 microliters. Cycling conditions: 94°C for sixty seconds followed by 35 cycles of 94°C (twenty-five seconds), 70°C (thirty seconds), 72°C (sixty seconds), and then at the end of the 35th cycle an incubation at 72°C for ten minutes.

The PCR reaction was run on an agarose gel and the 376-base pair Cloaked-2 PCR product was isolated from the agarose gel and cloned into pCR-TOPO 2.1 (Invitrogen Inc., Carlsbad, CA; TOPO TA Cloning Kit, catalog no. 45-0641). The insert was sequenced to confirm that the expected sequence had been amplified and cloned. A 400-base pair EcoRI fragment containing the Cloaked-2 insert was isolated from the clone by agarose gel electrophoresis and was labeled with $^{32}P$ and hybridized to Clontech Human Multiple Tissue Northern Blots representing multiple tissues (brain, heart, skeletal muscle, colon, thymus, spleen, kidney, liver, small intestine, placenta, lung, peripheral blood leukocytes, prostate, testis, ovary, amygdala, caudate nucleus, corpus callosum, hippocampus, total brain, substantia nigra, thalamus, pancreas, adrenal medulla, thyroid, adrenal cortex and stomach) using high stringency conditions as follows:

Prehybridization was done overnight at 68°C using ExpressHyb Hybridization Solution (Clontech Laboratories, Inc., Palo Alto, CA; catalog no. 8015-2). Hybridization was done for ninety minutes at 68°C using fresh ExpressHyb Hybridization Solution containing the labeled Cloaked-2 probe. The blots were rinsed in 2x SSC, 0.05% SDS at room temperature twice for 1 minute each time. The blots were then rinsed in 2x SSC, 0.05% SDS at room temperature twice for 20 minutes each time. The blots were then rinsed in 0.1x SSC, 0.1% SDS at 50°C three times for 15 minutes each time and were subsequently exposed to film.

The results of the Northern analysis indicated that Cloaked-2 was expressed most strongly in kidney and heart. Somewhat weaker expression was detected in placenta and skeletal muscle. Lower expression was detected in liver, pancreas, thyroid, adrenal cortex, amygdala, and thalamus.

## Claims

1. An isolated nucleic acid molecule comprising a nucleotide sequence selected from the group consisting of:

   (a) the nucleotide sequence as set forth in SEQ ID NO:1 or SEQ ID NO:3;
   (b) a nucleotide sequence encoding the polypeptide as set forth in SEQ ID NO:2 or SEQ ID NO:4;
   (c) a nucleotide sequence which hybridizes under moderately or highly stringent conditions to the complement of (a) or (b), wherein the encoded polypeptide has an activity of the polypeptide as set forth in SEQ ID NO:2 or SEQ ID NO:4; and
   (d) a nucleotide sequence complementary to any of .(a) - (c) .

2. An isolated nucleic acid molecule comprising a nucleotide sequence selected from the group consisting of:

   (a) a nucleotide sequence encoding a polypeptide that is at least about 70, 75, 80, 85, 90, 95, 96, 97, 98, or 99 percent identical to the polypeptide as set forth in SEQ ID N0:2 or SEQ ID NO:4, wherein the polypeptide has an activity of the polypeptide as set forth in SEQ ID NO:2 or SEQ ID NO:4;
   (b) a nucleotide sequence encoding an allelic variant or splice variant of the nucleotide sequence as set forth in SEQ ID NO:1 or SEQ ID NO:3, wherein the encoded polypeptide has an activity of the polypeptide as set forth in SEQ ID NO:2 or SEQ ID NO:4;
   (c) a nucleotide sequence of SEQ ID NO:1 or SEQ ID NO:3; (a) or (b) encoding a polypeptide fragment of at least about 25 amino acid residues, wherein the polypeptide has an activity of the polypeptide as set forth in SEQ ID NO:2 or SEQ ID NO:4;
   (d) a nucleotide sequence of SEQ ID NO:1 or SEQ ID NO:3, or (a)-(c) comprising a fragment of at least about 16 nucleotides;
   (e) a nucleotide sequence which hybridizes under moderately or highly stringent conditions to the complement of any of (a)-(d), wherein the polypeptide has an activity of the polypeptide as set forth in SEQ ID NO:2 or SEQ ID NO:4; and
   (f) a nucleotide sequence complementary to any of (a)-(c).

3. An isolated nucleic acid molecule comprising a nucleotide sequence selected from the group consisting of:

   (a) a nucleotide sequence encoding a polypeptide as set forth in SEQ ID NO:2 or SEQ ID NO:4 with at least one conservative amino acid substitution, wherein the polypeptide has an activity of the polypeptide as set forth in SEQ ID NO:2 or SEQ ID NO:4;
   (b) a nucleotide sequence encoding a polypeptide as set forth in SEQ ID NO:2 or SEQ ID NO:4 with at least one amino acid insertion, wherein the polypeptide has an activity of the polypeptide as set forth in SEQ ID NO: 2 or SEQ ID NO:4;
   (c) a nucleotide sequence encoding a polypeptide as set forth in SEQ ID NO:2 or SEQ ID NO:4 with at least one amino acid deletion, wherein the polypeptide has an activity of the polypeptide as set forth in SEQ ID NO: 2 or SEQ ID NO:4;
   (d) a nucleotide sequence encoding a polypeptide as set forth in SEQ ID NO:2 or SEQ ID N0:4 which has a C- and/or N- terminal truncation, wherein the polypeptide has an activity of the polypeptide as set forth in SEQ ID NO:2 or SEQ ID NO:4;
   (e) a nucleotide sequence encoding a polypeptide as set forth in SEQ ID NO:2 or SEQ ID NO:4 with at least one modification selected from the group consisting of amino acid substitutions, amino acid insertions, amino acid deletions, C-terminal truncation, and N-terminal truncation, wherein the polypeptide has an activity of the polypeptide as set forth in SEQ ID NO:2 or SEQ ID NO:4;
   (f) a nucleotide sequence of (a)-(e) comprising a fragment of at least about 16 nucleotides;
   (g) a nucleotide sequence which hybridizes under moderately or highly stringent conditions to the complement of any of (a)-(f), wherein the polypeptide has an activity of the polypeptide as set forth in SEQ ID NO:2 or SEQ ID NO:4; and
   (h) a nucleotide sequence complementary to any of (a)-(e).

4. A vector comprising the nucleic acid molecule of Claims 1, 2, or 3.

5. A host cell comprising the vector of Claim 4.

6. The host cell of Claim 5 that is a eukaryotic cell.

**7.** The host cell of Claim 5 that is a prokaryotic cell.

**8.** A process of producing a Cloaked-2 polypeptide comprising culturing the host cell of Claim 5 under suitable conditions to express the polypeptide, and optionally isolating the polypeptide from the culture.

**9.** A polypeptide produced by the process of Claim 8.

**10.** The process of Claim 8, wherein the nucleic acid molecule comprises promoter DNA other than the promoter DNA for the native Cloaked-2 polypeptide operatively linked to the DNA encoding the Cloaked-2 polypeptide.

**11.** The isolated nucleic acid molecule according to Claim 2 wherein the percent identity is determined using a computer program selected from the group consisting of GAP, BLASTP, BLASTN, FASTA, BLASTA, BLASTX, BestFit, and the Smith-Waterman algorithm.

**12.** A process for determining whether a compound inhibits Cloaked-2 polypeptide activity or production comprising exposing a cell according to Claims 5, 6, or 7 to the compound, and measuring Cloaked-2 polypeptide activity or production in said host cell.

**13.** An isolated polypeptide comprising the amino acid sequence set forth in SEQ ID NO:2 or SEQ ID NO:4.

**14.** An isolated polypeptide comprising the amino acid sequence selected from the group consisting of:

(a) the mature amino acid sequence as set forth in SEQ ID NO:2 or SEQ ID NO:4, optionally further comprising an amino-terminal methionine;
(b) an amino acid sequence for an ortholog of SEQ ID NO:2 or SEQ ID NO:4, wherein the encoded polypeptide has an activity of the polypeptide as set forth in SEQ ID NO:2 or SEQ ID NO:4;
(c) an amino acid sequence that is at least about 70, 80, 85, 90, 95, 96, 97, 98, or 99 percent identical to the amino acid sequence of SEQ ID NO:2 or SEQ ID NO:4, wherein the polypeptide has an activity of the polypeptide as set forth in SEQ ID NO:2 or SEQ ID NO:4;
(d) a fragment of the amino acid sequence set forth in SEQ ID NO:2 or SEQ ID NO:4 comprising at least about 25 amino acid residues, wherein the polypeptide has an activity of the polypeptide as set forth in SEQ ID NO: 2 or SEQ ID NO:4;
(e) an amino acid sequence for an allelic variant or splice variant of either the amino acid sequence as set forth in SEQ ID NO:2 or SEQ ID NO:4, or at least one of (a)-(c) wherein the polypeptide has an activity of the polypeptide as set forth in SEQ ID NO:2 or SEQ ID NO:4.

**15.** An isolated polypeptide comprising the amino acid sequence selected from the group consisting of:

(a) the amino acid sequence as set forth in SEQ ID NO:2 or SEQ ID NO:4 with at least one conservative amino acid substitution, wherein the polypeptide has an activity of the polypeptide as set forth in SEQ ID NO:2 or SEQ ID NO:4;
(b) the amino acid sequence as set forth in SEQ ID NO:2 or SEQ ID NO:4 with at least one amino acid insertion, wherein the polypeptide has an activity of the polypeptide as set forth in SEQ ID NO:2 or SEQ ID NO:4;
(c) the amino acid sequence as set forth in SEQ ID NO:2 or SEQ ID NO:4 with at least one amino acid deletion, wherein the polypeptide has an activity of the polypeptide as set forth in SEQ ID NO:2 or SEQ ID NO:4;
(d) the amino acid sequence as set forth in SEQ ID NO:2 or SEQ ID NO:4 which has a C- and/or N-terminal truncation, wherein the polypeptide has an activity of the polypeptide as set forth in SEQ ID NO:2 or SEQ ID NO:4; and
(e) the amino acid sequence as set forth in SEQ ID NO:2 or SEQ ID NO:4, with at least one modification selected from the group consisting of amino acid substitutions, amino acid insertions, amino acid deletions, C-terminal truncation, and N-terminal truncation, wherein the polypeptide has an activity of the polypeptide as set forth in SEQ ID NO:2 or SEQ ID NO:4.

**16.** A polypeptide according to claim 14 or 15 wherein the amino acid at position 9 of SEQ ID NO: 2 is aspartic acid or glutamic acid.

**17.** A polypeptide according to claim 14 or 15 wherein the amino acid at position 39 of SEQ ID NO: 2 is glycine, proline, or alanine.

**18.** A polypeptide according to claim 14 or 15 wherein the amino acid at position 58 of SEQ ID NO: 2 is arginine, lysine, glutamine, or asparagine.

**19.** A polypeptide according to claim 14 or 15 wherein the amino acid at position 81 of SEQ ID NO: 2 is valine, isoleucine, methionine, leucine, phenylalanine, alanine, or norleucine.

**20.** A polypeptide according to claim 14 or 15 wherein the amino acid at position 102 of SEQ ID NO: 2 is tryptophan, tyrosine, or phenylalanine.

**21.** A polypeptide according to claim 14 or 15 wherein the amino acid at position 154 of SEQ ID NO: 2 is serine, threonine, or alanine.

**22.** An isolated polypeptide encoded by the nucleic acid molecule of Claims 1, 2, or 3.

**23.** The isolated polypeptide according to Claim 14 wherein the percent identity is determined using a computer program selected from the group consisting of GAP, BLASTP, BLASTN, FASTA, BLASTA, BLASTX, BestFit, and the Smith-Waterman algorithm.

**24.** An antibody produced by immunizing an animal with a peptide comprising an amino acid sequence of SEQ ID NO: 2 or SEQ ID NO:4.

**25.** An antibody or fragment thereof that specifically binds the polypeptide of Claims 13, 14, or 15.

**26.** The antibody of Claim 25 that is a monoclonal antibody.

**27.** A hybridoma that produces a monoclonal antibody that binds to a peptide comprising an amino acid sequence of SEQ ID NO:2 or SEQ ID NO:4.

**28.** A method of detecting or quantitating the amount of Cloaked-2 polypeptide using the anti-Cloaked-2 antibody or fragment of Claims 24, 25, or 26.

**29.** A selective binding agent or fragment thereof that specifically binds at least one polypeptide wherein said polypeptide comprises the amino acid sequence selected from the group consisting of:

a) the amino acid sequence as set forth in SEQ ID NO:2 or SEQ ID NO:4; and
b) a fragment of the amino acid sequence set forth in at least one of SEQ ID NO:2 or SEQ ID NO:4; and
c) a naturally occurring variant of (a) or (b).

**30.** The selective binding agent of Claim 29 that is an antibody or a fragment thereof.

**31.** The selective binding agent of Claim 29 that is a humanized antibody.

**32.** The selective binding agent of Claim 29 that is a human antibody or a fragment thereof.

**33.** The selective binding agent of Claim 29 that is a polyclonal antibody or a fragment thereof.

**34.** The selective binding agent of Claim 29 that is a monoclonal antibody or a fragment thereof.

**35.** The selective binding agent of Claim 29 that is a chimeric antibody or a fragment thereof.

**36.** The selective binding agent of Claim 29 that is a CDR-grafted antibody or a fragment thereof.

**37.** The selective binding agent of Claim 29 that is an antiidiotypic antibody or a fragment thereof.

**38.** The selective binding agent of Claim 29 which is a variable region fragment.

**39.** The variable region fragment of Claim 38 which is a Fab or a Fab’ fragment.

**40.** A selective binding agent or fragment thereof comprising at least one complementarity-determining region with specificity for a polypeptide having the amino acid sequence of SEQ ID NO:2 or SEQ ID NO:4.

**41.** The selective binding agent of Claim 29 which is bound to a detectable label.

**42.** The selective binding agent of Claim 29 which antagonizes Cloaked-2 polypeptide biological activity.

**43.** A method for treating, preventing, or ameliorating a disease, condition, or disorder comprising administering to a patient an effective amount of a selective binding agent according to Claim 29.

**44.** A selective binding agent produced by immunizing an animal with a polypeptide comprising an amino acid sequence selected from the group consisting of SEQ ID NO:2 or SEQ ID NO:4.

**45.** A hybridoma that produces a selective binding agent capable of binding a polypeptide according to Claims 1, 2, or 3.

**46.** A composition comprising the polypeptide of Claims 13, 14, or 15 and a pharmaceutically acceptable formulation agent.

**47.** The composition of Claim 46 wherein the pharmaceutically acceptable formulation agent is a carrier, adjuvant, solubilizer, stabilizer, or antioxidant.

**48.** The composition of Claim 46 wherein the polypeptide comprises the mature amino acid sequence as set forth in SEQ ID NO:2 or SEQ ID NO:4.

**49.** A polypeptide comprising a derivative of the polypeptide of Claims 13, 14, or 15.

**50.** The polypeptide of Claim 49 which is covalently modified with a water-soluble polymer.

**51.** The polypeptide of Claim 50 wherein the water-soluble polymer is selected from the group consisting of polyethylene glycol, monomethoxy-polyethylene glycol, dextran, cellulose, poly-(N-vinyl pyrrolidone) polyethylene glycol, propylene glycol homopolymers, polypropylene oxide/ethylene oxide copolymers, polyoxyethylated polyols, and polyvinyl alcohol.

**52.** A composition comprising a nucleic acid molecule of Claims 1, 2, or 3 and a pharmaceutically acceptable formulation agent.

**53.** A composition of Claim 52 wherein said nucleic acid molecule is contained in a viral vector.

**54.** A viral vector comprising a nucleic acid molecule of Claims 1, 2, or 3.

**55.** A fusion polypeptide comprising the polypeptide of Claims 13, 14, or 15 fused to a heterologous amino acid sequence.

**56.** The fusion polypeptide of Claim 55 wherein the heterologous amino acid sequence is an IgG constant domain or a fragment thereof.

**57.** A method for treating, preventing or ameliorating a medical condition comprising administering to a patient the polypeptide of Claims 13, 14, or 15 or the polypeptide encoded by the nucleic acid of Claims 1, 2, or 3.

**58.** A method of diagnosing a pathological condition or a susceptibility to a pathological condition in a subject comprising:

(a) determining the presence or amount of expression of the polypeptide of Claims 13, 14, or 15 or the polypeptide encoded by the nucleic acid molecule of Claims 1, 2, or 3 in a sample; and
(b) diagnosing a pathological condition or a susceptibility to a pathological condition based on the presence or amount of expression of the polypeptide.

**59.** A device, comprising:

(a) a membrane suitable for implantation; and

(b) cells encapsulated within said membrane,

wherein said cells secrete a protein of Claims 13, 14, or 15, and wherein said membrane is permeable to said protein and impermeable to materials detrimental to said cells.

**60.** A method of identifying a compound which binds to a polypeptide comprising:

(a) contacting the polypeptide of Claims 13, 14, or 15 with a compound; and
(b) determining the extent of binding of the polypeptide to the compound.

**61.** A method of modulating levels of a polypeptide in an animal comprising administering to the animal the nucleic acid molecule of Claims 1, 2, or 3.

**62.** A transgenic non-human mammal comprising the nucleic acid molecule of Claims 1, 2, or 3.

# Figure 1

## A. Nucleic acid encoding human Cloaked-2 polypeptide with signal peptide (SEQ ID NO:1)

```
  1 TACTGGAAGGTGGCGTGCCCTCCTCTGGCTGGTACCATGCAGCTCCCACT
 51 GGCCCTGTGTCTCGTCTGCCTGCTGGTACACACAGCCTTCCGTGTAGTGG
101 AGGGCCAGGGGTGGCAGGCGTTCAAGAATGATGCCACGGAAATCATCCCC
151 GAGCTCGGAGAGTACCCCGAGCCTCCACCGGAGCTGGAGAACAACAAGAC
201 CATGAACCGGGCGGAGAACGGAGGGCGGCCTCCCCACCACCCCTTTGAGA
251 CCAAAGACGTGTCCGAGTACAGCTGCCGCGAGCTGCACTTCACCCGCTAC
301 GTGACCGATGGGCCGTGCCGCAGCGCCAAGCCGGTCACCGAGCTGGTGTG
351 CTCCGGCCAGTGCGGCCCGGCGCGCCTGCTGCCCAACGCCATCGGCCGCG
401 GCAAGTGGTGGCGACCTAGTGGGCCCGACTTCCGCTGCATCCCCGACCGC
451 TACCGCGCGCAGCGCGTGCAGCTGCTGTGTCCCGGTGGTGAGGCGCCGCG
501 CGCGCGCAAGGTGCGCCTGGTGGCCTCGTGCAAGTGCAAGCGCCTCACCC
551 GCTTCCACAACCAGTCGGAGCTCAAGGACTTCGGGACCGAGGCCGCTCGG
601 CCGCAGAAGGGCCGGAAGCCGCGGCCCCGCGCCCGGAGCGCCAAAGCCAA
651 CCAGGCCGAGCTGGAGAACGCCTACTAGAGCCCGCCCGCGCCCCTCCCCA
701 CCGGCGGGCGCCCCGGCCCTGAACCCGCGCCCCACATTTCTGTCCTCTGC
751 GCGTGGTTT
```

## B. Human Cloaked-2 polypeptide most likely mature form (SEQ ID NO:2)

```
  1 QGWQAFKNDATEIIPELGEYPEPPPELENNKTMNRAENGGRPPHHPFETK
 51 DVSEYSCRELHFTRYVTDGPCRSAKPVTELVCSGQCGPARLLPNAIGRGK
101 WWRPSGPDFRCIPDRYRAQRVQLLCPGGEAPRARKVRLVASCKCKRLTRF
151 HNQSELKDFGTEAARPQKGRKPRPRARSAKANQAELENAY
```

## C. Human Cloaked-2 polypeptide with signal peptide (SEQ ID NO:5)

```
  1 MQLPLALCLVCLLVHTAFRVVEGQGWQAFKNDATEIIPELGEYPEPPPEL
 51 ENNKTMNRAENGGRPPHHPFETKDVSEYSCRELHFTRYVTDGPCRSAKPV
101 TELVCSGQCGPARLLPNAIGRGKWWRPSGPDFRCIPDRYRAQRVQLLCPG
151 GEAPRARKVRLVASCKCKRLTRFHNQSELKDFGTEAARPQKGRKPRPRAR
201 SAKANQAELENAY
```

# Figure 2

## A. Nucleic acid encoding mouse Cloaked-2 polypeptide with signal peptide (SEQ ID NO:3)

```
  1 ATGCAGCCCTCACTAGCCCCGTGCCTCATCTGCCTACTTGTGCACGCTGC
 51 CTTCTGTGCTGTGGAGGGCCAGGGGTGGCAAGCCTTCAGGAATGATGCCA
101 CAGAGGTCATCCCAGGGCTTGGAGAGTACCCCGAGCCTCCTCCTGAGAAC
151 AACCAGACCATGAACCGGGCGGAGAATGGAGGCAGACCTCCCCACCATCC
201 CTATGACGCCAAAGATGTGTCCGAGTACAGCTGCCGCGAGCTGCACTACA
251 CCCGCTTCCTGACAGACGGCCCATGCCGCAGCGCCAAGCCGGTCACCGAG
301 TTGGTGTGCTCCGGCCAGTGCGGCCCCGCGCGGCTGCTGCCCAACGCCAT
351 CGGGCGCGTGAAGTGGTGGCGCCCGAACGGACCGGATTTCCGCTGCATCC
401 CGGATCGCTACCGCGCGCAGCGGGTGCAGCTGCTGTGCCCCGGGGGCGCG
451 GCGCCGCGCTCGCGCAAGGTGCGTCTGGTGGCCTCGTGCAAGTGCAAGCG
501 CCTCACCCGCTTCCACAACCAGTCGGAGCTCAAGGACTTCGGGCCGGAGA
551 CCGCGCGGCCGCAGAAGGGTCGCAAGCCGCGGCCCGGCGCCCGGGGAGCC
601 AAAGCCAACCAGGCGGAGCTGGAGAACGCCTACTAG
```

## B. Mouse Cloaked-2 polypeptide most likely mature form (SEQ ID NO:4)

```
  1 QGWQAFRNDATEVIPGLGEYPEPPPENNQTMNRAENGGRPPHHPYDAKDV
 51 SEYSCRELHYTRFLTDGPCRSAKPVTELVCSGQCGPARLLPNAIGRVKWW
101 RPNGPDFRCIPDRYRAQRVQLLCPGGAAPRSRKVRLVASCKCKRLTRFHN
151 QSELKDFGPETARPQKGRKPRPGARGAKANQAELENAY
```

## C. Mouse Cloaked-2 polypeptide with signal peptide (SEQ ID NO:6)

```
  1 MQPSLAPCLICLLVHAAFCAVEGQGWQAFRNDATEVIPGLGEYPEPPPEN
 51 NQTMNRAENGGRPPHHPYDAKDVSEYSCRELHYTRFLTDGPCRSAKPVTE
101 LVCSGQCGPARLLPNAIGRVKWWRPNGPDFRCIPDRYRAQRVQLLCPGGA
151 APRSRKVRLVASCKCKRLTRFHNQSELKDFGPETARPQKGRKPRPGARGA
201 KANQAELENAY
```

## Figure 3

```
GAP of: Human Cloaked-2  check: 5775  from: 1  to: 213
    to: Mouse Cloaked-2  check: 9489  from: 1  to: 211

Symbol comparison table:
/GCGDISK/gcg10/gcgcore/data/rundata/blosum62.cmp
CompCheck: 6430

         Gap Weight:      8        Average Match:  2.912
      Length Weight:      2     Average Mismatch: -2.003

            Quality:   1028              Length:    213
              Ratio:  4.872                Gaps:      1
 Percent Similarity: 91.469    Percent Identity: 88.152

        Match display thresholds for the alignment(s):
                    | = IDENTITY
                    : =   2
                    . =   1
```

Human Cloaked-2 (SEQ ID NO: 5) x
Mouse Cloaked-2 (SEQ ID NO: 6)

```
  1 MQLPLALCLVCLLVHTAFRVVEGQGWQAFKNDATEIIPELGEYPEPPPEL 50
    ||  || ||:||||| ||  |||||||||:|||||:|| |||||||||
  1 MQPSLAPCLICLLVHAAFCAVEGQGWQAFRNDATEVIPGLGEYPEPPP.. 48

 51 ENNKTMNRAENGGRPPHHPFETKDVSEYSCRELHFTRYVTDGPCRSAKPV 100
    |||.|||||||||||||||:: ||||||||||||:||:.|||||||||||
 49 ENNQTMNRAENGGRPPHHPYDAKDVSEYSCRELHYTRFLTDGPCRSAKPV 98

101 TELVCSGQCGPARLLPNAIGRGKWWRPSGPDFRCIPDRYRAQRVQLLCPG 150
    ||||||||||||||||||||| |||||.||||||||||||||||||||||
 99 TELVCSGQCGPARLLPNAIGRVKWWRPNGPDFRCIPDRYRAQRVQLLCPG 148

151 GEAPRARKVRLVASCKCKRLTRFHNQSELKDFGTEAARPQKGRKPRPRAR 200
    | |||.||||||||||||||||||||||||||| | ||||||||||| ||
149 GAAPRSRKVRLVASCKCKRLTRFHNQSELKDFGPETARPQKGRKPRPGAR 198

201 SAKANQAELENAY 213
     ||||||||||||
199 GAKANQAELENAY 211
```

## Figure 4

```
GAP of: Human Cloaked-1  check: 1888  from: 1  to: 183
    to: Human Cloaked-2  check: 185  from: 1  to: 190

  Symbol comparison table:
/GCGDISK/gcg10/gcgcore/data/rundata/blosum62.cmp
  CompCheck: 6430

        Gap Weight:      8      Average Match:  2.912
     Length Weight:      2    Average Mismatch: -2.003

           Quality:    335            Length:    196
             Ratio:  1.831              Gaps:      6
  Percent Similarity: 52.542  Percent Identity: 42.938

        Match display thresholds for the alignment(s):
                    | = IDENTITY
                    : =   2
                    . =   1
```

Human Cloaked-1 (SEQ ID NO: 25) x
Human Cloaked-2 (SEQ ID NO: 2)

```
                .         .         .         .         .
   1 .....FKNDATEILYSHVVKP.VPAHPSSNSTLNQARNGGRHFSNTGLDR 44
          ||||||||:      |  |    .| |:|.| ||||   .   :
   1 QGWQAFKNDATEIIPELGEYPEPPPELENNKTMNRAENGGRP.PHHPFET 49
                .         .         .         .         .
  45 NTRVQVGCRELRSTKYISDGQCTSISPLKELVCAGECLPLPVLPNWIGGG 94
         :  ||||  |:|:.|| | |  |. |||||.|:| |  .||| || |
  50 KDVSEYSCRELHFTRYVTDGPCRSAKPVTELVCSGQCGPARLLPNAIGRG 99
                .         .         .         .         .
  95 YGTKYWSRRSSQEWRCVNDKTRTQRIQLQCQDG.STRTYKITVVTACKCK 143
        |:| | |  :.||: |: | ||:|| |  | . |  |: .| .||||
 100 ...KWW.RPSGPDFRCIPDRYRAQRVQLLCPGGEAPRARKVRLVASCKCK 145
                .         .         .         .
 144 RYTRQHNESSHNFESMSPAKPVQHHRERKRASKSSKHSMS...... 183
     | || ||:|         |:| .  : | || :|.| . .
 146 RLTRFHNQSELKDFGTEAARPQKGRKPRPRA.RSAKANQAELENAY 190
```

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20855000 P
- US 22354200 P
- US 5780263 A, Hastings
- US 5480981 A
- US 5808029 A
- WO 9723614 A
- US 9723183 W
- WO 9014363 A
- US 5824469 A
- US 5763192 A
- US 5814476 A
- US 5723323 A
- US 5817483 A
- US 5234784 A
- EP 0154316 A
- EP 0401384 A
- US 4179337 A
- US 5252714 A
- US 5557032 A
- US 5489743 A
- WO 9428122 A
- US 4816567 A
- US 5585089 A
- US 5693762 A
- US 9605928 W
- US 9306926 W
- US 5545807 A
- US 91245 W
- GB 8901207 W
- EP 546073 B1
- EP 546073 A1
- US 9817364 W
- US 4376110 A
- US 94001875 W
- US 9300829 W
- US 3773919 A
- EP 58481 A
- EP 133988 A
- EP 36676 A
- EP 88046 A
- EP 143949 A
- US 5272071 A
- EP 9193051 A
- EP 505500 A
- US 9007642 W
- WO 9109955 A
- WO 9505452 A
- US 9409299 W
- US 4892538 A
- US 5011472 A
- US 5106627 A
- US 9100157 W
- US 9100155 W
- WO 9641865 A
- US 96099486 W
- WO 9731898 A
- US 9703137 W
- WO 9731899 A
- US 9503157 W
- US 5364791 A
- WO 9640911 A
- WO 9710337 A
- US 5514578 A
- WO 9738117 A
- WO 9637609 A
- WO 9303162 A
- US 5464758 A
- US 5650298 A
- US 5654168 A
- US 5741679 A
- US 5834186 A
- WO 9534670 A
- US 9507178 W
- US 5672344 A
- US 5399346 A
- US 5631236 A
- US 5672510 A
- US 5635399 A
- US 4970154 A
- WO 9640958 A
- US 5679559 A
- US 5676954 A
- US 5593875 A
- US 4945050 A

### Non-patent literature cited in the description

- **ISAACS.** *Current Opinion in Structural Biology,* 1995, vol. 5, 391-395
- **H. SIMONSEN ; H.F. LODISH.** *Trends in Pharmacological Sciences,* 1994, vol. 15, 437-441

- **TARTAGLIA et al.** *Cell,* 1995, vol. 83, 1263-1271
- **GRAHAM et al.** *Virology,* 1973, vol. 52, 456
- **SAMBROOK et al.** Molecular Cloning, a laboratory Manual. Cold Spring Harbor Laboratories, 1989
- **DAVIS et al.** Basic Methods in Molecular Biology. Elsevier, 1986
- **CHU et al.** *Gene,* 1981, vol. 13, 197
- **SAMBROOK ; FRITSCH ; MANIATIS.** Molecular Cloning: A Laboratory Manual. Cold Spring Harbor Laboratory, 1989
- **ANDERSON et al.** Nucleic Acid Hybridisation: a practical approach. IRL Press Limited
- **ANDERSON et al.** Nucleic Acid Hybridisation: A Practical Approach. IRL Press Limited
- **SUGGS et al.** Developmental Biology Using Purified Genes. 1981, 683
- **KYTE et al.** *J. Mol. Biol.,* 1982, vol. 157, 105-131
- **MOULT J.** *Curr. Op. in Biotech,* 1996, vol. 7 (4), 422-427
- **CHOU et al.** *Biochemistry,* 1974, vol. 13 (2), 222-245
- **CHOU et al.** *Biochemistry,* 1974, vol. 113 ((2)), 211-222
- **CHOU et al.** *Adv. Enzymol. Relat. Areas Mol. Biol,* 1978, vol. 47, 45-148
- **CHOU et al.** *Ann. Rev. Biochem.,* vol. 47, 251-276
- **CHOU et al.** *Biophys. J.,* 1979, vol. 26, 367-384
- **HOLM et al.** *Nucl. Acid. Res,* 1999, vol. 27 (1), 244-247
- **BRENNER et al.** *Curr. Op. Struct. Biol,* 1997, vol. 7 (3), 369-376
- **JONES, D.** *Curr. Opin. Struct. Biol,* 1997, vol. 7 (3), 377-87
- **SIPPL et al.** *Structure,* 1996, vol. 4 (1), 15-9
- **BOWIE et al.** *Science,* 1991, vol. 253, 164-170
- **GRIBSKOV et al.** *Meth. Enzym,* 1990, vol. 183, 146-159
- **GRIBSKOV et al.** *Proc. Nat. Acad. Sci.,* 1987, vol. 84 (13), 4355-4358
- **CAPON et al.** *Nature,* 1989, vol. 337, 525-31
- **ZHENG et al.** *J. Immunol.,* 1995, vol. 154, 5590-5600
- **FISHER et al.** *N. Engl. J. Med.,* 1996, vol. 334, 1697-1702
- **VAN ZEE et al.** *J. Immunol.,* 1996, vol. 156, 2221-2230
- **CAPON et al.** *Nature,* 1989, vol. 337, 525-531
- **HARVILL et al.** *Immunotech,* 1995, vol. 1, 95-105
- **LINSLEY.** *J. Exp. Med.,* 1991, vol. 174, 561-569
- Computational Molecular Biology. Oxford University Press, 1988
- Biocomputing: Informatics and Genome Projects. Academic Press, 1993
- Computer Analysis of Sequence Data, Part 1. Humana Press, 1994
- Sequence Analysis in Molecular Biology. Academic Press, 1987
- Sequence Analysis Primer. M. Stockton Press, 1991
- **CARILLO et al.** *SIAM J. Applied Math,* 1988, vol. 48, 1073
- **DEVEREUX et al.** *Nucl. Acid. Res,* 1984, vol. 12, 387
- **ALTSCHUL et al.** *J. Mol. Biol.,* 1990, vol. 215, 403-410
- **ALTSCHUL et al.** BLAST Manual. NCB/NLM/NIH
- **DAYHOFF et al.** *Atlas of Protein Sequence and Structure,* 1978, vol. 5 (3
- **HENIKOFF et al.** *Proc. Natl. Acad. Sci USA,* 1992, vol. 89, 10915-10919
- **NEEDLEMAN et al.** *J. Mol. Biol.,* 1970, vol. 48, 443-453
- **HENIKOFF et al.** *Proc. Natl. Acad. Sci. USA,* 1992, vol. 89, 10915-10919
- **NEEDLEMAN et al.** *J. Mol Biol.,* 1970, vol. 48, 443-453
- Program Manual. September 1997
- **SAMBROOK et al.** Molecular Cloning: A Laboratory Manual. Cold Spring Harbor Laboratory Press, 1989
- Current Protocols in Molecular Biology. Green Publishers Inc. and Wiley and Sons, 1994
- **ENGELS et al.** *Angew. Chem. Intl. Ed.,* 1989, vol. 28, 716-734
- Meth. Enz. Academic Press Inc, 1990, vol. 185
- **BERNOIST ; CHAMBON.** *Nature,* 1981, vol. 290, 304-310
- **YAMAMOTO et al.** *Cell,* 1980, vol. 22, 787-797
- **WAGNER et al.** *Proc. Natl. Acad. Sci. USA,* 1981, vol. 78, 144-1445
- **BRINSTER et al.** *Nature,* 1982, vol. 296, 39-42
- **VILLA-KAMAROFF et al.** *Proc. Natl. Acad. Sci. USA,* 1978, vol. 75, 3727-3731
- **DEBOER et al.** *Proc. Natl. Acad. Sci. USA,* 1983, vol. 80, 21-25
- **SWIFT et al.** *Cell,* 1984, vol. 38, 639-646
- **ORNITZ et al.** *Cold Spring Harbor Symp. Quant. Biol,* 1986, vol. 50, 399-409
- **MACDONALD.** *Hepatology,* 1987, vol. 7, 425-515
- **HANAHAN.** *Nature,* 1985, vol. 315, 115-122
- **GROSSCHEDL et al.** *Cell,* 1984, vol. 38, 647-658

- **ADAMES et al.** *Nature,* 1985, vol. 318, 533-538
- **ALEXANDER et al.** *Mol. Cell. Biol,* 1987, vol. 7, 1436-1444
- **LEDER et al.** *Cell,* 1986, vol. 45, 485-495
- **PINKERT et al.** *Genes and Devel,* 1987, vol. 1, 268-276
- **KRUMLAUF et al.** *Mol. Cell. Biol,* 1985, vol. 5, 1639-1648
- **HAMMER et al.** *Science,* 1987, vol. 235, 53-58
- **KELSEY et al.** *Genes and Devel,* 1987, vol. 1, 161-171
- **MOGRAM et al.** *Nature,* 1985, vol. 315, 338-340
- **KOLLIAS et al.** *Cell,* 1986, vol. 46, 89-94
- **READHEAD et al.** *Cell,* 1987, vol. 48, 703-712
- **SANI.** *Nature,* 1985, vol. 314, 283-286
- **MASON et al.** *Science,* 1986, vol. 234, 1372-1378
- **URLAUB et al.** *Proc. Natl. Acad. Sci. USA,* 1980, vol. 97, 4216-4220
- **KITTS et al.** *Biotechniques,* 1993, vol. 14, 810-817
- **LUCKLOW.** *Curr. Opin. Biotechnol,* 1993, vol. 4, 564-572
- **LUCKLOW et al.** *J. Virol.,* 1993, vol. 67, 4566-4579
- **MARSTON et al.** *Meth. Enz,* 1990, vol. 182, 264-275
- Current Protocols in Molecular Biology. John Wiley & Sons, 1993
- **MERRIFIELD et al.** *J. Am. Chem. Soc.,* 1963, vol. 85, 2149
- **HOUGHTEN et al.** *Proc. Natl. Acad. Sci. USA,* 1985, vol. 82, 5132
- **ROBERTS et al.** *Proc. Natl. Acad. Sci,* 1997, vol. 94, 12297-12303
- **ROBERTS, R.** *Curr. Opin. Chem. Biol.,* 1999, vol. 3, 268-273
- **FRANCIS et al.** *Focus on Growth Factors,* 1992, vol. 3, 4-10
- **KOHLER et al.** *Nature,* 1975, vol. 256, 495-497
- **KOZBOR.** *J. Immunol.,* 1984, vol. 133, 3001
- **BRODEUR et al.** Monoclonal Antibody Production Techniques and Applications. Marcel Dekker, Inc, 1987, 51-63
- **MORRISON et al.** *Proc. Natl. Acad. Sci.,* 1985, vol. 81, 6851-6855
- **JONES et al.** *Nature,* 1986, vol. 321, 522-525
- **RIECHMANN et al.** *Nature,* 1988, vol. 332, 323-327
- **VERHOEYEN et al.** *Science,* 1988, vol. 239, 1534-1536
- **JAKOBOVITS et al.** *Proc. Natl. Acad. Sci.,* 1993, vol. 90, 2551-2555
- **JAKOBOVITS et al.** *Nature,* 1993, vol. 362, 255-258
- **BRUGGERMANN et al.** *Year in Immuno,* 1993, vol. 7, 33
- **HOOGENBOOM et al.** *J. Mol. Biol.,* 1991, vol. 227, 381
- **MARKS et al.** *J. Mol. Biol.,* 1991, vol. 222, 581
- **SOLA.** Monoclonal Antibodies: A Manual of Techniques. CRC Press, Inc, 1987, 147-158
- **BAYER et al.** *Meth. Enz.,* 1990, vol. 184, 138-163
- Current Protocols in Molecular Biology. John Wiley & Sons, 1995
- **FALWELL et al.** *Proc. Natl. Acad. Sci.,* 1994, vol. 91, 664-668
- **SCHWARZE et al.** *Science,* 1999, vol. 285, 1569-1572
- **NAGAHARA et al.** *Nature Medicine,* 1998, vol. 4, 1449-1452
- **STRAUSS, E.** Introducing Proteins Into the Body's Cells. *Science,* 1999, vol. 285, 1466-1467
- **SUN ; DAVIES.** *Annual Review of Biophysics and Biomolecular Structure,* 1995, vol. 24, 269-291
- Remington's Pharmaceutical Sciences. Mack Publishing Company, 1990
- **SIDMAN et al.** *Biopolymers,* 1983, vol. 22, 547-556
- **LANGER et al.** *J. Biomed. Mater. Res.,* 1981, vol. 15, 167-277
- **LANGER.** *Chem. Tech.,* 1982, vol. 12, 98-105
- **EPPSTEIN et al.** *Proc. Natl. Acad. Sci. USA,* 1985, vol. 82, 3688-3692
- **KUCHERLAPATI.** *Prog. in Nucl. Acid Res. & Mol. Biol,* 1989, vol. 36, 301
- **THOMAS et al.** *Cell,* 1986, vol. 44, 419-428
- **THOMAS ; CAPECCHI.** *Cell,* 1987, vol. 51, 503-512
- **DOETSCHMAN et al.** *Proc. Natl. Acad. Sci.,* 1988, vol. 85, 8583-8587
- **DOETSCHMAN et al.** *Nature,* 1987, vol. 330, 576-578
- **SAUER.** *Current Opinion In Biotechnology,* 1994, vol. 5, 521-527
- **SAUER.** *Methods In Enzymology,* 1993, vol. 225, 890-900
- **BAUBONIS ; SAUER.** *Nucleic Acids Res.,* 1993, vol. 21, 2025-2029
- **O'GORMAN et al.** *Science,* 1991, vol. 251, 1351-1355
- **WINN et al.** *Exper. Neurol,* 1991, vol. 113, 322-329
- **AEBISCHER et al.** *Exper. Neurol,* 1991, vol. 111, 269-275
- **TRESCO et al.** *ASAIO,* 1992, vol. 38, 17-23
- *Science,* 2000, vol. 287, 816-817 826830
- **HEFTI.** *Neurobiology,* 1994, vol. 25, 1418-1435

SEQUENCE LISTING

<110> Amgen, Inc.

<120> Cysteine Knot Polypeptides: Cloaked-2 Molecules and Uses Thereof

<130> 01017/37428

<150> US 60/208,550
<151> 2000-06-01

<150> US 60/223,542
<151> 2000-08-04

<160> 25

<170> PatentIn version 3.0

<210> 1
<211> 759
<212> DNA
<213> Homo sapiens

<400> 1

tactggaagg tggcgtgccc tcctctggct ggtaccatgc agctcccact ggccctgtgt 60

ctcgtctgcc tgctggtaca cacagccttc cgtgtagtgg agggccaggg gtggcaggcg 120

ttcaagaatg atgccacgga aatcatcccc gagctcggag agtaccccga gcctccaccg 180

gagctggaga acaacaagac catgaaccgg gcggagaacg gagggcggcc tccccaccac 240

ccctttgaga ccaaagacgt gtccgagtac agctgccgcg agctgcactt cacccgctac 300

gtgaccgatg ggccgtgccg cagcgccaag ccggtcaccg agctggtgtg ctccggccag 360

tgcggcccgg cgcgcctgct gcccaacgcc atcggccgcg gcaagtggtg gcgacctagt 420

gggcccgact tccgctgcat ccccgaccgc taccgcgcgc agcgcgtgca gctgctgtgt 480

cccggtggtg aggcgccgcg cgcgcgcaag gtgcgcctgg tggcctcgtg caagtgcaag 540

cgcctcaccc gcttccacaa ccagtcggag ctcaaggact tcgggaccga ggccgctcgg 600

ccgcagaagg gccggaagcc gcggccccgc gcccggagcg ccaaagccaa ccaggccgag 660

ctggagaacg cctactagag cccgcccgcg cccctcccca ccggcgggcg ccccggccct 720

gaacccgcgc cccacatttc tgtcctctgc gcgtggttt 759

<210> 2
<211> 190
<212> PRT
<213> Homo sapiens

<400> 2

Gln Gly Trp Gln Ala Phe Lys Asn Asp Ala Thr Glu Ile Ile Pro Glu

Gln Gly Trp Gln Ala Phe Lys Asn Asp Ala Thr Glu Ile Ile Pro Glu
1               5                   10                  15

Leu Gly Glu Tyr Pro Glu Pro Pro Pro Glu Leu Glu Asn Asn Lys Thr
            20                  25                  30

Met Asn Arg Ala Glu Asn Gly Gly Arg Pro Pro His His Pro Phe Glu
        35                  40                  45

Thr Lys Asp Val Ser Glu Tyr Ser Cys Arg Glu Leu His Phe Thr Arg
    50                  55                  60

Tyr Val Thr Asp Gly Pro Cys Arg Ser Ala Lys Pro Val Thr Glu Leu
65                  70                  75                  80

Val Cys Ser Gly Gln Cys Gly Pro Ala Arg Leu Leu Pro Asn Ala Ile
                85                  90                  95

Gly Arg Gly Lys Trp Trp Arg Pro Ser Gly Pro Asp Phe Arg Cys Ile
            100                 105                 110

Pro Asp Arg Tyr Arg Ala Gln Arg Val Gln Leu Leu Cys Pro Gly Gly
        115                 120                 125

Glu Ala Pro Arg Ala Arg Lys Val Arg Leu Val Ala Ser Cys Lys Cys
    130                 135                 140

Lys Arg Leu Thr Arg Phe His Asn Gln Ser Glu Leu Lys Asp Phe Gly
145                 150                 155                 160

Thr Glu Ala Ala Arg Pro Gln Lys Gly Arg Lys Pro Arg Pro Arg Ala
                165                 170                 175

Arg Ser Ala Lys Ala Asn Gln Ala Glu Leu Glu Asn Ala Tyr
            180                 185                 190

<210> 3
<211> 636
<212> DNA
<213> Mus musculus

<400> 3

atgcagccct cactagcccc gtgcctcatc tgcctacttg tgcacgctgc cttctgtgct 60

gtggagggcc aggggtggca agccttcagg aatgatgcca cagaggtcat cccagggctt 120

ggagagtacc ccgagcctcc tcctgagaac aaccagacca tgaaccgggc ggagaatgga 180

ggcagacctc cccaccatcc ctatgacgcc aaagatgtgt ccgagtacag ctgccgcgag 240

ctgcactaca cccgcttcct gacagacggc ccatgccgca gcgccaagcc ggtcaccgag 300

ttggtgtgct ccggccagtg cggccccgcg cggctgctgc ccaacgccat cgggcgcgtg 360

aagtggtggc gcccgaacgg accggatttc cgctgcatcc cggatcgcta ccgcgcgcag 420

cgggtgcagc tgctgtgccc cgggggcgcg gcgccgcgct cgcgcaaggt gcgtctggtg 480

gcctcgtgca agtgcaagcg cctcacccgc ttccacaacc agtcggagct caaggacttc 540

gggccggaga ccgcgcggcc gcagaagggt cgcaagccgc ggcccggcgc ccggggagcc 600

aaagccaacc aggcggagct ggagaacgcc tactag 636

<210> 4
<211> 185
<212> PRT
<213> Mus musculus

<400> 4

Gln Gly Trp Gln Ala Phe Arg Asn Asp Ala Thr Glu Val Ile Pro Gly
1 5 10 15

Leu Gly Glu Tyr Pro Glu Pro Pro Pro Glu Asn Asn Gln Thr Met Asn
20 25 30

Arg Ala Glu Asn Gly Gly Arg Pro Pro His His Pro Tyr Asp Ala Lys
35 40 45

Asp Val Ser Glu Tyr Ser Cys Arg Glu Leu His Tyr Thr Arg Phe Leu
50 55 60

Thr Asp Gly Pro Cys Arg Ser Ala Lys Pro Val Thr Glu Leu Val Cys
65 70 75 80

Ser Gly Gln Cys Gly Pro Ala Arg Leu Leu Pro Asn Ala Ile Gly Arg
85 90 95

Val Lys Trp Trp Arg Pro Asn Gly Pro Asp Phe Arg Cys Ile Pro Asp
100 105 110

Arg Tyr Arg Ala Gln Arg Val Gln Leu Leu Cys Pro Gly Gly Ala Ala
115 120 125

Pro Arg Ser Arg Lys Val Arg Leu Val Ala Ser Cys Lys Cys Lys Arg
130 135 140

Leu Thr Arg Phe His Asn Gln Ser Glu Leu Lys Asp Phe Gly Pro Glu
145 150 155 160

Thr Ala Arg Pro Gln Lys Gly Arg Lys Pro Arg Pro Gly Ala Lys Ala
165 170 175

Asn Gln Ala Glu Leu Glu Asn Ala Tyr
180 185

<210> 5
<211> 213
<212> PRT
<213> Homo sapiens

<400> 5

Met Gln Leu Pro Leu Ala Leu Cys Leu Val Cys Leu Leu Val His Thr
1 5 10 15

Ala Phe Arg Val Val Glu Gly Gln Gly Trp Gln Ala Phe Lys Asn Asp
20 25 30

Ala Thr Glu Ile Ile Pro Glu Leu Gly Glu Tyr Pro Glu Pro Pro Pro
35 40 45

Glu Leu Glu Asn Asn Lys Thr Met Asn Arg Ala Glu Asn Gly Gly Arg
    50                  55                  60

Pro Pro His His Pro Phe Glu Thr Lys Asp Val Ser Glu Tyr Ser Cys
65                  70                  75                  80

Arg Glu Leu His Phe Thr Arg Tyr Val Thr Asp Gly Pro Cys Arg Ser
                85                  90                  95

Ala Lys Pro Val Thr Glu Leu Val Cys Ser Gly Gln Cys Gly Pro Ala
            100                 105                 110

Arg Leu Leu Pro Asn Ala Ile Gly Arg Gly Lys Trp Trp Arg Pro Ser
        115                 120                 125

Gly Pro Asp Phe Arg Cys Ile Pro Asp Arg Tyr Arg Ala Gln Arg Val
    130                 135                 140

Gln Leu Leu Cys Pro Gly Gly Glu Ala Pro Arg Ala Arg Lys Val Arg
145                 150                 155                 160

Leu Val Ala Ser Cys Lys Cys Lys Arg Leu Thr Arg Phe His Asn Gln
                165                 170                 175

Ser Glu Leu Lys Asp Phe Gly Thr Glu Ala Ala Arg Pro Gln Lys Gly
            180                 185                 190

Arg Lys Pro Arg Pro Arg Ala Arg Ser Ala Lys Ala Asn Gln Ala Glu
        195                 200                 205

Leu Glu Asn Ala Tyr
    210

<210> 6
<211> 208
<212> PRT
<213> Mus musculus

<400> 6

Met Gln Pro Ser Leu Ala Pro Cys Leu Ile Cys Leu Leu Val His Ala
1               5                   10                  15

Ala Phe Cys Ala Val Glu Gly Gln Gly Trp Gln Ala Phe Arg Asn Asp
            20                  25                  30

Ala Thr Glu Val Ile Pro Gly Leu Gly Glu Tyr Pro Glu Pro Pro Pro
        35                  40                  45

Glu Asn Asn Gln Thr Met Asn Arg Ala Glu Asn Gly Gly Arg Pro Pro
    50                  55                  60

His His Pro Tyr Asp Ala Lys Asp Val Ser Glu Tyr Ser Cys Arg Glu
65                  70                  75                  80

Leu His Tyr Thr Arg Phe Leu Thr Asp Gly Pro Cys Arg Ser Ala Lys
                85                  90                  95

Pro Val Thr Glu Leu Val Cys Ser Gly Gln Cys Gly Pro Ala Arg Leu
            100                 105                 110

Leu Pro Asn Ala Ile Gly Arg Val Lys Trp Trp Arg Pro Asn Gly Pro

115 120 125

Asp Phe Arg Cys Ile Pro Asp Arg Tyr Arg Ala Gln Arg Val Gln Leu
130 135 140

Leu Cys Pro Gly Gly Ala Ala Pro Arg Ser Arg Lys Val Arg Leu Val
145 150 155 160

Ala Ser Cys Lys Cys Lys Arg Leu Thr Arg Phe His Asn Gln Ser Glu
165 170 175

Leu Lys Asp Phe Gly Pro Glu Thr Ala Arg Pro Gln Lys Gly Arg Lys
180 185 190

Pro Arg Pro Gly Ala Lys Ala Asn Gln Ala Glu Leu Glu Asn Ala Tyr
195 200 205

<210> 7
<211> 24
<212> DNA
<213> Artificial

<220>
<223> Artificial: PCR primer

<400> 7

tactggaagg tggcgtgccc tcct 24

<210> 8
<211> 26
<212> DNA
<213> Artificial

<220>
<223> Artificial: PCR primer

<400> 8

aaaccacgcg cagaggacag aaatgt 26

<210> 9
<211> 29
<212> DNA
<213> Artificial

<220>
<223> Artificial: PCR primer

<400> 9

gccaggggtg gcaagccttc aagaatgat 29

<210> 10
<211> 24
<212> DNA
<213> Artificial

<220>

<223> Artificial: PCR primer

<400> 10

cgatccggga tgcagcggaa gtcg 24

<210> 11
<211> 27
<212> DNA
<213> Artificial

<220>
<223> Artificial: PCR primer

<400> 11

ccatcctaat acgactcact atagggc 27

<210> 12
<211> 24
<212> DNA
<213> Artificial

<220>
<223> Artificial: PCR primer

<400> 12

tgtcaggaag cgggtgtagt gcag 24

<210> 13
<211> 23
<212> DNA
<213> Artificial

<220>
<223> Artificial: PCR primer

<400> 13

actcactata gggctcgagc ggc 23

<210> 14
<211> 25
<212> DNA
<213> Artificial

<220>

<223> Artificial: PCR primer

<400> 14

ggacacatct ttggcgtcat aggga 25

<210> 15
<211> 21
<212> DNA

<213> Artificial

<220>
<223> Artificial: PCR primer

<400> 15

tacacccgct tcctgacaga c 21

<210> 16
<211> 27
<212> DNA
<213> Artificial

<220>
<223> Artificial: PCR primer

<400> 16

ccatcctaat acgactcact atagggc 27

<210> 17
<211> 21
<212> DNA
<213> Artificial

<220>
<223> Artificial: PCR primer

<400> 17

ggtcaccgag ttggtgtgct c 21

<210> 18
<211> 23
<212> DNA
<213> Artificial

<220>
<223> Artificial: PCR primer

<400> 18

actcactata gggctcgagc ggc 23

<210> 19
<211> 45
<212> DNA
<213> Artificial

<220>
<223> Artificial: PCR primer

<400> 19

cgtactagta agcttccacc atgcagccct cactagcccc gtgcc 45

<210> 20
<211> 41
<212> DNA
<213> Artificial

<220>
<223> Artificial: PCR primer

<400> 20

tttggatccc gatcgctagt aggcgttctc cagctccgcc t 41

<210> 21
<211> 27
<212> DNA
<213> Artificial

<220>
<223> Artificial: PCR primer

<400> 21

tgtgtctcgt ctgcctgctg gtacaca 27

<210> 22
<211> 23
<212> DNA
<213> Artificial

<220>
<223> Artificial: PCR primer

<400> 22

gaagtcgggc ccactaggtc gcc 23

<210> 23
<211> 11
<212> PRT
<213> Artificial: HIV TAT peptide

<400> 23

Tyr Gly Arg Lys Lys Arg Arg Gln Arg Arg Arg
1 5 10

<210> 24
<211> 15
<212> PRT
<213> Artificial

<220>
<223> Artificial: FITC conjugated - HIV TAT peptide construct

<400> 24

Gly Gly Gly Gly Tyr Gly Arg Lys Lys Arg Arg Gln Arg Arg Arg
1 5 10 15

<210> 25
<211> 183
<212> PRT
<213> Homo sapiens

<400> 25

```
Phe Lys Asn Asp Ala Thr Glu Ile Leu Tyr Ser His Val Val Lys Pro
1               5                   10                  15

Val Pro Ala His Pro Ser Ser Asn Ser Thr Leu Asn Gln Ala Arg Asn
            20                  25                  30

Gly Gly Arg His Phe Ser Asn Thr Gly Leu Asp Arg Asn Thr Arg Val
        35                  40                  45

Gln Val Gly Cys Arg Glu Leu Arg Ser Thr Lys Tyr Ile Ser Asp Gly
    50                  55                  60

Gln Cys Thr Ser Ile Ser Pro Leu Lys Glu Leu Val Cys Ala Gly Glu
65                  70                  75                  80

Cys Leu Pro Leu Pro Val Leu Pro Asn Trp Ile Gly Gly Gly Tyr Gly
                85                  90                  95

Thr Lys Tyr Trp Ser Arg Arg Ser Ser Gln Glu Trp Arg Cys Val Asn
            100                 105                 110

Asp Lys Thr Arg Thr Gln Arg Ile Gln Leu Gln Cys Gln Asp Gly Ser
        115                 120                 125

Thr Arg Thr Tyr Lys Ile Thr Val Val Thr Ala Cys Lys Cys Lys Arg
    130                 135                 140

Tyr Thr Arg Gln His Asn Glu Ser Ser His Asn Phe Glu Ser Met Ser
145                 150                 155                 160

Pro Ala Lys Pro Val Gln His His Arg Glu Arg Lys Arg Ala Ser Lys
                165                 170                 175

Ser Ser Lys His Ser Met Ser
            180
```